# EUROPEAN PATENT APPLICATION

(11) **EP 0 613 879 A1**
(43) Date of publication of application: **07.09.1994**
(21) Application number: 92910349.7
(22) Date of filing: 20.05.1992
(51) Int. Cl.: C07C 211/27, C07C 211/45, C07C 215/50, C07C 215/52, C07C 217/60, C07C 233/05, C07C 233/22, C07C 233/65, C07C 233/73, C07C 217/58, A61K 31/135, A61K 31/16, A61K 31/165, A61K 31/47

(54) **PHELLODENDRINE ANALOGS AND ALLERGY TYPE IV SUPPRESSOR CONTAINING THE SAME AS ACTIVE INGREDIENT**

(30) Priority: 20.05.1991 JP 143858/91; 20.05.1991 JP 143987/91
(71) Applicant: TSUMURA & CO., Chuo-ku, Tokyo 103 (JP)
(72) Inventor: KODA, Akihide, Gifu-shi Gifu 502 (JP); NISHIOKA, Itsuo, Fukuoka-shi Fukuoka 815 (JP); KAWAMURA, Hideki, Inashiki-gun Ibaraki 300-11 (JP); ONO, Yutaka, Inashiki-gun Ibaraki 300-11 (JP); HOSAKA, Kunio, Inashiki-gun Ibaraki 300-11 (JP); MATSUOKA, Yoshiyuki, Inashiki-gun Ibaraki 300-11 (JP); OGAWA, Yoshimitsu, Inashiki-gun Ibaraki 300-11 (JP); FUJISAWA, Shinichi, Inashiki-gun Ibaraki 300-11 (JP)
(74) Representative: Wächtershäuser, Günter, Prof. Dr.
(86) International application number: JP9200647
(87) International publication number: WO9220645

(57) **Abstract**

Phellodendrine analogs represented by general formula (I), wherein A represents the group (a); B represents hydrogen, lower alkyl or lower acyl, or alternatively A and B together with the adjacent nitrogen atom form a substituted 1,2,3,4-tetrahydroisoquinoline ring represented by general formula (II), R₁₁, R₁₂, R₂₁, R₂₂, R₃₁ and R₃₂ represent each hydrogen, hydroxyl or lower alkoxy; n₁ represents a number of 0 to 2; n₂ represents a number of 1 and 2; and m₁ represents a number of 0 to 1, provided tsat when A represents the group (b), and n₂ is 2, B is lower acyl, and that when A and B together form a substituted 1,2,3,4-tetrahydroisoquinoline ring, n₁ is 1 and m₁ is not 0. These analogs (I) and related compounds have an excellent activity of suppressing allergy type IV and hence are utilizable as a medicine efficacious against diseases wherein allergy type IV participates, such as chronic hepatitis, intractable asthma, nephrotic syndrome or rheumatism.

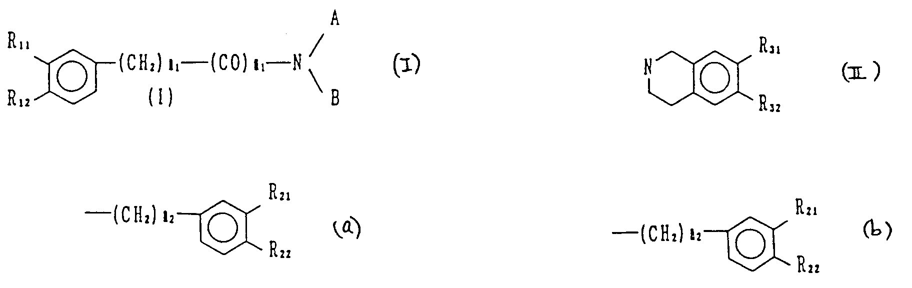

## Description

### Field of the Invention

The present invention relates to a novel phellodendrine analog and an allergy type IV suppressor comprising the same as an active ingredient.

### Background of the Invention

There are a number of patients of diseases involving allergy type IV, such as chronic hepatitis, intractable asthma, nephrotic syndrome, rheumatism, or the like, and a great social attentions are given to these diseases.

Conventionally, immunosuppressors, typically including steroidal agents, which suppress cellular antibody production have been used for treating these diseases.

However, because conventionally used immunosuppressors, such as steroidal agents and the like, have strong side effects, their use has been restricted. Development of an allergy type IV suppressor exhibiting minimal side effects and excellent pharmaceutical effects has therefore been desired.

### Disclosure of the Invention

The present inventors have synthesized a number of compounds and undertaken earnest studies on their pharmacological actions in order to provide an allergy type IV suppressor effective for curing diseases involving the IV-type allergy, such as chronic hepatitis, intractable asthma, nephrotic syndrome, rheumatism, or the like. As a result, the present inventors have found that novel phellodendrine analogs represented by the following formula (I) exhibit excellent actions on suppression of the allergy type IV, and completed the present invention.

Accordingly, a first object of the present invention is to provide phellodendrine analogs represented by the following formula (I), 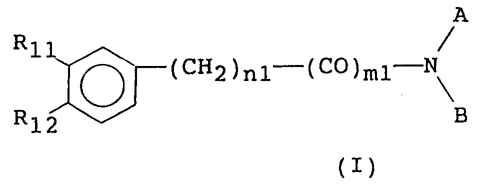
wherein A represents a group 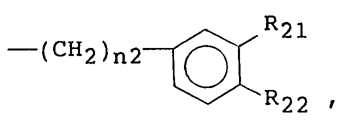
wherein R₂₁ and R₂₂ individually represent a hydrogen atom, a hydroxy group, or a lower alkoxy group; and n2 is 1 or 2; B represents a hydrogen atom, a lower alkyl group, or a lower acyl group, or A and B may form, together with the adjacent nitrogen atom, a substituted 1,2,3,4-tetrahydroisoquinolyl ring represented by the following formula, 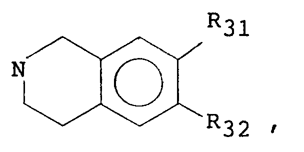
wherein R₃₁ and R₃₂ individually represent a hydrogen atom, a hydroxy group, or a lower alkoxy group; R₁₁ and R₁₂ individually represent a hydrogen atom, a hydroxy group, or a lower alkoxy group; n1 denotes a number 0-2; and m1 denotes a number 0-1; provided that when A is a group 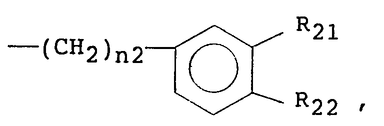
if n2 is 2, B is a lower acyl group, and further that when A and B together form a substituted 1,2,3,4-tetrahydroisoquinolyl ring, n1 is 1 and m1 is not 0.

A second object of the present invention is to provide an allergy type IV suppressor comprising a phellodendrine analog represented by the above formula (I), its related compound, and the like as an active ingredient.

### Best Mode for Carrying out the Invention

The phellodendrine analogs (I) of the present invention are novel compounds. They are broadly classified into the following three groups of compounds (Ia) to (Ic), 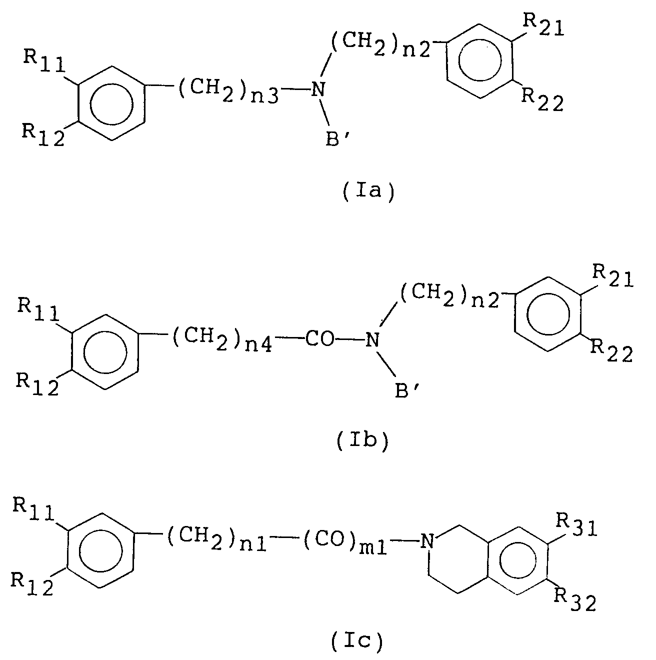
wherein R₁₁, R₁₂, R₂₁, R₂₂, R₃₁, R₃₂, n1, n2, and m1 have the same meanings as defined above, n3 is 1 or 2, n4 is 0 or 1, B' represents a hydrogen atom, a lower alkyl group, or a lower acyl group, provided that when either one of R₁₁ or R₁₂ is a hydrogen atom and either one of R₂₁ or R₂₂ is a hydrogen atom, B' is a lower acyl group.

The phellodendrine analogs (I) of the present invention can be prepared, for example, by the following processes.

### Preparation Process 1:

(1) A compound having a hydrogen atom for B, 1 for m1, and 0 or 1 for n1 in formula (I) (Compound I-1) can be prepared by acylating the nitrogen of a compound represented by formula (II) with a compound represented by formula (III) according to the following formula. 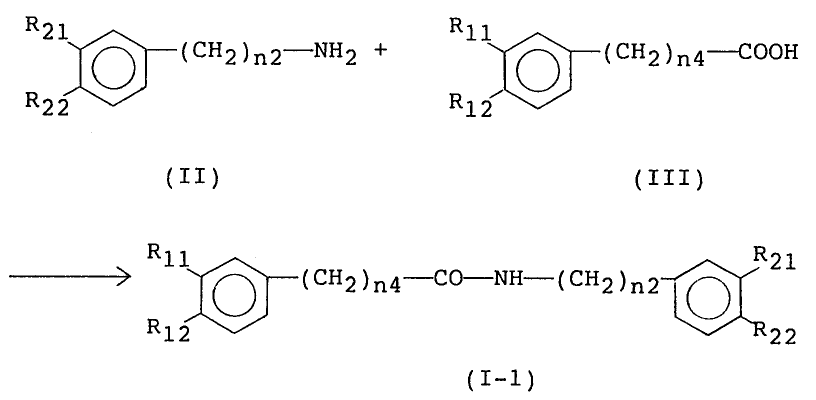 wherein R₁₁, R₁₂, R₂₁, R₂₂, R₃₁, R₃₂, n2, and n4 have the same meanings as defined above.
(2) A compound having a lower acyl group for B in formula (I) (Compound I-3) can be prepared by acylating the nitrogen of a compound having a hydrogen atom for B in formula (I) (Compound I-2) with a compound represented by formula (IV) according to the following formula. 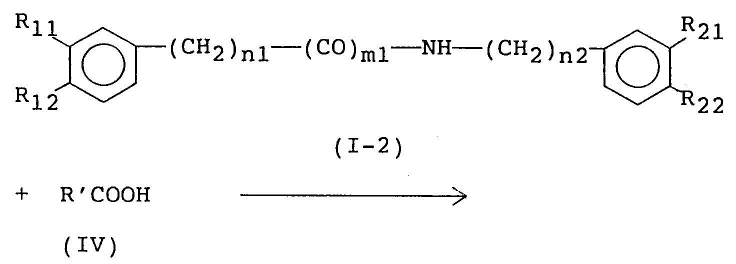 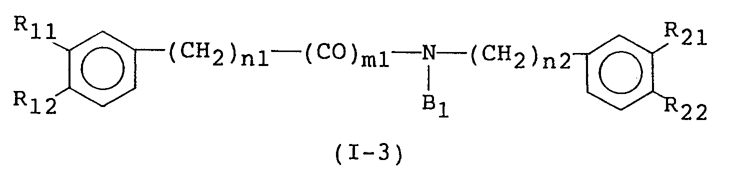 wherein R₁₁, R₁₂, R₂₁, R₂₂, n1, n2, and m1 have the same meanings as defined above, R' is a lower alkyl group, and B₁ is a lower acyl group.
(3) A compound having 1 for m1 and 0 for n1 in formula (Ic) (Compound I-4) can be prepared by acylating the nitrogen of a tetrahydroisoquinoline derivative of formula (V) with a compound represented by formula (III) according to the following formula. 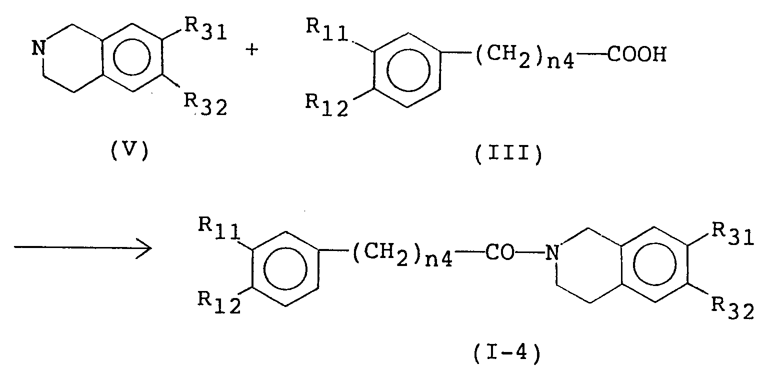 wherein R₁₁, R₁₂, R₃₁, R₃₂, and n4 have the same meanings as defined above.

The acylation of nitrogen atoms in the above reactions can be carried out by a conventional method, wherein a carboxylic acid represented by formula (III) or (IV), or a reactive derivative thereof, such as an acid chloride, an acid anhydride, or the like, and a primary or secondary amine represented by formula (II) or (I-2) are reacted at -78°C to 150°C in a solvent, such as a common organic solvent (e.g., hydrocarbon, methylene chloride, chloroform, carbon tetrachloride, ether, tetrahydrofuran (THF), dimethylformamide (DFA), dimethylsulfoxide (DMSO), etc.), an amine compound (e.g., a tertiary amine, etc.), water, or the like.

### Preparation Process 2:

A compound having a lower alkyl group for B in formula (I) (Compound I-6) can be prepared by alkylating a compound having a hydrogen atom for B in formula (I) (Compound I-5) with a compound (VI) according to the following formula. 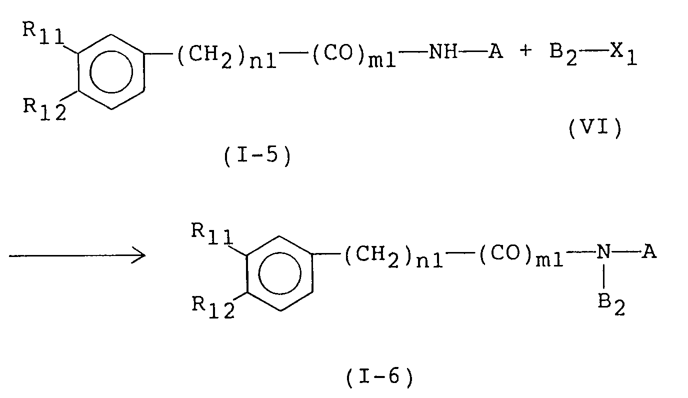
wherein A, R₁₁, R₁₂, n1, and m1 have the same meanings as defined above, B₂ represents a lower alkyl group, and X₁ is a releasing group such as a halogen atom, a tosyl group, a mesyl group, or the like.

The N-alkylation can be carried out by producing an anion using a base, such as sodium hydride, lithium diisopropylamide (LDA), sodium hexamethyldisilazide, potassium hydroxide, sodium hydroxide, or the like, in an amount equivalent to or greater than the amount of compound (I-5), and reacting with an equivalent or greater amount of compound (VI), which is an alkylating agent.

A hydrocarbon, methylene chloride, chloroform, carbon tetrachloride, ether, tetrahydrofuran (THF), dimethylformamide (DMF), dimethylsulfoxide (DMSO), or the like can be used as a solvent. The reaction temperature in the range of -78°C to 150°C can be employed depending on the reagents used.

Specifically, methylation or ethylation, for instance, can be carried out by reacting using methyl iodide or ethyl iodide and potassium hydroxide in DMSO. Propylation can be achieved advantageously by reacting using propyl iodide and sodium hydroxide in DMSO.

### Preparation Process 3:

(1) Compound (Ia) can be prepared by reducing the carbonyl group of compound (Ib) according to the following formula. 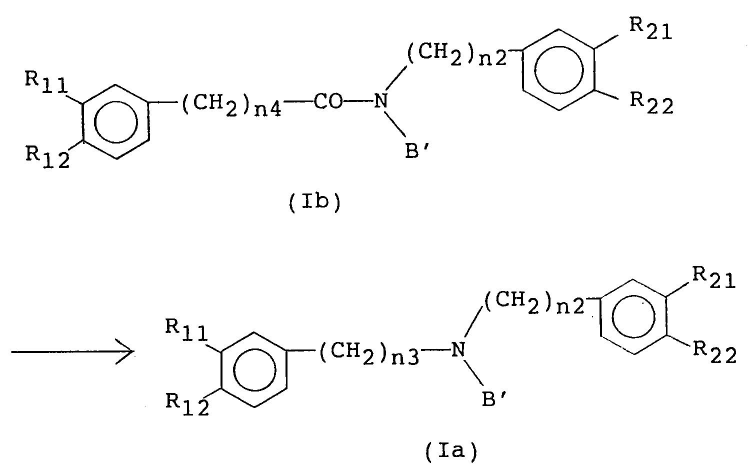 wherein B, R₁₁, R₁₂, R₂₁, R₂₂, n2, n3, and n4 have the same meanings as defined above.
(2) A compound having a hydrogen atom for B in formula (Ia) (Compound I-7) can be prepared by reducing the group -CH=N of a compound represented by formula (VII) according to the following formula. 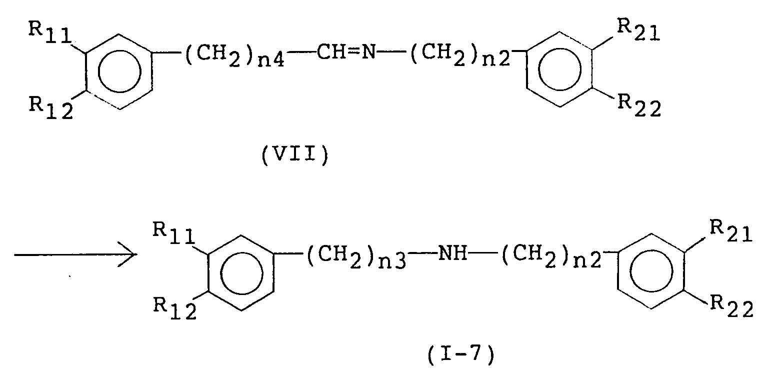 wherein R₁₁, R₁₂, R₂₁, R₂₂, n2, n3, and n4 have the same meanings as defined above.

The above reducing reactions can be carried out using a metal hydride (e.g., diisobutyl aluminum hydride, an organic tin hydride, hydrosilane, etc.), a metal hydride complex (e.g., lithium aluminum hydride, sodium borohydride, etc.), or a complex (e.g., boran, diboran, etc.).

This reaction can be carried out in air or an inert gas using a reducing agent of an amount equivalent to or greater than the amount of compound (Ib) or compound (VII) and at least one kind of organic solvent selected from the group consisting of lower alcohols, hydrocarbons, methylene chloride, chloroform, ether, tetrahydrofuran, and the like under atmospheric pressure or under a pressure. The reaction temperature may be determined depending on the reagents used, normally within the range of -78°C to 150°C.

### Preparation Process 4:

A compound having a hydroxy group for at least one of R₁₁, R₁₂, R₂₁, R₂₂, R₃₁, or R₃₂ in formula (I) can be prepared by subjecting a compound having a lower alkoxy group for the corresponding hydroxy group to a de-lower alkyl group reaction.

The de-lower alkyl group reaction can be carried out by reacting boron tribromide or a Lewis acid (brone trichloride, boron tribromide, aluminum chloride, boron trifluoride, etc.) with said compound in an organic solvent such as a hydrocarbon, methylene chloride, tetrahydrofuran, or the like, thus removing the lower alkyl groups from the target alkoxy groups and converting them into hydroxy groups.

### Preparation Process 5:

A compound having a hydroxy group for either one of R₁₁ or R₁₂, R₂₁ or R₂₂, and R₃₁ or R₃₂ in formula (I), and having a lower alkoxy group for the other, can be prepared by subjecting a compound having a benzyloxy group for the corresponding hydroxy group to a de-benzylation reaction.

The de-benzylation reaction can be achieved by heating the compound in a mineral acid or by catalytically reducing it in a solvent such as a lower alcohol, ethyl acetate, or the like in the presence of a palladium-carbon catalyst.

### Preparation Process 6:

Compound (X) can be prepared by reacting compound (VIII) with phosphorus oxychloride in the absence or presence of a hydrocarbon solvent such as toluene, xylene, benzene, or the like at 0 to 150°C, thus producing compound (IX), and then reducing the compound (IX) according to the following formula. 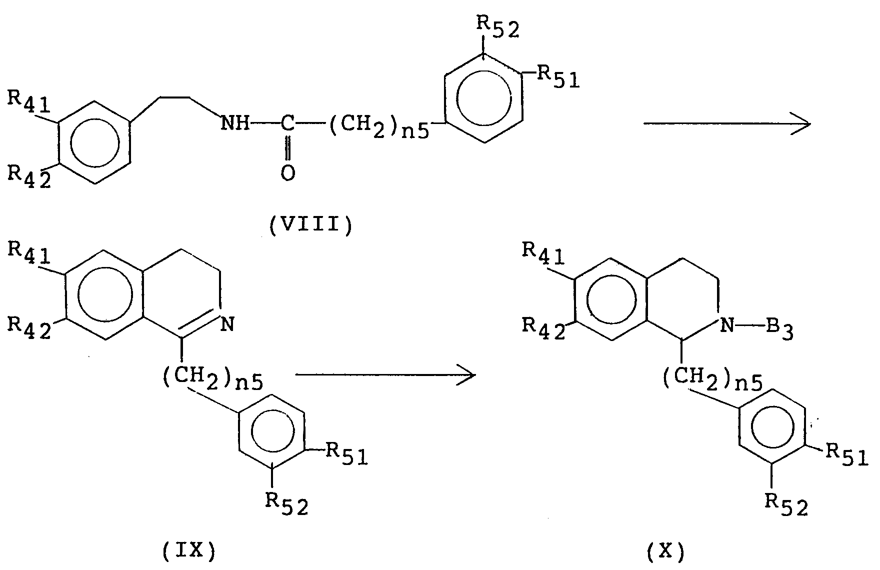
wherein R₄₁, R₄₂, R₅₁, and R₅₂ individually represent a hydrogen atom, a hydroxy group, or a lower alkoxy group; B₃ represents a hydrogen atom, a lower alkyl group, or a lower acyl group; and n5 denotes 0 or 1.

This reducing reaction can be carried out in air or an inert gas using a reducing agent of an amount equivalent to or greater than the amount of compound (IX) and at least one kind of organic solvent selected from the group consisting of lower alcohols, hydrocarbons, methylene chloride, chloroform, ether, tetrahydrofuran, and the like under atmospheric pressure or under a pressure. A metal hydride compound (e.g., diisobutyl aluminum hydride, an organic tin compound, hydrosilane, etc.), a metal hydride complex (e.g., lithium aluminum hydride, sodium borohydride, etc.), or a complex (e.g., boran, diboran, etc.) can be used as a reducing agent. The reaction temperature may be determined depending on the reagents used, normally within the range of -78°C to 150°C.

In the above-mentioned reactions, when one or more groups among R₁₁, R₁₂, R₂₁, R₂₂, R₃₁, R₃₂, R₄₁, R₄₂, R₅₁, and R₅₂ are groups which are reactive with amino group or carboxyl group, such as hydroxy group, it is desirable that the reaction be carried out after protecting such reactive groups. Conventionally known protective groups such as, for example, benzyl group and the like are given as the protective groups.

The phellodendrine analogs (I) of the present invention prepared by the above processes may be converted into non-toxic salts, if necessary.

The preparation of the non-toxic salts can be achieved by reacting the phellodendrine analog (I) and amino acid, potassium hydroxide, hydrogen chloride, citric acid, maleic acid, acetic acid, sodium methoxide, or the like in a solvent such as the aforementioned common organic solvent, water, or an alcohol, or in a mixture of these solvents, at 0 to 150°C.

In this instance, the salt thus produced must be a pharmaceutically acceptable salt.

Next, the pharmacological actions (the IV-type allergy suppressing effects) of phellodendrine analogs, including the phellodendrine analogs (I) prepared by the above processes, are illustrated by way of experimental examples.

### Experimental Example 1

A spleen cell suspension was subcutaneously transferred into right sole of CBF₁ mice (male, age: 7 weeks), F₁ of BALB/c mouse and C57BL/6 mouse, in an amount of 5 x 10⁶ or 7.5 x 10⁶ spleen cells per mouse. The intensity of the local graft versus host reaction was determined to evaluate the allergy type IV suppressing effects of the test compounds.

The spleen cell suspension was prepared by passing the spleen extracted from BALB/c mouse through a stainless wire mesh (200 mesh) in a Hanks' balanced salt solution.

The test compounds were orally administered at a concentration of 1.0 x 10⁻⁴ mol/kg or 3.0 x 10⁻⁴ mol/kg for eight days starting from the day on which the spleen cells were transferred.

The allergy type IV suppressing effects of the test compounds were evaluated by extracting right and left politeal lymph nodes nine days after the spleen cell transfer, measuring the wet weight, and subtracting the left lymph nodes weight from the right lymph nodes weight, thus calculating the intensity of the local graft versus host reaction. The rates of suppression against the control are presented in Tables 1 to 3.

**TABLE 1**

| Tested compound | Number of transferred cells | Concentration of test compound (mol/kg) | Rate of suppression (%) | Number of tested animals |
|---|---|---|---|---|
| Control 1* | 7.5 x 10⁶ | | | 6 |
| Compound of Synthetic Example 6 | 7.5 x10⁶ | 3.0 x 10⁻⁴ | 37.4 | 7 |
| Compound of Synthetic Example 20 | 7.5 x 10⁶ | 3.0 x 10⁻⁴ | 28.9 | 7 |
| Compound of Synthetic Example 30 | 7.5 x 10⁶ | 3.0 x 10⁻⁴ | 28.7 | 7 |
| Compound of Synthetic Example 46 | 7.5 x 10⁶ | 3.0 x 10⁻⁴ | 37.2 | 7 |
| Compound of Synthetic Example 60 | 7.5 x 10⁶ | 3.0 x 10⁻⁴ | 49.6 | 7 |
| Compound of Synthetic Example 61 | 7.5 x 10⁶ | 3.0 x 10⁻⁴ | 59.2 | 7 |
| Compound of Synthetic Example 64 | 7.5 x 10⁶ | 3.0 x 10⁻⁴ | 23.5 | 8 |
| Control 1 * | 5 x 10⁶ | | | 8 |
| Compound of Synthetic Example 1 | 5 x 10⁶ | 3.0 x 10⁻⁴ | 25.1 | 7 |
| Compound of Synthetic Example 14 | 5 x 10⁶ | 3.0 x 10⁻⁴ | 35.9 | 7 |
| Compound of Synthetic Example 19 | 5 x 10⁶ | 3.0 x 10⁻⁴ | 30.6 | 7 |
| Compound of Synthetic Example 20 | 5 x 10⁶ | 3.0 x 10⁻⁴ | 50.8 | 8 |
| Compound of Synthetic Example 21 | 5 x 10⁶ | 3.0 x 10⁻⁴ | 26.8 | 8 |
| Compound of Synthetic Example 27 | 5 x 10⁶ | 3.0 x 10⁻⁴ | 33.9 | 7 |
| Compound of Synthetic Example 29 | 5 x 10⁶ | 3.0 x 10⁻⁴ | 37.1 | 8 |
| Compound of Synthetic Example 30 | 5 x 10⁶ | 3.0 x 10⁻⁴ | 51.8 | 7 |
| Compound of Synthetic Example 31 | 5 x 10⁶ | 3.0 x 10⁻⁴ | 36.3 | 7 |
| Compound of Synthetic Example 35 | 5 x 10⁶ | 3.0 x 10⁻⁴ | 43.5 | 7 |
| Compound of Synthetic Example 46 | 5 x 10⁶ | 3.0 x 10⁻⁴ | 32.9 | 8 |
| Compound of Synthetic Example 47 | 5 x 10⁶ | 3.0 x 10⁻⁴ | 28.7 | 8 |
| Compound of Synthetic Example 51 | 5 x 10⁶ | 3.0 x 10⁻⁴ | 35.0 | 8 |
| Compound of Synthetic Example 55 | 5 x 10⁶ | 3.0 x 10⁻⁴ | 24.1 | 7 |

| | | | | |
|---|---|---|---|---|
| * Control 1 : 0.5% gum arabic-purified water | | | | |

**TABLE 2**

| Tested compound | Number of transferred cells | Concentration of test compound (mol/kg) | Rate of suppression (%) | Number of tested animals |
|---|---|---|---|---|
| Control 2 ** | 7.5 x 10⁶ | | | 8 |
| Compound of Synthetic Example 6 | 7.5 x 10⁶ | 1.0 x 10⁻⁴ | 30.1 | 8 |
| Compound of Synthetic Example 6 | 7.5 x 10⁶ | 3.0 x 10⁻⁴ | 32.9 | 8 |
| Compound of Synthetic Example 7 | 7.5 x 10⁶ | 3.0 x 10⁻⁴ | 27.0 | 8 |
| Compound of Synthetic Example 15 | 7.5 x 10⁶ | 3.0 x 10⁻⁴ | 22.3 | 8 |
| Compound of Synthetic Example 25 | 7.5 x 10⁶ | 1.0 x 10⁻⁴ | 38.1 | 8 |
| Compound of Synthetic Example 25 | 7.5 x 10⁶ | 3.0 x 10⁻⁴ | 48.6 | 8 |
| Compound of Synthetic Example 30 | 7.5 x 10⁶ | 3.0 x 10⁻⁴ | 33.7 | 8 |
| Compound of Synthetic Example 36 | 7.5 x 10⁶ | 3.0 x 10⁻⁴ | 29.2 | 6 |
| Control 2 ** | 5 x 10⁶ | | | 7 |
| Compound of Synthetic Example 6 | 5 x 10⁶ | 3.0 x 10⁻⁴ | 29.0 | 7 |
| Compound of Synthetic Example 7 | 5 x 10⁶ | 3.0 x 10⁻⁴ | 37.2 | 8 |
| Compound of Synthetic Example 25 | 5 x 10⁶ | 3.0 x 10⁻⁴ | 38.5 | 8 |
| Compound of Synthetic Example 36 | 5 x 10⁶ | 3.0 x 10⁻⁴ | 27.8 | 8 |

| | | | | |
|---|---|---|---|---|
| ** Control 2 : 5% ethanol-olive oil | | | | |

**TABLE 3**

| Tested compound | Number of transferred cells | Concentration of test compound (mol/kg) | Rate of suppression (%) | Number of tested animals |
|---|---|---|---|---|
| Control 3⁺ | 7.5 x 10⁶ | | | 8 |
| Compound of Synthetic Example 7 | 7.5 x 10⁶ | 1.0 x 10⁻⁴ | 23.9 | 8 |
| Compound of Synthetic Example 7 | 7.5 x 10⁶ | 3.0 x 10⁻⁴ | 37.1 | 8 |
| Compound of Synthetic Example 27 | 7.5 x 10⁶ | 3.0 x 10⁻⁴ | 34.3 | 8 |
| Control 4 ++ | 7.5 x 10⁶ | | | 8 |
| Compound of Synthetic Example 62 | 7.5 x 10⁶ | 3.0 x 10⁻⁴ | 50.8 | 8 |

| | | | | |
|---|---|---|---|---|
| + Control 3 : Purified water | | | | |
| ++ Control 4 : Olive oil | | | | |

### Experimental Example 2

The actions of the compounds of the present invention in the course of creation of the cellular immunity were studied using the sole reaction in sheep red blood cells (SRBC).

BALB/c mice (female, age: 7 weeks) were sensitized by injecting 0.2 ml of SRBC adjusted to 2.5 x 10⁵ cells/ml with a physiological saline solution into caudal vein. Four days after the sensitization, the volume of the right foot of the mice was measured. Then, 0.025 ml of SRBC from the same lot as above adjusted to 4 x 10⁹ cells/ml was subcutaneously injected into the right sole to induce a reaction. 24 hours after the reaction has been induced, the intensity of the reaction was determined by subtracting the right foot volume before the reaction from the volume after the reaction. The rate of suppression against the control is shown in Table 4. The test compounds at a concentration of 3.0 x 10⁻⁴ mol/kg were orally administered for four days after the sensitization.

**TABLE 4**

| Tested compound | Rate of suppression (%) | Number of tested animals |
|---|---|---|
| Control 1 * | | 8 |
| Compound of Synthetic Example 46 | 38.1 | 7 |
| Control 2 ** | | 8 |
| Compound of Synthetic Example 25 | 26.7 | 7 |
| Compound of Synthetic Example 30 | 33.2 | 8 |
| Compound of Synthetic Example 61 | 21.8 | 7 |
| Control 3 + | | 7 |
| Compound of Synthetic Example 7 | 54.4 | 6 |
| Compound of Synthetic Example 27 | 32.8 | 7 |

| | | |
|---|---|---|
| * Control 1 : 0.5% gum arabic-purified water | | |
| ** Control 2 : 5% ethanol-olive oil | | |
| + Control 3 : Purified water | | |

### Experimental Example 3

Compounds obtained in Synthetic Examples 18, 35, and 66 were orally administered to ICR mice (each dose to each group consisting of 10 mice). It was found that no death was caused in animals up to a dose of 1,000 mg/kg, evidencing that the compounds of the present invention have low toxicity and are highly safe.

As a result of the above experiments, the compounds of the present invention were confirmed to exhibit the allergy type IV suppressing actions and to be highly safe.

Because the phellodendrine analogs of the present invention exhibits actions as mentioned above, they can be administered as are or together with commonly used carriers to animals and human as an allergy type IV suppressor.

Phellodendrine analogs which can be used as allergy type IV suppressors are compounds represented by the following formula (I'), 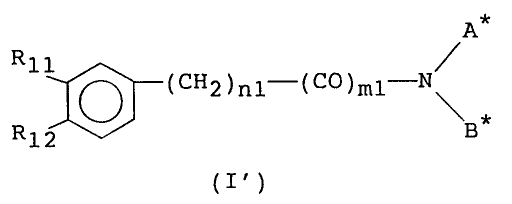
wherein R₁₁, R₁₂, n1, and m1 have the same meanings as previously defined; A* represents a group, 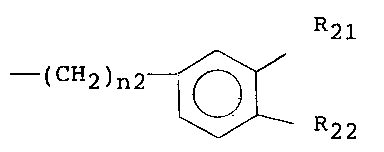
wherein R₂₁ and R₂₂ individually represent a hydrogen atom, a hydroxy group, or a lower alkoxy group, and n2 is 1 or 2; B* represents a hydrogen atom, a lower alkyl group, or a lower acyl group, or A* and B* may form, together with the adjacent nitrogen atom, a substituted 1,2,3,4-tetrahydroisoquinolyl ring represented by the following formula, 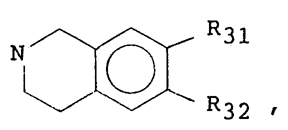
wherein R₃₁ and R₃₂ individually represent a hydrogen atom, a hydroxy group, or a lower alkoxy group. Most of the compounds represented by the above formula (I') are included in said phellodendrine analogs (I), while some of them are known compounds. However, these known compounds have not been known at all to exhibit the excellent allergy IV type suppressing actions.

Furthermore, the present inventors have found that compounds represented by the following formula, which are related to said phellodendrine analogs (Ic), also exhibit excellent allergy type IV suppressing actions. 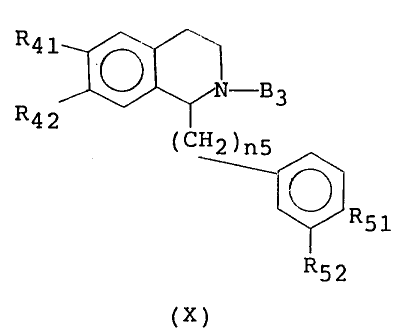
wherein R₄₁, R₄₂, R₅₁, and R₅₂ individually represent a hydrogen atom, a hydroxy group, or a lower alkoxy group; B₃ represents a hydrogen atom, a lower alkyl group, or a lower acyl group; and n5 denotes 1 or 0.

There are no specific limitations as to the form of the phellodendrine analogs of formula (I') or (X), when they are administered as allergy type IV suppressor. Depending on the requirement, this form may be suitably selected from tablets, capsules, granules, fine particles, powders, or the like for oral administration or for parenteral administration, such as injection, suppository, or the like.

In order to exhibit a desired effect as the allergy type IV suppressor by oral administration, a dose for an adult of 10 mg to 6 g, as a phellodendrine analog, per day, divided several times a day, would be normally appropriate, although the amount varies depending on the age, weight, and degree of the disease of the patient.

The drug preparations for the oral administration can be prepared by a conventional method using, for example, starch, lactose, white sugar, mannitol, carboxymethyl cellulose, corn starch, inorganic salts, and the like.

In addition to said vehicles, binders, disintegrators, surfactants, lubricants, fluidity enhancers, sweeteners, colorants, perfumes, and the like may be used as appropriate for this type of drug preparations. Specific examples of these additives are as follows.

### [Binders]

Starch, dextrin, gum arabic powder, gelatin, hydroxypropyl starch, methyl cellulose, sodium carboxymethyl cellulose, hydroxypropyl cellulose, crystalline cellulose, ethyl cellulose, polyvinylpyrrolidone, macrogol.

### [Disintegrators]

Starch, hydroxypropyl starch, sodium carboxymethyl cellulose, calcium carboxymethyl cellulose, carboxymethyl cellulose, low-substituted hydroxypropyl cellulose.

### [Surfactants]

Sodium lauryl sulfate, soybean lecithin, sucrose fatty acid ester, polysorbate 80.

### [Lubricants]

Talc, waxes, hydrogenated vegetable oils, sucrose fatty acid ester, magnesium stearate, aluminum stearate, polyethylene glycol.

### [Fluidity enhancers]

Light anhydrous silicic acid, dry aluminum hydroxide gel, synthetic aluminum silicate, magnesium silicate.

Furthermore, the phellodendrine analogs of the present invention may be administered as a suspension, an emulsion preparation, a syrup preparation, an elixir preparation, or the like. These preparations may contain sweeteners, corrigants, colorants, and the like.

On the other hand, in order to exhibit a desired effect as an allergy type IV suppressor by parenteral administration, administration of 0.5 mg to 200 mg, as a phellodendrine analog, per day for an adult by means of intravenous injection, intravenous drip infusion, subcutaneous injection, intramuscular injection, would be normally appropriate, although the amount varies depending on the age, weight, and degree of the disease of the patient.

The preparations for parenteral administration can be also prepared by a conventional method using distilled water for injection, a physiological saline solution, a glucose aqueous solution, vegetable oil for injection, sesame oil, peanut oil, soybean oil, corn oil, propylene glycol, polyethylene glycol, or the like as a diluent. In addition, an antibacterial agent, a preservative, and a stabilizer may be added as needed. Furthermore, from the aspect of the stability, the parenteral preparation may be filled in vials or the like and frozen, and water is removed therefrom by a conventional lyophilizing technique. A liquid preparation can be prepared from the lyophilized product immediately before use. If required, it is further possible to add an isotonic agent, a stabilizer, a preservative, a soothing agent, and the like as appropriate.

Preparations in the form of liquid or ointment for external application, suppositories for rectal application, and the like are given as other parenteral agent. They can be prepared also according to conventional methods.

### Examples

The present invention will now be described in more detail by way of Synthetic Examples and Preparation Examples.

### Synthetic Example 1

19 ml of m-methoxybenzylamine, 100 ml of benzene, and 14 ml of pyridine were placed in a 500 ml three-necked flask. A solution of 25 g of m-anisoyl chloride in 25 ml of benzene was added dropwise under cooling with ice and the mixture was stirred for 30 minutes. The reaction temperature was maintained the same as the room temperature and the stirring was continued for a further 16 hours.

After evaporating the solvent, the residue was extracted by ether, washed with water, 1 N hydrochloric acid, water, 5% sodium hydroxide, and then water, and dried over anhydrous sodium sulfate. The solvent was evaporated, and the residue was washed with n-hexane and dried, thus obtaining 37.6 g (94.3%) of the following compound. The compound was recrystallized from a chloroform/hexane mixed solvent (cooled to -20°C).
N-(3-methoxybenzyl)-3-methoxybenzamide
m.p. (°C): 60-61
IR νmax (KBr) cm⁻¹ : 3332, 3068, 3004, 2964, 2932, 2836, 1638, 1612, 1584, 1538, 1488, 1454, 1434, 1326, 1306, 1290, 1264, 1236
¹H-NMR (δ ppm in CDCl₃): 3.80 (3H, s), 3.85 (3H, s), 4.61 (2H, d, J=5.6Hz), 6.39 (1H, t, J=5.6Hz), 6.81-7.06 (4H, m), 7.24-7.40 (4H, m)
MS m/z (%) [EI]: 271 (M⁺, 87), 136 (100)

| EA (as C₁₆H₁₇NO₃): | | | |
|---|---|---|---|
| Calculated | C: 70.83 | H: 6.32 | N: 5.16 |
| Found | C: 70.81 | H: 6.32 | N: 5.44 |

### Synthetic Example 2

A mixture of 4.0 g of N-(3-methoxybenzyl)-3-methoxybenzamide and 75 ml of methylene chloride was cooled by ice, and 75 ml of a 0.8 M boron tribromide solution in methylene chloride was added to the mixture. After stirring for 1.45 hours at room temperature, the mixture was charged into ice water to collect deposited crystals by filtration. The crystals were washed with water and dried to obtain 2.63 g of crystals. The filtrate was extracted with ethyl acetate, washed with water, and dried, then the solvent was removed by evaporation to obtain 0.65 g of a residue. The following compound was obtained from the crystals and the residue at a total yield of 3.28 g (91.3%). The compound was recrystallised from a n-hexane/ethyl acetate mixed solvent. Colorless needle-like crystals.
N-(3-hydroxybenzyl)-3-hydroxybenzamide
m.p. (°C): 132-133
IR νmax (KBr) cm⁻¹ : 3436, 3372, 3138, 2936, 3188, 2936, 1626, 1604, 1588, 1538, 1460, 1432, 1366, 1338, 1314, 1280, 1248, 1228
¹H-NMR (δ ppm in DMSO-d₆): 4.37 (2H, d, J=6.1Hz), 6.59-7.34 (8H, m), 8.87 (1H, t, J=6.1Hz), 9.5 (2H, bs)
MS m/z (%) [EI]: 243(M⁺, 54), 122(82), 121(100)

| EA (as (C₁₄H₁₃NO₃): | | | |
|---|---|---|---|
| Calculated | C: 69.12 | H: 5.39 | N: 5.76 |
| Found | C: 70.81 | H: 5.92 | N: 5.98 |

### Synthetic Example 3

A mixture of 18.13 g of N-(3-methoxybenzyl)-3-methoxybenzamide and 100 ml of tetrahydrofuran was cooled with ice. To this was added dropwise 150 ml of a 1 M boran/tetrahydrofuran complex solution, and the mixture was stirred, then heated for 1 hour while refluxing. After stirring for 2 days at room temperature, the mixture was cooled to 0°C and 20 ml of 6 N hydrochloric acid was carefully added.

The reaction mixture was allowed to rise to room temperature, heated for 50 minutes while refluxing, cooled to 0°C, and neutralized with sodium hydroxide, followed by stirring for 1 hour. The solvent was evaporated. After addition of water, the residue was extracted with ether, washed with water, and dried. The solvent was evaporated to obtain 17.02 g (99%) of the following compound as an oily substance. A portion of this product was dissolved into ether and 10% hydrochloric acid-methanol was added to it to obtain the hydrochloride (white crystals). Maleic acid salt was also obtained by a conventional method.
N,N-bis(3-methoxybenzyl)amine
Property: oil
IR νmax (KBr) cm⁻¹ : 3350, 1600, 1586, 1488, 1464, 1436, 1358, 1314, 1266, 1190, 1154, 1114, 1084
¹H-NMR (δ ppm in CDCl₃): 3.79 (4H, s), 3.81 (6H, s), 6.79-6.94 (6H, m), 7.20-7.29 (2H, m)
MS m/z (%) [EI]: 257 (M⁺, 13), 121 (100)

| MS [HMS] (as C₁₆H₁₉NO₂): | |
|---|---|
| Calculated | 257.14150 |
| Found | 257.14150 |

N,N-bis(3-methoxybenzyl)amine hydrochloride
m.p. (°C): 136-138
IR νmax (KBr) cm⁻¹ 2956, 2936, 2832, 2764, 2688, 2612, 2432, 1604, 1588, 1492, 1462, 1450, 1426, 1368, 1328, 1300, 1278, 1268, 1254, 1192, 1176, 1164, 1064
¹H-NMR (δ ppm in CD₃OD): 3.82 (6H, s), 4.19 (4H, s), 6.97-7.06 (6H, m), 7.33-7.41 (2H, m)
MS m/z (%) [FD]: 257(M⁺-HCl)

| EA (as C₁₆H₁₉NO₂·HCl): | | | |
|---|---|---|---|
| Calculated | C: 65.41 | H: 6.86 | N: 4.78 |
| Found | C: 65.30 | H: 6.93 | N: 4.98 |

N,N-bis(3-methoxybenzyl)amine maleate
m.p. (°C): 147-149
IR νmax (KBr) cm⁻¹ : 2964, 2836, 1588, 1494, 1366, 1266, 1160, 1036, 992
¹H-NMR (δ ppm in DMSO-d₆): 3.78 (6H, s), 4.14 (4H, s), 6.02 (2H, s), 6.96-7.08 (6H, m), 7.32-7.40 (2H, m)

| EA (as C₁₆H₁₉NO₂·C₄H₄O₄): | | | |
|---|---|---|---|
| Calculated | C: 64.33 | H: 6.21 | N: 3.75 |
| Found | C: 64.45 | H: 6.37 | N: 3.94 |

### Synthetic Example 4

A mixture of 6.33 g of N,N-bis(3-methoxybenzyl)-amine, 3 ml of pyridine, and 3 ml of acetic anhydride was stirred for 3 hours at room temperature.

The reaction mixture was charged into water and extracted with ether. The extract was washed with water, 1 N hydrochloric acid, water, 10% sodium hydroxide, and then water, and dried. The solvent was evaporated to obtain 6.61 g of a residue. The residue was purified by flash column chromatography (silica gel, 230-400 mesh; eluent, n-hexane:ethyl acetate = 8:7), thus obtaining 5.85 g (79.4%) of the following compound as a colorless oil.
N,N-bis(3-methoxbenzyl)acetamide
IR νmax (KBr) cm⁻¹ : 3000, 2940, 2836, 1650, 1602, 1588, 1490, 1466, 1420, 1346, 1284, 1262, 1240, 1192, 1154, 1044
¹H-NMR (δ ppm in DMSO-d₆): 1.99 (3H, s), 3.72 (6H, s), 4.46 (2H, s), 4.48 (2H, s), 6.7-6.87 (6H, m), 7.19-7.32 (2H, m)
MS m/z (%) [EI]: 299(M⁺, 13), 178(55), 136(100)

| MS [HMS] (as C₁₈H₂₁NO₃ (M⁺)): | |
|---|---|
| Calculated | 299.15203 |
| Found | 299.15203 |

### Synthetic Example 5

A mixture of 373 mg of N-(3-methoxybenzyl)-3-methoxybenzamide, 1 ml of dimethylsulfoxide, and 2.42 g of potassium hydroxide was cooled and stirred. To the mixture was added 0.11 ml of methyl iodide, followed by stirring for 20 minutes. After stirring for a further 40 minutes at room temperature, water was added to the reaction mixture. The mixture was then extracted with ether, washed with water, and dried. The solvent was evaporated to obtain 340 mg (86.6%) of the following compound as an oily product.
N-methyl-N-(3-methoxybenzyl)-3-methoxybenzamide
IR νmax (KBr) cm⁻¹ : 3560, 3000, 2940, 2836, 1632, 1602, 1586, 1490, 1466, 1434, 1402, 1352, 1288, 1266, 1204, 1184, 1154, 1068, 1044
¹H-NMR (δ ppm in DMSO-d₆, at 80°C): 3.05 (3H, s), 3.75 (6H, s), 4.54 (2H, s), 6.81-7.00 (6H, m), 7.23-7.37 (2H, m)
MS m/z (%) [EI]: 285 (M⁺, 87), 150 (68), 135 (100)

| MS [HMS] (as C₁₇H₁₉NO₃ (M⁺)): | |
|---|---|
| Calculated | 285.13605 |
| Found | 285.13625 |

### Synthetic Example 6

A mixture of 4.47 g of N-methyl-N-(3-methoxybenzyl)-3-methoxybenzamide and 70 ml of methylene chloride was cooled with ice. To this was added 67 ml of a 1.0 M boron tribromide solution in methylene chloride, and the mixture was stirred for 16 hours at room temperature. After charging into ice water, the mixture was extracted with ethyl acetate, washed with water, and dried. The solvent was evaporated and the residue was purified by flash column chromatography (silica gel, 230-400 mesh; eluent, ethyl acetate), thus obtaining 3.4 g (76.4%) of the following compound.
N-methyl-N-(3-hydroxybenzyl)-3-hydroxybenzamide
IR νmax (KBr) cm⁻¹ : 3272 (br), 1586, 1512, 1480, 1454, 1408, 1368, 1280, 1236, 1156, 1072, 1042
¹H-NMR (δ ppm in DMSO-d₆, at 80°C): 2.83 (3H, s), 4.48 (2H, s), 6.65-6.84 (6H, m), 7.09-7.24 (2H, m)
MS m/s (%) [EI]: 257 (M⁺, 69), 256 (52), 136 (47), 121 (100)

| MS [HMS] (as C₁₅H₁₅NO₃ (M⁺)): | |
|---|---|
| Calculated | 257.10514 |
| Found | 257.10504 |

### Synthetic Example 7

A mixture of 3.62 g of N,N-bis(3-methoxybenzyl)amine and 60 ml of methylene chloride was cooled with ice. To the mixture was added 60 ml of a 1.0 M methylene chloride solution of boron tribromide. After stirring for 1.5 hours at room temperature, the mixture was charged into ice water, followed by stirring for a further 16 hours. The reaction mixture was neutralized with sodium bicarbonate, extracted with ethyl acetate, washed with water, and dried. The solvent was evaporated and the residue was purified by flash column chromatography (silica gel, 230-400 mesh; eluent, chloroform:methanol = 1:1), thus obtaining 2.74 g (78%) of the following compound as an oily product.
N,N-bis(3-hydroxybenzyl)amine
IR νmax (KBr) cm⁻¹ : 3300-2600 (br), 1590, 1484, 1462, 1360, 1314, 1272, 1158, 1072
¹H-NMR (δ ppm in DMSO-d₆, at 80°C): 3.60 (4H, s), 6.59-6.76 (6H, m), 7.05-7.13 (2H, m)
MS m/z (%) [EI]: 229 (M⁺, 5), 228 (5), 123 (100)

| MS [HMS] (as C₁₄H₁₅NO₂ (M⁺)): | |
|---|---|
| Calculated | 229.11035 |
| Found | 229.11085 |

### Synthetic Example 8

A mixture of 7.71 g of N,N-bis(3-methoxybenzyl)amine, 10 ml of methanol, and 3 ml of 35% formalin was stirred for 30 minutes at room temperature. The solution was charged into a methanol suspension containing 800 mg of 5% palladium-carbon to carry out hydrogenation. The reaction mixture was filtered through cerite, the filtrate was concentrated, and the residue was purified by flash column chromatography (silica gel, 230-400 mesh; eluent, chloroform), thus obtaining 7.74 g (95%) of the following N-methyl-N,N-bis(3-methoxybenzyl)amine as an oily product.

7.49 g of the amine thus obtained was dissolved into 50 ml of ether and the solution was added to an ether solution containing 3.61 g of maleic acid. Deposited crystals were collected by filtration, washed with ether, and dried, thus obtaining 8.69 g of maleic acid salt.
N-methyl-N,N-bis(3-methoxybenzyl)amine
IR νmax (KBr) cm⁻¹ : 2996, 2944, 2836, 2784, 2708, 1602, 1586, 1488, 1454, 1436, 1418, 1364, 1338, 1314, 1266, 1190, 1152, 1078, 1046
¹H-NMR (δ ppm in CDCl₃): 2.20 (3H, s), 3.50 (4H, s), 3.81 (6H, s), 6.75-7.96 (6H, m), 7.19-7.27 (2H, m)
MS m/z (%) [EI]: 271 (M⁺, 30), 150 (87), 122 (99), 121 (100)

| MS [HMS] (as C₁₇H₂₁NO₂ (M⁺)): | |
|---|---|
| Calculated | 271.15712 |
| Found | 271.15712 |

N-methyl-N,N-bis(3-methoxybenzyl)amine maleate
m.p. (°C) : 140-144
IR νmax (KBr) cm⁻¹ : 3040, 3008, 2964, 2840, 2724, 2664, 2548, 2412, 1612, 1588, 1480, 1440, 1416, 1380, 1358, 1302, 1288, 1266, 1238, 1198, 1164, 1094, 1072, 1050
¹H-NMR (δ ppm in DMSO-d₆): 2.49 (3H, s), 3.79 (6H, s), 4.15 (4H, bs), 6.07 (2H, s), 6.99-7.07 (6H, m), 7.34-7.42 (2H, m)
MS m/z (%) (FD]: 272 (M⁺, 1)

| EA (as C₁₇H₂₁NO₂): | | | |
|---|---|---|---|
| Calculated | C: 65.10 | H: 6.5 | N: 3.62 |
| Found | C: 65.26 | H: 6.6 | N: 3.85 |

### Synthetic Example 9

A mixture of 3.62 g of N-methyl-N,N-bis(3-methoxybenzyl)amine and 60 ml of methylene chloride was cooled with ice. To the mixture was added 60 ml of a 1.0 M methylene chloride solution of boron tribromide. After stirring for 3 hours at room temperature, the mixture was charged into ice water, extracted with ethyl acetate, washed with water, and dried. The solvent was evaporated and 20 ml of 6 N hydrochloric acid was added to the residue, followed by stirring for one hour at 0°C. The residue was allowed to stand at room temperature, neutralized with the addition of sodium bicarbonate, extracted with ethyl acetate, washed with water, and dried. The solvent was evaporated and the residue was purified by flash column chromatography (silica gel, 230-400 mesh; eluent, chloroform:methanol = 1:1), thus obtaining 2.77 g (82%) of the following compound as a colorless oily product.
N-methyl-bis(3-hydroxybenzyl)amine
IR νmax (KBr) cm⁻¹ : 3040, 3008, 2964, 2840, 2724, 2664, 2548, 2412, 1612, 1588, 1480, 1440, 1416, 1380, 1358, 1302, 1288, 1266, 1238, 1198, 1164, 1094, 1072, 1050
¹H-NMR (δ ppm in DMSO-d₆): 2.06 (3H, s), 3.38 (4H, s), 6.61-6.78 (6H, m), 7.10-7.14 (2H, m)
MS m/z (%) [EI]: 243 (M⁺, 29), 150 (32), 136 (93), 108 (41), 107 (100)

| HMS (as C₁₅H₁₇NO₂ (M⁺)): | |
|---|---|
| Calculated | 243.12594 |
| Found | 243.12614 |

### Synthetic Example 10

A mixture of 4.13 g of N,N-bis(3-methoxybenzyl)acetamide and 60 ml of methylene chloride was cooled with ice. To the mixture was added 60 ml of a 1.0 M methylene chloride solution of boron tribromide. After stirring for 3 hours at room temperature, the mixture was charged into ice water, extracted with ethyl acetate, washed with water, and dried. The solvent was evaporated and the residue was purified by flash column chromatography (silica gel, 230-400 mesh; eluent: ethyl acetate), thus obtaining 3.35 g (87.5%) of the following compound as a colorless oily product.
N,N-bis(3-hydroxybenzyl)acetamide
IR νmax (KBr) cm⁻¹ : 3300 (br), 1600, 1486, 1454, 1366, 1328, 1258, 1156
¹H-NMR (δ ppm in DMSO-d₆): 2.08 (3H, s), 4.38 (4H, s), 6.59-6.69 (6H, m), 7.06-7.12 (2H, m)
MS m/z (%) [EI]: 271 (M⁺, 19), 164 (54), 122 (100)

| HMS (as C₁₆H₁₇NO₃ (M⁺)): | |
|---|---|
| Calculated | 271.12087 |
| Found | 271.12157 |

### Synthetic Example 11

(1) 65.2 g of 4-benzyloxy-3-methoxybenzyl alcohol, 200 ml of benzene, 25 ml of pyridine, and 22 ml of thionyl chloride were successively charged into a 500 ml eggplant-type flask, and the mixture was heated at 60°C for 1 hour while stirring. The reaction mixture was charged into water and the deposited crystals were collected by filtration, washed with water, and dried. To this product, without being purified, 48 g of potassium cyanide and 200 ml of dimethylformamide were added, and the mixture was heated at 60°C for 25 hours while stirring. The reaction mixture was charged into water and the deposited crystals were collected by filtration, washed with water, and dried. 49 g (72%) of the following compound was obtained by recrystallization from n-hexane. Colorless needle-like crystals.
4-Benzyloxy-3-methoxyphenylacetonitrile
m.p. (°C): 70-71
IR νmax (KBr) cm⁻¹ : 3060, 3023, 3012, 2976, 2940, 2928, 2880, 2840, 2248, 1608, 1590, 1512, 1466, 1452, 1422, 1384, 1342, 1298, 1250, 1226, 1188, 1142, 1034, 1004
¹H-NMR (δ ppm in CDCl₃): 3.67 (2H, s), 3.90 (3H, s), 5.15 (2H, s), 6.75-6.88 (3H, m), 7.25-7.45 (5H, m)
MS m/z (%) [EI]: 253 (M⁺, 10), 91 (100)

| MS [HMS] (as C₁₆H₁₅NO₂): | |
|---|---|
| Calculated | 253.10124 |
| Found | 253.10944 |

(2) 37 g of 4-benzyloxy-3-methoxyphenylacetonitrile, 600 ml of 10 N sodium hydroxide, and 600 ml of ethanol were charged into a 2 l eggplant-type flask, and the mixture was heated under refluxing for 5 hours. The reaction mixture was charged into ice water and concentrated hydrochloric acid was added to it. After allowing to stand overnight, the deposited crystals were collected by filtration, washed with water, and dried, thus obtaining 39 g (97%) of the following compound. Recrystallized from a mixed solvent of water and methanol. Colorless needle-like crystals.
4-Benzyloxy-3-methoxyphenylacetate
m.p. (°C): 119-121
IR νmax (KBr) cm⁻¹ : 3028-2544 (br), 1712, 1610, 1594, 1512, 1464, 1408, 1386, 1334, 1266, 1218, 1190, 1166, 1140, 1032, 1014
¹H-NMR (δ ppm in CDCl₃): 3.57 (2H, s), 3.87 (3H, s), 5.13 (2H, s), 6.74 (1H, dd, J=8.3Hz, 1.7Hz), 6.82 (1H, d, J=1.7Hz), 6.83 (1H, d, J=8.3Hz), 7.26-7.50 (5H, m)
MS m/z (%) [EI]: 272 (M⁺, 6), 91 (100)

| MS [HMS] (as C₁₆H₁₆O₄): | |
|---|---|
| Calculated | 272.10481 |
| Found | 272.10451 |

(3) 30 g of 4-benzyloxy-3-methoxyphenylacetic acid, 150 ml of benzene, 20 ml of thionyl chloride, and one drop of dimethylformamide were successively charged into a 500 ml eggplant-type flask, and the mixture was heated at 60°C for 30 minutes while stirring. The solvent and an excessive amount of thionyl chloride were evaporated and the residue was dissolved into 150 ml of benzene. This solution was added dropwise to 25 g of 4-benzyloxy-3-methoxybenzylamine dissolved in a mixed solvent of 300 ml of 10% aqueous solution of sodium hydroxide and 200 ml of benzene, followed by stirring for 1 hour. The solvent was evaporated, and the residue was washed with water and dried, thus obtaining 43 g (85%) of colorless needle-like crystals of the following compound. The compound was recrystallized from acetone.
N-(4-benzyloxy-3-methoxybenzyl)-4-benzyloxy-3-methoxyphenylacetamide
m.p. (°C): 152-154
IR νmax (KBr) cm⁻¹ : 3312, 3064, 3036, 3008, 2936, 2904, 2860, 1642, 1606, 1592, 1538, 1512, 1464, 1454, 1418, 1378, 1338, 1318, 1258, 1230, 1190, 1174, 1156, 1136, 1026, 1010
¹H-NMR (δ ppm in CDCl₃): 3.53 (2H, s), 3.81 (3H, s), 3.84 (3H, s), 4.32 (2H, d, J=5.9Hz), 5.12 (4H, s), 5.66 (1H, t, J=5.9Hz), 6.60-6.85 (6H, m), 7.25-7.44 (10H, m)
MS m/z (%) [EI]: 497 (M⁺, 1), 91 (100)

| MS [HMS] (as C₃₁H₃₁NO₅): | |
|---|---|
| Calculated | 497.22013 |
| Found | 497.21973 |

(4) To a suspension prepared in advance by adding 10 ml of ethanol to 100 mg of 5% palladium-carbon and by absorbing hydrogen, 5.03 g of N-(4-benzyloxy-3-methoxybenzyl)-4-benzyloxy-3-methoxyphenylacetamide and 150 ml of ethanol were added to carry out hydrogenation. The reaction mixture was collected by filtration and the solvent was evaporated from the residue. The residue was purified by flash column chromatography (silica gel, 230-400 mesh; eluent: chloroform:methanol = 20:1), thus obtaining 3.1 g (98%) of the following compound. The compound was recrystallized from ethyl acetate to obtain colorless prismatic crystals.
N-(4-hydroxy-3-methoxybenzyl)-4-hydroxy-3-methoxyphenyl-acetamide
m.p. (°C): 113.5-115
IR νmax (KBr) cm⁻¹ : 3356-2840 (br), 1616, 1598, 1515, 1466, 1454, 1432, 1410, 1382, 1350, 1320, 1278, 1260, 1242, 1218, 1192, 1158, 1126, 1052, 1030
¹H-NMR (δ ppm in CDCl₃): 3.32 (2H, s), 3.67 (3H, s), 3.72 (3H, s), 4.14 (2H, d, J=5.6Hz), 6.59-6.84 (6H, m), 8.31 (1H, t, J=5.6Hz)
MS m/z (%) [EI]: 317 (M⁺, 23), 137 (100)

| MS [HMS] (as C₁₇H₁₉NO₅): | |
|---|---|
| Calculated | 317.12623 |
| Found | 317.12503 |

### Synthetic Example 12

(1) 9.95 g of N-(4-benzyloxy-3-methoxybenzyl)-4-benzyloxy-3-methoxyphenylacetamide prepared in Synthetic Example 11, 3.0 g of potassium hydroxide powder, and 100 ml of dimethylsulfoxide were charged into a 300 ml eggplant-type flask. 3.4 ml of methyl iodide was added dropwise to the mixture under cooling with ice, followed by stirring for 30 minutes. The mixture was stirred for a further 16 hours at room temperature. The reaction mixture was charged into water and extracted with chloroform. The extract was washed with water and dried over anhydrous sodium sulfate. The solvent was evaporated and the residue was purified by flash column chromatography (silica gel, 230-400 mesh; eluent: n-hexane:ethyl acetate = 1:1), thus obtaining 2.11 g of the following compound. The compound was recrystallized from ethanol to obtain colorless flaky crystals.
N-(4-benzyloxy-3-methoxybenzyl)-N-methyl-4-benzyloxy-3-methoxyphenylacetamide
m.p. (°C): 74.5-76
IR νmax (KBr) cm⁻¹ : 3032, 3000, 2936, 2872, 1640, 1606, 1590, 1512, 1454, 1418, 1398, 1264, 1228, 1138, 1030
¹H-NMR (δ ppm in DMSO-d₆): 2.88 (3H, brs), 3.66 (2H, s), 3.70 (3H, s), 3.73 (3H, s), 4.46 (2H, s), 5.04 (4H, s), 6.67-6.97 (6H, m), 7.25-7.46 (10H, m)
MS m/z (%) [EI]: 511 (M⁺, 10), 91 (100)

| MS [HMS] (as C₃₂H₃₃NO₅): | |
|---|---|
| Calculated | 511.23584 |
| Found | 511.23614 |

(2) To a suspension prepared in advance by adding 10 ml of ethanol to 100 mg of 5% palladium-carbon and by absorbing hydrogen, 1.03 g of N-(4-benzyloxy-3-methoxybenzyl)-N-methyl-4-benzyloxy-3-methoxyphenylacetamide, 150 ml of ethanol, and 150 ml of ethyl acatate were added to carry out hydrogenation. The reaction mixture was collected by filtration and the solvent was evaporated from the residue, thus obtaining 632 mg (95%) of the following compound as colorless syrup oil.
N-(4-hydroxy-3-methoxybenzyl)-N-methyl-4-hydroxy-3-methoxyphenylacetamide
IR νmax (KBr) cm⁻¹ : 3396-2840 (br), 1622, 1598, 1514, 1452, 1432, 1404, 1372, 1276, 1238, 1214, 1150, 1124, 1032
¹H-NMR (δ ppm in DMSO-d₆): 2.83 (3H, brs), 3.61 (2H, s), 3.68 (3H, s), 3.71 (3H, s), 4.41 (2H, s), 6.61-6.79 (6H, m)
MS m/z (%) [EI]: 331 (M⁺, 33), 137 (100)

| MS [HMS] (as C₁₈H₂₁NO₅): | |
|---|---|
| Calculated | 331.14198 |
| Found | 331.14288 |

### Synthetic Example 13

(1) A mixture of 7.1 g of 3-hydroxy-4-methoxybenzaldehyde, 4.6 g of hydroxylamine hydrochloride, 7 g of sodium acetate, and 70 ml of formic acid was heated under refluxing for 1.5 hours. After allowing the mixture to cool down to room temperature, water was added to collect precipitate by filtration. The precipitate was washed with water and dried. To this were added 6.6 g of potassium carbonate, 30 ml of dimethylformamide, and 5.5 ml of benzyl bromide, and the mixture was stirred at room temperature. The reaction mixture was charged into water to collect deposited crystals by filtration. The crystals were washed with water, dried, and recrystallized from n-hexane, thus obtaining 8.7 g (78%) of the following compound.
3-Benzyloxy-4-methoxybenzonitrile
m.p. (°C): 74-76
IR νmax (KBr) cm⁻¹ : 2968, 2936, 2872, 2840, 2220, 1600, 1582, 1512, 1454, 1438, 1416, 1386, 1332, 1270, 1240, 1220, 1158, 1134, 1012, 912
¹H-NMR (δ ppm in CDCl₃): 3.93 (3H, s), 5.14 (2H, s), 6.8-7.4 (8H, m)
MS m/z (%) [EI]: 239 (M⁺, 5), 91 (100)

| MS [HMS] (as C₁₅H₁₃NO₂): | |
|---|---|
| Calculated | 239.09462 |
| Found | 239.09502 |

(2) 5.83 g of lithium aluminum hydride was placed in a 500 ml three-necked flask and 90 ml of tetrahydrofuran was added at 0°C under a nitrogen atmosphere. Then, 18.1 g of 3-benzyloxy-4-methoxybenzonitrile dissolved in 90 ml of tetrahydrofuran was added dropwise at room temperature. After stirring for 4 hours, 6 ml of water, 6 ml of 15% sodium hydroxide solution, and 18 ml of tetrahydrofuran were carefully added to the mixture in sequence under cooling with ice. The reaction mixture was filtered through cerite. After the addition of water, the filtrate was extracted with chloroform, washed with water, and dried over anhydrous sodium sulfate. The solvent was evaporated to obtain 17 g (93%) of an oily product. A portion of this oily product was dissolved in ether, and 10% hydrochloric acid-methanol solution was added to it to collect deposited crystals by filtration. The crystals were dried to obtain the following hydrochloride. Recrystallized from methanol. Colorless needle-like crystals.
3-Benzyloxy-4-methoxybenzylamine hydrochloride
m.p. (°C): 133-134.5
IR νmax (KBr) cm⁻¹ : 2956-2588 (br), 1604, 1592, 1518, 1498, 1456, 1440, 1426, 1392, 1374, 1344, 1312, 1290, 1262, 1244, 1216, 1190, 1168, 1136, 1098, 1016
¹H-NMR (δ ppm in DMSO-d₆): 3.77 (3H, s), 3.92 (2H, s), 5.07 (2H, s), 7.01-7.45 (8H, m)
MS [FD]: 243 (M⁺ - HCl)
(3) 25 g of 4-benzyloxy-3-methoxybenzoic acid, 150 ml of benzene, 20 ml of thionyl chloride, one drop of dimethylformamide were successively charged into a 500 ml eggplant-type flask, and the mixture was heated at 60°C for 30 minutes while stirring. The solvent and the excessive amount of thionyl chloride were evaporated and the residue was dissolved into 150 ml of benzene. This solution was added dropwise to 29 g of 3-benzyloxy-4-methoxybenzylamine hydrochloride dissolved in a mixed solvent of 300 ml of 10% aqueous solution of sodium hydroxide and 200 ml of benzene, followed by stirring for 1 hour. The solvent was evaporated, and the residue was washed with water and dried, thus obtaining 40 g (83%) of the following compound. Recrystallized from acetone. Colorless needle-like crystals.
N-(3-benzyloxy-4-methoxybenzyl)-4-benzyloxy-3-methoxybenzamide
m.p. (°C): 147.5-149.5
JR νmax (KBr) cm⁻¹ : 3308, 3088, 3060, 3032, 2932, 2872, 2836, 1630, 1602, 1584, 1538, 1508, 1466, 1454, 1426, 1388, 1336, 1302, 1266, 1224, 1182, 1138, 1020
¹H-NMR (δ ppm in CDCl₃): 3.87 (3H, s), 3.93 (3H, s), 4.50 (2H, d, J=5.4Hz), 5.13 (2H, s), 5.20 (2H, s), 6.18 (1H, t, J=5.4Hz), 6.86-7.45 (16H, m)
MS m/z (%) [EI]: 483 (M⁺, 7), 241 (22), 91 (100)

| MS [HMS] (as C₃₀H₂₉NO₅): | |
|---|---|
| Calculated | 483.20449 |
| Found | 483.20389 |

(4) To a suspension prepared in advance by adding 200 ml of ethanol to 0.5 g of 5% palladium-carbon and by absorbing hydrogen, 5.0 g of N-(3-benzyloxy-4-methoxybenzyl)-4-benzyloxy-3-methoxybenzamide was added to carry out hydrogenation. The reaction mixture was collected by filtration and the filtrate was concentrated to obtain 3.08 g (98%) of the following compound. Recrystallized from ethanol. Colorless particle crystals.
N-(3-hydroxy-4-methoxybenzyl)-4-hydroxy-3-methoxybenzamide
m.p. (°C): 160-161.5
JR νmax (KBr) cm⁻¹ : 3448-2736 (br), 1632, 1596, 1554, 1530, 1512, 1460, 1438, 1378, 1318, 1296, 1268, 1248, 1232, 1216, 1180, 1154, 1130, 1048, 1030
¹H-NMR (δ ppm in DMSO-d₆): 3.72 (3H, s), 3.81 (3H, s), 4.32 (2H, d, J=5.9Hz), 6.65-6.85 (4H, m), 7.36-7.47 (2H, m), 8.64 (1H, t, J=5.9Hz)
MS m/z (%) [EI]: 303 (M⁺, 53), 151 (100)

| MS [HMS] (as C₁₆H₁₇NO₅): | |
|---|---|
| Calculated | 303.11064 |
| Found | 303.11054 |

### Synthetic Example 14

(1) A mixture of 9.67 g of N-(3-benzyloxy-4-methoxybenzyl)-4-benzyloxy-3-methoxybenzamide prepared in Synthetic Example 13, 3.0 g of potassium hydroxide, and 100 ml of dimethylsulfoxide was cooled to 0°C. To the mixture was added dropwise 3.4 ml of methyl iodide, followed by stirring for 30 minutes. The mixture was stirred for a further 16 hours at room temperature.
The reaction mixture was charged into water to collect precipitate by filtration. The precipitate was washed with water and dried to obtain 8.7 g (87.4%) of the following compound. Recrystallised from a mixed solvent of n-hexane and ethyl acetate. Colorless needle-like crystals.
N-(3-benzyloxy-4-methoxybenzyl)-N-methyl-4-benzyloxy-3-methoxybenzamide
m.p. (°C): 62-64
IR νmax (KBr) cm⁻¹ : 2936, 2876, 2836, 1636, 1514, 1462, 1422, 1392, 1324, 1266, 1232, 1218, 1182, 1156, 1132, 1074, 1026, 1012
¹H-NMR (δ ppm in DMSO-d₆, at 80°C): 2.82 (3H, s), 3.75 (3H, s), 3.78 (3H, s), 4.47 (2H, s), 5.07 (2H, s), 5.10 (2H, s), 6.84-7.05 (6H, m), 7.27-7.44 (10H, m)
MS m/z (%) [EI]: 497 (M⁺, 11), 241 (16), 91 (100)

| MS [HMS] (as C₃₁H₃₁NO₅): | |
|---|---|
| Calculated | 497.22025 |
| Found | 497.22045 |

(2) To a suspension prepared in advance by adding 400 ml of ethanol to 0.2 g of 5% palladium-carbon and by absorbing hydrogen, 2.1 g of N-(3-benzyloxy-4-methoxybenzyl)-N-methyl-4-benzyloxy-3-methoxybenzamide was added to carry out hydrogenation. The reaction mixture was collected by filtration and the filtrate was concentrated to obtain 1.27 g (95%) of the following compound. Recrystallized from ethanol. Colorless particle crystals.
N-(3-hydroxy-4-methoxybenzyl)-N-methyl-4-hydroxy-3-methoxybenzamide
m.p. (°C): 217-220
IR νmax (KBr) cm⁻¹ : 3308-2848 (br), 1604, 1584, 1524, 1480, 1462, 1440, 1400, 1284, 1242, 1216, 1182, 1154, 1130, 1074, 1024
¹H-NMR (δ ppm in DMSO d₆, at 80°C): 2.83 (3H, s), 3.75 (6H, s), 4.43 (2H, s), 6.62-6.98 (6H, m)
MS m/z (%) [EI]: 317 (M⁺, 32), 286 (13), 285 (18), 181 (34), 166 (31), 152 (20), 151 (100)

| MS [HMS] (as C₁₇H₁₉NO₅): | |
|---|---|
| Calculated | 317.12625 |
| Found | 317.12595 |

### Synthetic Example 15

(1) 25 g of lithium aluminum hydride was placed in a 1000 ml three-necked flask and 500 ml of tetrahydrofuran was added at 0°C. Then, while stirring, 500 ml of tetrahydrofuran solution containing 50 g of 3-benzyloxy-4-methoxy- -nitrostyrene was added dropwise and the mixture was stirred. Stirring was continued for one hour at room temperature and for a further 2 hours over a hot water bath at 70-75°C. 20 ml of water and 20 ml of 20% sodium hydroxide solution were carefully added under cooling with ice, and the mixture was allowed to stand until the mixture ceased to produce bubbles. The reaction mixture was filtered and the filtrate was concentrated. After the addition of water, the concentrate was extracted with benzene, washed with water, and dried over anhydrous sodium sulfate. The solvent was evaporated, and the residue was dissolved into 400 ml of ether. 8 ml of acetic acid was added to this solution while stirring at room temperature. Deposited crystals were collected by filtration, washed with ether, and dried to obtain 32.4 g (64%) of the following compound. Colorless particle crystals.
3-Benzyloxy-4-methoxyphenetylamine acetate
m.p. (°C): 136-138
IR νmax (KBr) cm⁻¹ : 3200-2800 (br), 1650, 1628, 1606, 1588, 1566, 1534, 1512, 1472, 1454, 1424, 1402, 1384, 1348, 1332, 1266, 1236, 1158, 1136, 1082, 1048, 1008
¹H-NMR (δ ppm in DMSO-d₆): 1.82 (3H, s), 2.57-2.83 (4H, m), 3.74 (3H, s), 5.06 (2H, s), 6.71 (1H, dd, J=8.1Hz, 1.9Hz), 6.88 (1H, d, J=8.1Hz), 6.90 (1H, dd, J=1.9Hz), 7.39-7.43 (5H, m)
(2) A mixture of 23.8 g of 3-benzyloxy-4-methoxyphenetylamine acetate, 2.25 g of paraformaldehyde, and 78 ml of formic acid was heated at 50°C while stirring for 3 hours. The solvent was evaporated under reduced pressure and the residue was dissolved into 150 ml of ethanol. To the solution was added dropwise 150 ml of an ethanol solution containing 13.5 g of oxalic acid, followed by stirring for 30 minutes. The produced precipitate was collected by filtration, washed with ethanol, and dried under vacuum. After the addition of 300 ml of 10% sodium hydroxide, the precipitate was extracted with benzene, washed with water, and dried over anhydrous sodium sulfate. The solvent was evaporated to obtain 19.1 g (93%) of the following compound.
6-Benzyloxy-7-methoxy-1,2,3,4-tetrahydroisoquinoline
IR νmax (KBr) cm⁻¹ : 3068, 3064, 3032, 2932, 2836, 2808, 2784, 2732, 1608, 1514, 1470, 1454, 1416, 1392, 1378, 1360, 1324, 1302, 1264, 1254, 1218, 1194, 1180, 1162, 1132, 1104, 1066, 1014
¹H-NMR (δ ppm in CDCl₃): 2.64 (2H, t, J=5.6Hz), 3.08 (2H, t, J=5.6Hz), 3.84 (3H, s), 3.93 (2H, s), 5.10 (2H, s), 6.53 (1H, s), 6.61 (1H, s), 7.26-7.45 (5H, m)

(3) A mixture of 13.6 g of 4-benzyloxy-3-methoxyphenylacetic acid, 100 ml of benzene, and 15 ml of thionyl chloride was stirred for 1 hour and 40 minutes at room temperature. The solvent and the excessive amount of thionyl chloride were evaporated and the residue was dissolved into 100 ml of benzene. This solution was added to a mixed solution of 13.5 g of 6-benzyloxy-7-methoxy-1,2,3,4-tetrahydroisoquinoline, 100 ml of benzene, and 100 ml of 10% aqueous solution of sodium hydroxide, followed by stirring for 1 hour. The reaction mixture was extracted with benzene, washed with water, and dried over anhydrous sodium sulfate. The solvent was then removed by evaporation to obtain 26.1 g (98%) of an oily product.
N-(4-benzyloxy-3-methoxyphenylacetyl)-6-benzyloxy-7-methoxy-1,2,3,4-tetrahydroisoquinoline
IR νmax (KBr) cm⁻¹ : 3060, 3028, 3000, 2932, 2864, 2836, 1640, 1610, 1590, 1514, 1462, 1454, 1382, 1366, 1334, 1306, 1258, 1220, 1140, 1108, 1056, 1026
¹H-NMR (δ ppm in DMSO-d₆, at 80°C): 2.66 (2H, m), 3.64-3.71 (4H, m), 3.74 (6H, s), 4.56 (2H, s), 5.03 (4H, s), 6.7-6.93 (3H, m), 6.76 (1H, s), 6.79 (1H, s), 7.28-7.43 (10H, m)
MS m/z (%) [EI]: 523 (M⁺, 64), 432 (90), 268 (28), 254 (33), 163 (67), 44 (100)

| MS [HMS] (as C₃₃H₃₃NO₅): | |
|---|---|
| Calculated | 523.23592 |
| Found | 523.23742 |

(4) Into a 500 ml conical flask 2 g of 5% palladium carbon, 200 ml of ethyl acetate, 150 ml of ethanol, and 9.76 g of N-(4-benzyloxy-3-methoxyphenylacetyl)-6-benzyloxy-7-methoxy-1,2,3,4-tetrahydroisoquinoline were successively charged to carry out hydrogenation, while the mixture was stirred. The reaction mixture was filtered and the filtrate was concentrated. The residue was purified by flash column chromatography (silica gel, 230-400 mesh; eluent: ethyl acetate), thus obtaining 5.87 g (92%) of the following compound. The compound was recrystallized from ethanol to produce colorless particle crystals.
N-(4-hydroxy-3-methoxyphenylacetyl)-6-hydroxy-7-methoxy-1,2,3,4-tetrahydroisoquinoline
m.p. (°C): 152-154
IR νmax (KBr) cm⁻¹ : 3500-3100 (br), 3008, 2980, 2932, 2844, 1612, 1588, 1516, 1482, 1462, 1450, 1430, 1410, 1364, 1314, 1272, 1230, 1202, 1156, 1056, 1028
¹H-NMR (δ ppm in dDMSO-d₆, at 80°C): 2.48 (2H, m), 3.60-3.66 (4H, m), 3.70 (3H, s), 3.72 (3H, s), 4.51 (2H, s), 6.60-6.71 (3H, m), 6.53 (1H, s), 6.67 (1H, s)
MS m/z (%) [EI]: 343 (M⁺, 100), 219 (61), 206 (51), 178 (38), 177 (74)

| MS [HMS] (as C₁₉H₂₁NO₅): | |
|---|---|
| Calculated | 343.14203 |
| Found | 343.14493 |

### Synthetic Example 16

(1) 50 ml of a 1 M boran/tetrahydrofuran complex solution in tetrahydrofuran was added to a mixture of 9.5 g of N-(4-benzyloxy-3-methoxyphenylacetyl)-6-benzyloxy-7-methoxy-1,2,3,4-tetrahydroisoquinoline prepared in Example 15 and 50 ml of tetrahydrofuran, and the mixture was heated for one hour while refluxing. 40 ml of 6 N hydrochloric acid was added at 0°C and, after bubbling has ceased to occur, the mixture was heated at 50-60°C for 2 hours while stirring. The reaction mixture was alkalinized with the addition of 10% sodium hydroxide at room temperature, extracted with chloroform, washed with water, and dried over anhydrous potassium carbonate. The solvent was evaporated, and 30 ml of methanol was added to the residue. 70 ml of a methanol solution containing 9.1 g of oxalic acid was added and the mixture was stirred. After allowing to stand overnight, deposited crystals were collected by filtration, washed with methanol, and dried under vacuum, thus obtaining 7.68 g of the oxalic acid salt of the following compound. 50 ml of 10% sodium hydroxide was added to this product. The mixture was extracted with chloroform, washed with water, and dried over anhydrous potassium carbonate. The solvent was evaporated, and the residue was recrystallized from ethanol to obtain 4.0 g (yeild 44%) of colorless particle crystals.
N-(4-benzyloxy-3-methoxyphenetyl)-6-benzyloxy-7-methoxy-1,2,3,4-tetrahydroisoquinoline
m.p. (°C): 110-111
IR νmax (KBr) cm⁻¹ : 2912, 1516, 1464, 1454, 1378, 1252, 1226, 1128, 1006
¹H-NMR (δ ppm in DMSO-d₆): 2.62-2.80 (8H, m), 3.54 (2H, s), 3.72 (3H, s), 3.76 (3H, s), 5.01 (2H, s), 5.03 (2H, s), 6.66 (1H, s), 6.72 (1H, dd, J=8.3Hz, 2.0Hz), 6.75 (1H, s), 6.89 (1H, d, J=2.0Hz), 6.91 (1H, d, J=8.3Hz)
MS m/z (%) [EI]: 509 (M⁺, 1), 282 (100)

| MS [HMS] (as C₃₃H₃₅NO₄): | |
|---|---|
| Calculated | 509.25663 |
| Found | 509.25863 |

(2) 0.2 g of 5% palladium-carbon, 400 ml of ethanol, and 2.0 g of N-(4-benzyloxy-3-methoxyphenetyl)-6-benzyloxy-7-methoxy-1,2,3,4-tetrahydroisoquinoline were successively charged into a 500 ml three-necked flask. Hydrogenation was carried out while stirring. The reaction mixture was filtered and the filtrate was concentrated. The concentrate was purified by flash column chromatography (silica gel, 230-400 mesh; eluent, chloroform:methanol = 20:1), thus obtaining the following compound. Colorless flaky crystals (946 mg) of the compound were recrystallized from methanol (yield 73%).
N-(4-hydroxy-3-methoxyphenetyl)-6-hydroxy-7-methoxy-1,2,3,4-tetrahydroisoquinoline
m.p. (°C): 186-188
IR νmax (KBr) cm⁻¹ : 3396, 2996, 2956, 2932, 2912, 2832, 1606, 1522, 1464, 1448, 1426, 1378, 1342, 1306, 1280, 1266, 1232, 1208, 1180, 1152, 1122, 1090, 1070, 1040, 1022
¹H-NMR (δ ppm in CD₃OD): 2.74-2.83 (8H, m), 3.63 (2H, s), 3.80 (3H, s), 3.84 (3H, s), 6.56 (1H, s), 6.61 (1H, s), 6.64 (1H, dd, J=8.1Hz, 1.9Hz), 6.72 (1H, d, J=8.1Hz), 6.81 (1H, d, J=1.9Hz)
MS m/z (%) [EI]: 329 (M⁺, 15), 193 (100), 151 (25)

| MS [HMS] (as C₁₉H₂₃NO₄): | |
|---|---|
| Calculated | 329.16259 |
| Found | 329.16119 |

### Synthetic Example 17

(1) 15.2 g of 3-benzyloxy-4-methoxyphenylacetic acid, 9.4 g of anhydrous potassium carbonate, 200 ml of acetone, and 8.2 ml of diethyl sulfate were successively added to a 500 ml eggplant-type flask, and the mixture was stirred for 16 hours at room temperature. After the addition of water, the reaction mixture was extracted with ether, washed with water, and dried over anhydrous sodium sulfate. The solvent was evaporated and the residue was recrystallised from ethanol, thus obtaining 14 g (yield 83%) of colorless flaky crystals.
Ethyl 3-benzyloxy-4-methoxyphenylacetate
m.p. (°C): 69-70
IR νmax (KBr) cm⁻¹ : 2980, 2936, 1730, 1590, 1516, 1458, 1446, 1430, 1386, 1304, 1290, 1268, 1254, 1232, 1158, 1140, 1022, 1008
¹H-NMR (δ ppm in CDCl₃): 1.22 (3H, t, J=7.3Hz), 3.49 (2H, s), 3.87 (3H, s), 4.11 (2H, q, J=7.3Hz), 5.14 (2H, s), 6.84-7.47 (8H, m)
MS m/z (%) [EI]: 300 (M⁺, 19), 91 (100)

| MS [HMS] (as C₁₈H₂₀O₄): | |
|---|---|
| Calculated | 300.13610 |
| Found | 300.13410 |

(2) 9.0 g of ethyl 3-benzyloxy-4-methoxyphenylacetate and 45 ml of dimethylformamide were placed in a 200 ml three-necked flask. 1.4 g of 60% sodium hydride was added to the mixture while stirring and cooling at 0°C. Then, after the addition of 8.04 g of 3-benzyloxy-4-methoxybenzyl chloride, the mixture was stirred for 1.5 hours at room temperature. The reaction mixture was charged into water and neutralized with diluted hydrochloric acid. The precipitate was collected by filtration, washed with water, and dried under vacuum. The product was subjected to column chromatography (silica gel, 230-400 mesh; eluent, n-hexane:ethyl acetate = 4:1) for separation and purification. 12.6 g (yield 80%) of the following compound was obtained by recrystallization from ethanol as colorless needle-like crystals.
Ethyl 2-(3-benzyloxy-4-methoxyphenyl)-3-(3-benzyloxy-4-methoxyphenyl)propionate
m.p. (°C): 121-123
IR νmax (KBr) cm⁻¹ : 2976, 2948, 2904, 2836, 1740, 1606, 1590, 1514, 1456, 1442, 1424, 1380, 1336, 1292, 1258, 1230, 1192, 1154, 1136, 1082, 1058, 1010
¹H-NMR (δ ppm in CDCl₃): 1.10 (3H, t, J=7.3Hz), 2.81 (1H, dd, J=14.2Hz, 6.8Hz), 3.19 (1H, dd, J=14.2Hz, 8.3Hz), 3.60 (1H, dd, J=8.3Hz, 6.8Hz), 3.83 (3H, s), 3.85 (3H, s), 4.0 (1H, dq, J=14.6Hz, 7.3Hz), 4.01 (1H, dq, J=14.6, 7Hz, 7.3Hz), 5.01 (2H, s), 5.11 (2H, s), 6.57-6.87 (6H, m), 7.25-7.47 (10H, m)
MS m/z (%) [EI]: 526 (M⁺, 35), 299 (14), 227 (100)

| MS [HMS] (as C₃₃H₃₄O₆): | |
|---|---|
| Calculated | 526.23547 |
| Found | 526.23427 |

(3) 12.6 g of ethyl 2-(3-benzyloxy-4-methoxyphenyl)-3-(3-benzyloxy-4-methoxyphenyl)propionate, 100 ml of 10% sodium hydroxide, and 200 ml of ethanol were placed in a 500 ml eggplant-type flask, and the mixture was heated for two hour while refluxing. The solvent was evaporated and, after having been acidified with the addition of 1 N hydrochloric acid, the residue extracted with chloroform, washed with water, and dried over anhydrous sodium sulfate. The solvent was evaporated, and the residue was recrystallized from a mixed solvent of water and methanol to obtain 10.5 g (yield 84%) of the following compound as colorless fine needle-like crystals.
2-(3-Benzyloxy-4-methoxyphenyl)-3-(3-benzyloxy-4-methoxyphenyl)propionic acid
m.p. (°C): 134.5-136.5
¹H-NMR (δ ppm in DMSO-d₆): 2.84 (1H, m), 3.15 (1H, m), 3.70 (3H, s), 3.71 (1H, m), 3.73 (3H, s), 4.96 (2H, s), 5.01 (2H, s), 6.62-7.02 (6H, m), 7.34-7.42 (10H, m)
MS m/z (%) [EI]: 498 (M⁺, 10), 228 (17), 227 (92), 91(100)

| MS [HMS] (as C₃₁H₃₀O₆): | |
|---|---|
| Calculated | 498.2042 |
| Found | 498.2046 |

(4) 1000 mg of 2-(3-benzyloxy-4-methoxyphenyl)-3-(3-benzyloxy-4-methoxyphenyl)propionic acid, 50 ml of acetone, 2 ml of water, and 0.306 ml of triethylamine were placed in a 200 ml eggplant-type flask, and the mixture was cooled to 0°C. 0.288 ml of isobutyl chloroformate was added to it, followed by stirring for 30 minutes. Then, 1 ml of an aqueous solution containing 180 mg of sodium azide was added dropwise, followed by stirring for 1 hour. After the addition of 200 ml of water, the reaction mixture was allowed to stand for 1 hour at 0°C. Deposited crystals were collected by filtration and dried under vacuum at room temperature. 876 mg of the azide thus obtained and 10 ml of toluene were added to a 200 ml eggplant-type flask, and the mixture was heated for 20 minutes while refluxing. Solvent was evaporated under reduced pressure. To the residue were added 6 ml of dioxane and 6 ml of 50% phosphoric acid. The mixture was heated for 1 hour at 100°C while stirring. After allowing to cool to room temperature, the resulting mixture was alkalinized with the addition of 10% aqueous solution of sodium hydroxide. The residue was extracted with chloroform, washed with water, and dried over anhydrous potassium carbonate. The solvent was evaporated, and the residue was dissolved into 5 ml of chloroform. To the solution were added 1 ml of acetic acid, then 30 ml of ether, and the mixture was stirred. After allowing to stand overnight, deposited crystals were collected by filtration and washed with ether to obtain 699 mg (yield 78%) of the following compound as a colorless powder.
1-(3-Benzyloxy-4-methoxyphenyl)-3-benzyloxy-4-methoxyphenetylamine acetate
m.p. (°C): 125-127
IR νmax (KBr) cm⁻¹ : 3100-255 (br), 1518, 1262, 1238, 1164, 1140, 1026, 1006
¹H-NMR (δ ppm in DMSO-d₆): 1.88 (3H, s), 2.72 (2H, d, J=7.0Hz), 3.71 (3H, s), 3.72 (3H, s), 3.96 (1H, t, J=7.0Hz), 4.94 (2H, s), 5.01 (2H, s), 6.57-7.03 (6H, m), 7.35-7.56 (10H, m)
MS m/z (%) [FD]: 469.3 (M⁺-AcOH)
(5) A mixture of 1.9 g of 1-(3-benzyloxy-4-methoxyphenyl)-3-benzyloxy-4-methoxyphenetylamine acetate, 108 mg of p-formaldehyde, and 6 ml of formic acid was heated for 3.5 hours at 60°C while stirring. The reaction mixture was charged into ice water and alkalinized with the addition of 10% sodium hydroxide, extracted with chloroform, washed with water, and dried over anhydrous potassium carbonate. The solvent was evaporated, and the residue was recrystallized from methanol to obtain 1.6 g (yield 94%) of the following compound as colorless needle-like crystals.
3-(3-Benzyloxy-4-methoxyphenyl)-6-benzyloxy-7-methoxy-1,2,3,4-tetrahydroisoquinoline
m.p. (°C): 136-138
IR νmax (KBr) cm⁻¹ : 3292, 3004, 2932, 2832, 1610, 1512, 1460, 1424, 1386, 1374, 1386, 1374, 1326, 1306, 1258, 1236, 1222, 1192, 1176, 1156, 1142, 1114, 1096, 1012
MS m/z (%) [EI]: 481 (M⁺, 27), 240 (62), 91 (100)

| MS [HMS] (as C₃₁H₃₁NO₄): | |
|---|---|
| Calculated | 481.22524 |
| Found | 481.22464 |

(6) 100 mg of 5% palladium-carbon, 50 ml of ethanol, and 1.0 g of 3-(3-benzyloxy-4-methoxyphenyl)-6-benzyloxy-7-methoxy-1,2,3,4-tetrahydroisoquinoline were successively charged into a 200 ml conical flask. Hydrogenation was carried out while stirring. The reaction mixture was filtered and the filtrate was concentrated. A 20:1 mixed solvent of chloroform and methanol was added to the concentrate to crystallize. The crystals were collected by filtration and recrystallized from methanol, thus obtaining 523 g (yield 83.4%) of the following compound as a colorless powder.
3-(3-Hydroxy-4-methoxyphenyl)-6-hydroxy-7-methoxy-1,2,3,4-tetrahydroisoquinoline
m.p. (°C): 186-188
IR νmax (KBr) cm⁻¹ : 3400-2700, 1606, 1530, 1504, 1462, 1442, 1368, 1336, 1260, 1228, 1212, 1118, 1030
¹H-NMR (δ ppm in CD₃OD): 3.04 (2H, m), 3.85, (3H, s), 3.88 (3H, s), 4.22-4.49 (3H, m), 6.68 (1H, s), 6.79 (1H, s), 6.92-7.11 (3H, m)
MS m/z (%) [EI]: 301 (M⁺, 37), 152 (100), 150 (87)

| MS [HMS] (as C₁₇H₁₉NO₄): | |
|---|---|
| Calculated | 301.13139 |
| Found | 301.13219 |

### Synthetic Example 18

(1) 20.95 g (76.96 mmol) of 3-benzyloxy-4-methoxyphenylacetic acid was placed in a 1 l eggplant-type flask equipped with a reflux condenser. After replacing the internal atmosphere with argon, the compound was dissolved into 80 ml of anhydrous benzene. After the addition of 22.5 ml (307.8 mmol) of thionyl chloride, the mixture was stirred at 50-60°C (outside temperature) for 2 hours. The reaction mixture was distilled under reduced pressure (distillation was repeated two or three times with the addition of anhydrous benzene) to obtain acid chloride of said acetic acid compound as a brown solid. This solid was dissolved into 100 ml of anhydrous benzene, and to the solution was added a mixed solution of 20.0 g (77.72 mmol) of 4-benzyloxy-3-methoxyphenetylamine, 80 ml of anhydrous benzene, and 270 ml of 10% sodium hydroxide in portions while stirring under cooling with ice. After stirring for 30 minutes at room temperature, the reaction mixture was evaporated to remove benzene. The insoluble components were collected by filtration (thoroughly washed with water, 5% hydrochloric acid, then water) and dried (in the presence of phosphorus pentaoxide, at 60-70°C/2-3 mmHg) to obtain a yellow-white solid (38.64 g). This solid was recrystallized in ethanol (about 1.5 l) to obtain 35.9 g (yield 91%) of N-(4-benzyloxy-3-methoxyphenetyl)-3-benzyloxy-4-methoxyphenylacetamide.
N-(4-benzyloxy-3-methoxyphenetyl)-3-benzyloxy-4-methoxyphenylacetamide
m.p. (°C): 140-141
IR νmax (KBr) cm⁻¹ : 3308 (NH), 2932, 2868, 2836, 1644, 1590, 1548, 1514, 1454, 1262, 1236, 1138, 1014, 748, 698
¹H-NMR (δ ppm in CDCl₃): 2.60 (2H, t, J=6.8Hz), 3.36 (2H, t, J=6.8Hz), 3.41 (2H, s), 3.83 (3H, s), 3.87 (3H, s), 5.09 (4H, s), 5.31 (1H, br, NH), 6.43 (1H, dd, J=8.3, 2.0Hz), 6.62 (1H, d, J=2.0Hz), 6.67 (1H, dd, J=7.8, 2.0Hz), 6.72 (1H, d, J=2.0Hz), 6.72 (1H, d, J=8.3Hz), 6.80 (1H, d, J=7.8Hz), 7.25-7.50 (10H, m)
MS m/z (%) [EI]: 511 (M⁺, 21), 421 (8), 330 (8), 271 (11), 252 (12), 240 (100), 227 (20), 149 (40), 137 (51), 106 (52)
(2) 1.8 g (3.52 mmol) of N-(4-benzyloxy-3-methoxyphenetyl)-3-benzyloxy-4-methoxyphenylacetamide, 750 mg of 5% palladium-carbon, and 50 ml of methanol were placed in a 300 ml flask. The mixture was subjected to catalytic hydrogenation and stirred for 3 hours at room temperature. The reaction mixture was filtered through cerite and the filtrate was evaporated under reduced pressure to obtain a colorless solid. This solid was subjected to flash column chromatography (silica gel, 230-400 mesh; φ 3.5 cm x 5 cm, about 25 g; eluent: ethyl acetate; pressure: 0.2 kg/cm²; 1 fraction = 70 ml) to separate 1.06 g (yield 91%) of N-(4-hydroxy-3-methoxyphenetyl)-3-hydroxy-4-methoxyphenylacetamide as a colorless powder. This compound was further recrystallized from ethyl acetate/n-hexane to obtain colorless needle-like crystals.
N-(4-hydroxy-3-methoxyphenetyl)-3-hydroxy-4-methoxyphenylacetamide
m.p. (°C): 132-134
IR νmax (KBr) cm⁻¹ : 3400, 3304, 2968, 2936, 2840, 1658, 1548, 1514, 1456, 1438, 1348, 1270, 1232, 1190, 1154, 1128, 1032, 970, 922, 876, 846, 818, 800, 762, 736, 632, 606, 584, 564, 534
¹H-NMR (δ ppm in CD₃OD): 2.65 (2H, t, J=7.1Hz), 3.32 (3H, s), 3.35 (2H, t, J=7.1Hz), 3.76 (3H, s), 3.80 (3H, s), 6.54 (1H, dd, J=8.1, 2.0Hz), 6.60 (1H, dd, J=8.3, 2.0Hz), 6.68 (1H, d, J=8.1Hz), 6.72 (1H, d, J=2.0Hz), 6.73 (1H, d, J=2.0Hz), 6.79 (1H, d, J=8.3Hz)
MS m/z (%) [EI]: 331 (M⁺, 8), 181 (12), 150 (100), 137 (42)

| MS [HMS] (as C₁₈H₂₁NO₅): | |
|---|---|
| Calculated | 331.14190 |
| Found | 331.14030 |

### Synthetic Example 19

(1) 21.3 g (41.60 mmol) of N-(4-benzyloxy-3-methoxyphenetyl)-3-benzyloxy-4-methoxyphenylacetamide, 210 ml of anhydrous benzene, and 58.2 ml (0.624 mmol) of phosphorus oxychloride were placed in a 500 ml eggplant-type flask equipped with a reflux condenser. The mixture was heated with refluxing for 2.5 hours while stirring. The reaction mixture was cooled to room temperature and poured into cold n-hexane (about 1.5 l). Deposited insoluble components were collected by filtration (thoroughly washed with n-hexane) and the moisture absorptive product was immediately dried (in the presence of phosphorus pentaoxide, at 50-60°C/2-3 mmHg) to obtain a yellow-white solid (25.95 g). This solid was recrystallized from a mixed solvent of methanol and ether to obtain 20.8 g (yield 94%) of 1-(3-benzyloxy-4-methoxybenzyl)-6-methoxy-7-benzyloxy-3,4-dihydroisoquinoline hydrochloride as an light yellow needle-like crystals.
1-(3-Benzyloxy-4-methoxybenzyl)-6-methoxy-7-benzyloxy-3,4-dihydroisoquinoline hydrochloride
m.p. (°C): 203-204
IR νmax (KBr) cm⁻¹ : 3450, 3024, 2836, 2684, 1652, 1606, 1568, 1512, 1454, 1334, 1296, 1276, 1248, 1154, 1042, 1026, 776, 736, 696
¹H-NMR (δ ppm in CDCl₃): 2.79 (2H, t, J=8.3Hz), 3.80 (2H, t, J=8.3Hz), 3.83 (3H, s), 3.98 (3H, s), 4.34 (2H, s), 5.09 (2H, s), 5.19 (2H, s), 6.47 (1H, dd, J=8.3, 2.0Hz), 6.67 (1H, d, J=8.3Hz), 6.72 (1H, s), 7.09 (1H, d, J=2.0Hz), 7.20 (1H, s), 7.20-7.50 (10H, m)
MS m/z (%) [EI]: 493 (M⁺, 12), 414 (16), 403 (100), 400 (23), 312 (30), 268 (29)
(2) 21.5 g (40.64 mmol) of 1-(3-benzyloxy-4-methoxybenzyl)-6-methoxy-7-benzyloxy-3,4-dihydroisoquinoline hydrochloride, 1.4 l of methanol, and 70 ml of water were charged into a 2 l eggplant-type flask equipped with a reflux condenser. After the addition of 21.5 g of sodium borohydride in portions while stirring at room temperature, the mixture was heated with refluxing for one hour. The reaction liquid was evaporated under reduced pressure and water was added to the residue. The residue was then extracted with ether twice (400 ml x 2). The ether layer was washed with saturated brine once and dried (K₂CO₃). The solvent was evaporated under reduced pressure to obtain 19.7 g (yield 98%) of 1-(3-benzyloxy-4-methoxybenzyl)-6-methoxy-7-benzyloxy-1,2,3,4-tetrahydroisoquinoline as a colorless resinous material.
1-(3-Benzyloxy-4-methoxybenzyl)-6-methoxy-7-benzyloxy-1,2,3,4-tetrahydroisoquinoline.
IR νmax (KBr) cm⁻¹ : 3450, 3028, 2924, 2836, 1608, 1588, 1514, 1454, 1262, 1220, 1138, 1112, 1024, 736, 696
¹H-NMR (δ ppm in CDCl₃): 2.60-3.20 (6H, m), 3.86 (3H, s), 3.87 (3H, s), 3.97 (1H, dd, J=8.8, 3.9Hz), 5.07 (2H, s), 5.11 (2H, s), 6.59 (1H, s), 6.66 (1H, s), 6.70 (1H, d, J=2.0Hz), 6.72 (1H, dd, J=8.8, 2.0Hz), 6.83 (1H, d, J=8.8Hz), 7.25-7.50 (10H, m)
MS m/z (%) [EI]: 494 (M-1, 1), 402 (5), 268 (100), 177 (21), 148 (10), 91 (24)
(3) 5.0 g (10.09 mmol) of 1-(3-benzyloxy-4-methoxybenzyl)-6-methoxy-7-benzyloxy-1,2,3,4-tetrahydroisoquinoline, 50 ml of ethanol, and 50 ml of 35% hydrochloric acid were charged into a 300 ml eggplant-type flask equipped with a reflux condenser. While stirring, the mixture was heated for 1.5 hours under refluxing and stirred for 1 hour at room temperature. The reaction mixture was evaporated under reduced pressure and 100 ml of water was added to the the residue. The mixture was alkalinized with a saturated aqueous solution of sodium bicarbonate to collect deposited white precipitate by filtration. The filtrated was extracted with chloroform, dried (Na₂SO₄), and evaporated under reduced pressure to remove solvent. The residue was combined with said white precipitate, and subjected to flash column chromatography (silica gel, 230-400 mesh; φ 4.5 cm x 5 cm, about 40 g; eluent: methanol:chloroform = 1:25-1:10; pressure: 0.2 kg/cm²; 1 fraction = 70 ml) to separate 2.06 g (yield 65%) of 1-(3-hydroxy-4-methoxybenzyl)-6-methoxy-7-hydroxy-1,2,3,4- tetrahydroisoquinoline as an yellow powder as the first elution fraction (9-21).
1-(3-Hydroxy-4-methoxybenzyl)-6-methoxy-7-hydroxy-1,2,3,4-tetrahydroisoquinoline
m.p. (°C): 90-95
IR νmax (KBr) cm⁻¹ : 3408, 2928, 2836, 1590, 1512, 1444, 1374, 1270, 1222, 1128, 1106, 1028, 868, 796, 758
¹H-NMR (δ ppm in CD₃OD): 2.60-2.90 (4H, m), 3.00-3.20 (2H, m), 3.81 (3H, s), 3.83 (3H, s), 4.02 (1H, dd, J=9.8, 4.9Hz), 6.64 (1H, s), 6.67 (1H, s), 6.68 (1H, dd, J=8.1, 2.0Hz), 6.73 (1H, d, J=2.0Hz), 6.87 (1H, d, J=8.1Hz)
MS m/z (%) [FD]: 316 (M⁺ 1), 178

| MS [HMS] (as C₁₈H₂₂O₄): | |
|---|---|
| Calculated | 316.15488 |
| Found | 316.15571 |

### Synthetic Example 20

(1) 5.0 g (10.09 mmol) of 1-(3-benzyloxy-4-methoxybenzyl)-6-methoxy-7-benzyloxy-1,2,3,4-tetrahydroisoquinoline prepared in Synthetic Example 19, 12 ml of acetic acid anhydride, and 12 ml of anhydrous pyridine were placed in a 300 ml eggplant-type flask equipped with a reflux condenser. The mixture was stirred for 2 hours at room temperature and for 1 hour with heating (outside temperature 60-70°C). The reaction mixture was poured into ice water (300 ml). After stirring for one hour at room temperature, the mixture was extracted with chloroform twice. The chloroform layer was washed with saturated brine once and dried (Na₂CO₄). A residue which was obtained by evaporation under reduced pressure was subjected to flash column chromatography (silica gel, 230-400 mesh; φ 4.5 cm x 20 cm, about 160 g; eluent: ethyl acetate; pressure: 0.2 kg/cm²; 1 fraction = 70 ml) to separate 5.1 g (yield 94%) of N-acetyl-1-(3-benzyloxy-4-methoxybenzyl)-6-methoxy-7-benzyloxy-1,2,3,4-tetrahydroisoquinoline as a white powder from the first elution fraction (8-25). This powder was recystallized from a mixed solvent of benzene and n-hexane to obtain colorless needle-like crystals.
N-acetyl-1-(3-benzyloxy-4-methoxybenzyl)-6-methoxy-7-benzyloxy-1,2,3,4-tetrahydroisoquinoline
IR νmax (KBr) cm⁻¹ : 3065, 3029, 3005, 2913, 2833, 1642, 1514, 1444, 1426, 1388, 1356, 1334, 1260, 1234, 1220, 1158, 1136, 1116, 1024, 1004, 938, 884, 852, 834, 810, 794, 768, 748, 728, 694
¹H-NMR (δ ppm in CDCl₃): 1.49 and 2.07 (3H, s), 2.40-3.10 (6H, m), 3.23 and 3.53 (1H, m), 3.83 and 3.84 (3H, s), 3.87 (3H, s), 4.83 and 4.92 (1H, d, J=12Hz), 4.99 (1H, s), 5.06 (1H, s), 5.09 and 5.17 (1H, d, J=12Hz), 6.23 (1H, s), 6.43 and 6.55 (1H, dd, J=8.1, 2.4Hz), 6.46 and 6.56 (1H, s), 6.62 (1H, d, J=2.4Hz), 6.66 and 6.76 (1H, d, J=8.1Hz)
MS m/z (%) [FD]: 538 (M⁺ 1), 310
(2) 4.0 g (7.44 mmol) of N-acetyl-1-(3-benzyloxy-4-methoxybenzyl)-6-methoxy-7-benzyloxy-1,2,3,4-tetrahydroisoquinoline, 400 mg of 5% palladium-carbon, and 100 ml of methanol were placed in a 300 ml flask. The mixture was stirred for 3 hours at room temperature. The reaction mixture was filtered through cerite and the filtrate was evaporated under reduced pressure to obtain a white solid. This solid was subjected to flash column chromatography (silica gel, 230-400 mesh; φ 4.5 cm x 10 cm, about 80 g; eluent: methanol:chloroform = 1:20; pressure: 0.2 kg/cm²; 1 fraction = 80 ml) for separation, thus obtaining 2.4 g (yield 91%) of N-acetyl-1-(3-hydroxy-4-methoxybenzyl)-6-methoxy-7-hydroxy-1,2,3,4-tetrahydroisoquinoline as a white powder from the first elution fraction (5-10). This powder was recystallized from a mixed solvent of chloroform and n-hexane to obtaib colorless prismatic crystals.
N-acetyl-1-(3-hydroxy-4-methoxybenzyl)-6-methoxy-7-hydroxy-1,2,3,4-tetrahydroisoquinoline
m.p. (°C): 207-210
IR νmax (KBr) cm⁻¹ : 3420, 3268, 2988, 2968, 2928, 2832, 1624, 1598, 1512, 1462, 1436, 1370, 1284, 1272, 1250, 1234, 1202, 1178, 1036, 1020, 940, 870, 838, 808, 786, 758, 656, 622, 582, 526
¹H-NMR (δ ppm in DMSO₆): 1.79 (3H, brs), 2.40-2.90 (6H, m), 3.74 (3H, s), 3.75 (3H, s), 4.00 (1H, m), 5.15 (1H, br), 6.49 (1H, dd, J=8.1, 2.0Hz), 6.51 (1H, s), 6.61 (1H, d, J=2.0Hz), 6.64 (1H, s), 6.76 (1H, d, J=8.1Hz)
MS m/z (%) [FD]: 357 (M⁺), 220

| MS [HMS] (as C₂₀H₂₃NO₅): | |
|---|---|
| Calculated | 357.15755 |
| Found | 357.15655 |

### Synthetic Example 21

5.8 g (1.76 mmol) of 1-(3-benzyloxy-4-methoxybenzyl)-6-methoxy-7-benzyloxy-1,2,3,4-tetrahydroisoquinoline obtained in Synthetic Example 19 was placed in a 500 ml flask and dissolved into 116 ml of methanol. The mixture was subjected to catalytic hydrogenation with the addition of 58 ml of 35% formalin and 1.7 g of 5% palladium-carbon, and stirred for 2 days at room temperature. The reaction mixture was filtered through cerite and the filtrate was evaporated under reduced pressure to obtain a residue. The residue was dissolved in 5% methanol/chloroform, washed with saturated aqueous solution of sodium bicarbonate once, dried (Na₂SO₄), and solvent was removed by evaporation under reduced pressure to obtain a residue (on TLC, the product was alkalinized because salt formation was presumed). The residue was subjected to flash column chromatography (silica gel, 230-400 mesh; φ 4.5 cm x 10 cm, about 80 g; eluent: methanol:chloroform = 1:10; pressure: 0.2 kg/cm²; 1 fraction = 80 ml) for separation, thus obtaining 2.28 g (yield 59%) of N-methyl-1-(3-hydroxy-4-methoxybenzyl)-6-methoxy-7-hydroxy-1,2,3,4-tetrahydroisoquinoline as a white powder from the first elution fraction (10-15).
N-methyl-1-(3-hydroxy-4-methoxybenzyl)-6-methoxy-7-hydroxy-1,2,3,4-tetrahydroisoquinoline
m.p. (°C): 65-70
IR νmax (KBr) cm⁻¹ : 3420, 2936, 2840, 1592, 1512, 1444, 1372, 1276, 1218, 1130, 1098, 1024, 870, 808, 792, 758, 622
¹H-NMR (δ ppm in CD₃OD): 2.45 (3H, s), 2.50-2.90 (4H, m), 2.90-3.20 (2H, m), 3.68 (1H, dd, J=7.3, 4.6Hz), 3.79 (3H, s), 3.81 (3H, s), 6.12 (1H, s), 6.52 (1H, dd, J=8.1, 2.0Hz), 6.60 (1H, d, J=2.0Hz), 6.62 (1H, s), 6.80 (1H, d, J=8.1Hz)
MS m/z (%) [FD]: 330 (M⁺ H), 192

| MS [HMS] (as C₁₉H₂₄O₄N): | |
|---|---|
| Calculated | 330.17053 |
| Found | 330.17046 |

### Synthetic Example 22

17.7 g (129 mmol) of p-methoxybenzylamine, 19 ml (234 mmol) of anhydrous pyridine, and 200 ml of anhydrous benzene were dissolved in a 1 l eggplant-type flask equipped with a calcium chloride tube and a dropping funnel. A solution of 20.0 g (117 mmol) of anisoyl chloride in 200 ml of anhydrous benzene was added dropwise under cooling with ice while stirring. The mixture was stirred for 2 hours at room temperature. Benzene was removed from the reaction mixture by evaporation under reduced pressure. After the addition of water, the residue was extracted twice with chloroform (400 ml x 2), and the chloroform layer was washed with 5% hydrochloric acid, 5% sodium hydroxide, and water, once with each, and dried over Na₂SO₄. The solvent was evaporated under reduced pressure to obtain a residue. The residue was subjected to flash column chromatography (silica gel, 230-400 mesh; φ 6.5 cm x 20 cm, about 300 g; eluent: ethyl acetate:n-hexane = 1:2-1:1; pressure: 0.2 kg/cm²; 1 fraction = 100 ml) for separation, thus obtaining 26.9 g (yield 85%) of N-(4-methoxybenzyl)-3-methoxybenzamide as a white solid from the first elution fraction (20-31).

This solid was recrystallized from chloroform/n-hexane to obtain colorless prismatic crystals.
N-(4-methoxybenzyl)-3-methoxybenzamide
IR νmax (KBr) cm⁻¹ : 3280 (NH), 3072, 2956, 2908, 2836, 1640, 1584, 1552, 1514, 1488, 1454, 1426, 1322, 1286, 1248, 1174, 1032, 1000, 874, 812, 778, 754, 704, 658, 510
¹H-NMR (δ ppm in DMSO-d₆): 3.72 (3H, s), 3.80 (3H, s), 4.40 (2H, d, J=5.9Hz), 6.88 (1H, dd, J=8.8, 2.0Hz), 6.89 (1H, dd, J=8.8, 2.0Hz), 7.08 (1H, ddd, J=7.8, 2.0, 1.0Hz), 7.23 (1H, dd, J=8.8, 2.0Hz), 7.24 (1H, dd, J=8.8, 2.0Hz), 7.37 (1H, dd, J=7.8, 7.8Hz), 7.44 (1H, dd, J=2.0, 2.0Hz), 7.47 (1H, ddd, J=7.8, 1.0, 1.0Hz), 8.96 (1H, t, J=5.9Hz)
MS m/z (%) [EI]: 271 (M⁺, 74), 136 (100)

| MS [HMS] (as C₁₆H₁₇O₃N): | |
|---|---|
| Calculated | 271.12079 |
| Found | 271.11929 |

### Synthetic Example 23

4.0 g (14.74 mmol) of N-(4-methoxybenzyl)-3-methoxybenzamide obtained in Synthetic Example 22 was charged into a 300 ml eggplant-type flask. 74 ml of anhydrous methylene chloride was added to it and dissolved. After replacing the internal atmosphere with argon, 74 ml (58.98 mmol) of 0.8 M boron tribromide in methylene chloride was added under cooling with ice while stirring. The mixture was stirred for 30 minutes under cooling with ice and for 3 hours at room temperature. The reaction mixture was poured into ice water, extracted twice with ethyl acetate (300 ml x 2). The ethyl acetate layer was washed with water and saturated brine, once with each, and dried over MgSO₄. The solvent was evaporated under reduced pressure to obtain a residue. The residue was recrystallized from ethyl acetate/n-hexane to obtain 3.28 g (yield 92%) of N-(4-hydroxybenzyl)-3-hydroxybenzamide as colorless needle-like crystals.
N-(4-hydroxybenzyl)-3-hydroxybenzamide
m.p. (°C): 154-156
IR νmax (KBr) cm⁻¹ : 3372 (OH), 3332 (OH), 3248 (NH), 1642, 1596, 1542, 1516, 1454, 1380, 1308, 1284, 1232, 1174, 824, 798, 676, 656, 634
¹H-NMR (δ ppm in DMSO-d₆): 4.33 (2H, d, J=5.9Hz), 6.70 (1H, dd, J=8.3, 1.0Hz), 6.71 (1H, dd, J=8.3, 1.0Hz), 6.90 (1H, ddd, J=7.3, 3.0, 1.5Hz), 7.11 (1H, dd, J=8.3, 1.0Hz), 7.11 (1H, dd, J=8.3, 1.0Hz), 7.23 (1H, dd, J=7.3, 7.3Hz), 7.27 (1H, dd, J=1.5, 1.5Hz), 7.29 (1H, ddd, J=7.3, 1.5, 1.5Hz), 8.81 (1H, t, J=5.9Hz), 9.25 (1H, s, D₂O exchangable), 9.61 (1H, s, D₂O exchangable)
MS m/z (%) [EI]: 243 (M⁺, 64), 121 (100)

| MS [HMS] (as C₁₄H₁₃O₃N): | |
|---|---|
| Calculated | 243.08855 |
| Found | 243.09085 |

### Synthetic Example 24

15.0 g (55.29 mmol) of N-(4-methoxybenzyl)-3-methoxybenzamide obtained in Synthetic Example 22 and 4.6 g (82.93 mmol) of potassium hydroxide (a solid product was pulverized into powder in mortar) were placed in a 200 ml eggplant-type flask. After replacing the internal atmosphere with argon, 55 ml of dimethylsulfoxide was added to dissolve the mixture. 17.2 ml (276.5 mmol) of methyl iodide was added under cooling with ice while stirring. The mixture was stirred for 4 hours at room temperature. The reaction mixture was poured into ice water (300 ml), extracted twice with ether (300 ml x 2), and the ether layer was washed with water three times and with saturated brine once, and dried over MgSO₄. The solvent was evaporated under reduced pressure to obtain a residue. The residue was subjected to flash column chromatography (silica gel, 230-400 mesh; φ 6.5 cm x 10 cm, about 150 g; eluent: ethyl acetate:n-hexane = 1:1; pressure: 0.2 kg/cm²; 1 fraction = 80 ml) for separation, thus obtaining 15.21 g (yield 96%) of N-(4-methoxybenzyl)-N-methyl-3-methoxybenzamide as a colorless viscous oily product from the first elution fraction (8-17).
N-(4-methoxybenzyl)-N-methyl-3-methoxybenzamide
IR νmax (KBr) cm⁻¹ : 3004, 2936, 2836, 1636, 1614, 1582, 1514, 1462, 1400, 1288, 1270, 1248, 1176, 1068, 1038, 846, 816, 794, 750, 694
¹H-NMR (δ ppm in DMSO-d₆ at 80°C): 2.82 (3H, s), 3.75 (3H, s), 3.76 (3H, s), 4.49 (2H, s), 6.90-7.40 (8H, m)
MS m/z (%) [EI]: 285 (M⁺, 55), 135 (100)

| MS [HMS] (as C₁₇H₁₉O₃N): | |
|---|---|
| Calculated | 285.13651 |
| Found | 285.13691 |

### Synthetic Example 25

3.5 g (12.27 mmol) of N-(4-methoxybenzyl)-N-methyl-3-methoxybenzamide obtained in Synthetic Example 24 was placed in a 300 ml eggplant-type flask. After replacing the internal atmosphere with argon, 61 ml of anhydrous methylene chloride was added to dissolve the mixture. 61 ml (49.08 mmol) of a 3 M boron tribromide solution in methylene chloride was added under cooling with ice while stirring. The mixture was stirred for 30 minutes under cooling with ice and one hour at room temperature. The reaction mixture was poured into ice water, extracted twice with ethyl acetate (300 ml x 2). The ethyl acetate layer was washed with water and saturated brine, once with each, and dried over MgSO₄. The solvent was evaporated under reduced pressure to obtain a residue. The residue was subjected to flash column chromatography (silica gel, 230-400 mesh; φ 4.5 cm x 10 cm, about 80 g; eluent: ethyl acetate; pressure: 0.2 kg/cm²; 1 fraction = 80 ml) for separation, thus obtaining 2.52 g (yield 80%) of N-(4-hydroxybenzyl)-N-methyl-3-hydroxybenzamide as a colorless amorphous product from the first elution fraction (4-6).
N-(4-hydroxybenzyl)-N-methyl-3-hydroxybenzamide
m.p. (°C): 45-50
IR νmax (KBr) cm⁻¹ : 3304 (OH), 2808, 1584, 1514, 1446, 1408, 1358, 1310, 1232, 1172, 1070, 824, 794, 746, 694
¹H-NMR (δ ppm in DMSO-d₆ at 80°C): 2.80 (3H, s), 4.43 (2H, s), 6.70-7.30 (8H, m), 9.10 (1H, br, D₂O exchangable), 9.35 (1H, br, D₂O exchangable)
MS m/z (%) [EI]: 257 (M⁺, 48), 121 (100)

| MS [HMS] (as C₁₅H₁₅O₃N): | |
|---|---|
| Calculated | 257.10513 |
| Found | 257.10273 |

### Synthetic Example 26

20.0 g (73.72 mmol) of N-(4-methoxybenzyl)-3-methoxybenzamide obtained in Synthetic Example 22 was placed in a 500 ml two-necked flask equipped with septum rubber and a reflux condenser. After replacing the internal atmosphere with argon, 100 ml of anhydrous tetrahydrofuran was added to dissolve the mixture. 184 ml (184.3 mmol) of a 1 M boran/tetrahydrofuran solution was added under cooling with ice while stirring. The mixture was stirred for 30 minutes under cooling with ice and for one hour at room temperature, and heated under refluxing for one hour. After the addition of 30 ml of 6 N hydrochloric acid under cooling with ice, the reaction mixture was heated under refluxing for one hour.

After neutralizing the reaction mixture with 35-40 ml of 6 N sodium hydroxide, the reaction mixture was extracted twice with ether (300 ml x 2). The ether layer was washed with water and saturated brine, once with each, and dried over K₂CO₃. The solvent was evaporated under reduced pressure to obtain a residue. 100 ml of ether solution of this residue was added to a solution of 20.5 g of maleic acid in 600 ml of ether at room temperature, and the mixture was stirred for 30 minutes at room temperature. Deposited white precipitate was collected by filtration to obtain 26.68 g (yield 90%) of white powdery crystals (m.p. 142-144°C). The crystals were alkalinized with 5% sodium hydroxide, extracted twice with ether (300 ml x 2). The ether layer was washed with water and saturated brine, once with each, and dried over K₂CO₃. The solvent was evaporated under reduced pressure to obtain 16.8 g (yield 82%) of N-(4-methoxybenzyl)-3-methoxybenzylamine as a colorless oily product.
N-(4-methoxybenzyl)-3-methoxybenzylamine
m.p. (°C): 142-144 (maleate)
IR νmax (KBr) cm⁻¹ : 3450 (NH), 2952, 2836, 1612, 1586, 1512, 1488, 1466, 1248, 1174, 1154, 1038, 818, 782, 694
¹H-NMR (δ ppm in CDCl₃): 3.76 (2H, s), 3.78 (2H, s), 3.80 (3H, s), 3.81 (3H, s), 6.75-7.35 (8H, m)
MS m/z (%) [EI]: 257 (M⁺, 7), 256 (10), 137 (84), 122 (100)

| MS [HMS] (as C₁₆H₁₉O₂N): | |
|---|---|
| Calculated | 257.14142 |
| Found | 257.14292 |

### Synthetic Example 27

8.8 g (30.84 mmol) of N-(4-methoxybenzyl)-N-methyl-3-methoxybenzamide obtained in Synthetic Example 24 was placed in a 300 ml eggplant-type flask equipped with septum rubber and a reflux condenser. After replacing the internal atmosphere with argon, 40 ml of anhydrous tetrahydrofuran was added to dissolve the mixture. 77 ml (77.10 mmol) of a 1 M boran/tetrahydrofuran solution was added under cooling with ice while stirring. The mixture was stirred for 10 minutes at room temperature and heated under refluxing for one hour. After the addition of 13 ml of 6 N hydrochloric acid under cooling with ice, the reaction mixture was heated under refluxing for one hour. After neutralizing with 13-15 ml of 6 N sodium hydroxide, the reaction mixture was extracted twice with ether (200 ml x 2). The ether layer was washed with water and saturated brine, once with each, and dried over K₂CO₃. The solvent was evaporated under reduced pressure to obtain a residue. 40 ml of ether solution of this was added to a solution of 4.3 g of maleic acid in 260 ml of ether at room temperature, and the mixture was stirred for 30 minutes at room temperature. Deposited white precipitate was collected by filtration to obtain 7.34 g (yield 80%) of white powdery crystals (m.p. 100-102°C). The crystals were alkalinized with 5% sodium hydroxide, extracted twice with ether (200 ml x 2), and the ether layer was washed with water and saturated brine, once. with each, and dried over K₂CO₃. The solvent was evaporated under reduced pressure to obtain 6.1 g (yield 72%) of N-(4-methoxybenzyl)-N-methyl-3-methoxybenzylamine as a colorless oily product.
N-(4-methoxybenzyl)-N-methyl-3-methoxybenzylamine
m.p. (°C): 100-102 (maleate)
IR νmax (KBr) cm⁻¹ : 2984, 2836, 2784, 1612, 1586, 1512, 1488, 1450, 1366, 1246, 1170, 1152, 1038, 810, 782, 694
¹H-NMR (δ ppm in CDCl₃): 2.14 (3H, s), 3.44 (2H, s), 3.46 (2H, s), 3.77 (3H, s), 3.78 (3H, s), 6.80-7.35 (8H, m)
MS m/z (%) [EI]: 271 (M⁺, 16), 150 (51), 121 (100)

| MS [HMS] (as C₁₇H₂₁NO₂): | |
|---|---|
| Calculated | 271.15728 |
| Found | 271.15908 |

### Synthetic Example 28

10.0 g (38.86 mmol) of N-(4-methoxybenzyl)-3-methoxybenzylamine obtained in Synthetic Example 26, 10 ml of anhydrous pyridine, and 10 ml of anhydrous acetic acid were charged in a 200 ml eggplant-type flask. After replacing the internal atmosphere with argon, the mixture was stirred for 4 hours at room temperature. Water was added to the reaction mixture, followed by extraction twice with ether (200 ml x 2). The ether layer was washed with 5% hydrochloric acid, water, and saturated brine, once with each, and dried over MgSO₄. The solvent was evaporated under reduced pressure to obtain a residue. The residue was subjected to flash column chromatography (silica gel, 230-400 mesh; φ 4.5 cm x 10 cm, about 80 g; eluent: ethyl acetate:n-hexane = 1:1; pressure: 0.2 kg/cm²; 1 fraction = 80 ml) for separation, thus obtaining 11.57 g (yield 99%) of N-(4-methoxybenzyl)-N-(3-methoxybenzyl)acetamide as a colorless oily product from the first elution fraction (3-10).
N-(4-methoxybenzyl)-N-(3-methoxybenzyl)acetamide
IR νmax (KBr) cm⁻¹ : 2936, 2836, 1650, 1614, 1512, 1416, 1416, 1360, 1288, 1248, 1176, 1036, 986, 818, 778, 694, 516
¹H-NMR (δ ppm in DMSO-d₆ at 80°C): 2.08 (3H, S), 3.72 (3H, s), 3.74 (3H, s), 4.42 (2H, s), 4.44 (2H, s), 6.70-7.30 (8H, m)
MS m/z (%) [EI]: 299 (M⁺, 8), 178 (48), 136 (100)

| MS [HMS] (as C₁₈H₂₁O₃N): | |
|---|---|
| Calculated | 299.15207 |
| Found | 299.15137 |

### Synthetic Example 29

5.3 g (17.70 mmol) of N-(4-methoxybenzyl)-N-(3-methoxybenzyl)acetamide obtained in Synthetic Example 28 was placed in a 500 ml eggplant-type flask. After replacing the internal atmosphere with argon, 89 ml of anhydrous methylene chloride was added to dissolve the mixture. 89 ml (70.82 mmol) of a 0.8 M boron tribromide solution in methylene chloride was added under cooling with ice while stirring. The mixture was stirred for 30 minutes under cooling with ice and for 3 hours at room temperature. The reaction mixture was poured into ice water and extracted twice with ethyl acetate (400 ml x 2). The ethyl acetate layer was washed with water and saturated brine, once with each, and dried over MgSO₄. The solvent was evaporated under reduced pressure to obtain a residue. The residue was subjected to flash column chromatography (silica gel, 230-400 mesh; φ 6.5 cm x 5 cm, about 80 g; eluent: ethyl acetate; pressure: 0.2 kg/cm²; 1 fraction = 80 ml) for separation, thus obtaining 4.71 g (yield 98%) of N-(4-hydroxybenzyl)-N-(3-hydroxybenzyl)acetamide as a colorless amorphous product from the first elution fraction (3-6).
N-(4-hydroxybenzyl)-N-(3-hydroxybenzyl)acetamide
m.p. (°C): 128-130
IR νmax (KBr) cm⁻¹ : 3312 (OH), 1590, 1518, 1490, 1454, 1440, 1352, 1276, 1246, 1232, 1156, 1144.998, 846, 830, 772, 744, 688, 582, 564, 486
¹H-NMR (δ ppm in DMSO-d₆ at 80°C): 2.07 (3H, s), 4.35 (4H, s), 6.60-7.20 (8H, m), 8.10 (1H, br, D₂O exchangable)
MS m/z (%) [EI]: 271 (M⁺, 10), 164 (43), 122 (100)

| MS [HMS] (as C₁₆H₁₇O₃N): | |
|---|---|
| Calculated | 271.12088 |
| Found | 271.12138 |

### Synthetic Example 30

1.0 g (3.89 mmol) of N-(4-hydroxybenzyl)-N-methyl-3-hydroxybenzamide obtained in Synthetic Example 25 was placed in a 100 ml eggplant-type flask equipped with a reflux condenser and a dropping funnel. After replacing the internal atmosphere with argon, 18 ml of anhydrous tetrahydrofuran was added to dissolve the mixture. 18 ml (18.00 mmol) of a 1 M boran/tetrahydrofuran solution was added under cooling with ice while stirring. The mixture was stirred for 30 minutes at room temperature and heated under refluxing for 3 hours. After the addition of 2 ml of 6 N hydrochloric acid under cooling with ice in portions, the reaction mixture was heated under reflux for one hour. After neutralizing (pH 8) with a saturated aqueous solution of sodium bicarbonate under cooling with ice, the reaction mixture was extracted twice with ethyl acetate (100 ml x 2). The ethyl acetate layer was washed with water and saturated brine, once with each, and dried over Na₂SO₄. The solvent was evaporated under reduced pressure to obtain a residue. The residue was subjected to flash column chromatography (silica gel, 230-400 mesh; φ 4.5 cm x 10 cm, about 80 g; eluent: methanol:chloroform = 1:10; pressure: 0.2 kg/cm²; 1 fraction = 80 ml) for separation, thus obtaining 620 mg (yield 66%) of N-(4-hydroxybenzyl)-N-methyl-3-hydroxybenzylamine as a colorless amorphous product from the first elution fraction (3-11).
N-(4-hydroxybenzyl)-N-methyl-3-hydroxybenzylamine
IR νmax (KBr) cm⁻¹ : 3500-2500 (OH, NH), 3024, 2952, 2800, 1592, 1514, 1464, 1368, 1250, 1172, 998, 846, 824, 786, 756, 696
¹H-NMR (δ ppm in CD₃OD): 2.15 (3H, s), 4.87 (4H, s), 6.70-7.20 (8H, m)
MS m/z (%) [EI]: 243 (M⁺, 12), 136 (100), 107 (90), 78 (51)

| MS [HMS] (as C₁₅H₁₇NO₂): | |
|---|---|
| Calculated | 243.12599 |
| Found | 243.12619 |

### Synthetic Example 31

2.0 g (7.77 mmol) of N-(4-methoxybenzyl)-3-methoxybenzylamine obtained in Synthetic Example 26 was placed in a 200 ml eggplant-type flask. 39 ml of anhydrous methylene chloride was added to dissolve this compound. After replacing the internal atmosphere with argon, 39 ml (31.09 mmol) of a 0.8 M boron tribromide solution in methylene chloride was added under cooling with ice while stirring. The mixture was stirred for 30 minutes under cooling with ice and for 3 hours at room temperature. After the addition of water and methanol under cooling with ice, the reaction mixture was stirred for 1 hour at room temperature, then heated for 15 minutes at outside temperature of 80°C. The solvent was evaporated under reduced pressure, and the residue was neutralized (pH 8) with a saturated aqueous solution of sodium bicarbonate and extracted twice with ethyl acetate (150 ml x 2). The ethyl acetate layer was washed with water and saturated brine, once with each, and dried over Na₂SO₄. The solvent was evaporated under reduced pressure to obtain a residue. The residue was subjected to flash column chromatography (silica gel, 230-400 mesh; φ 4.5 cm x 10 cm, about 80 g; eluent: methanol:chloroform =1:10; pressure: 0.2 kg/cm²; 1 fraction = 80 ml) for separation, thus obtaining 1.26 g (yield 71%) of N-(4-hydroxybenzyl)-3-hydroxybenzylamine as a colorless amorphous product from the first elution fraction (13-30).
N-(4-hydroxybenzyl)-3-hydroxybenzylamine
m.p. (°C): 165-170
IR νmax (KBr) cm⁻¹ : 3020, 2784, 2616 (NH, OH), 1602, 1480, 1452, 1414, 1284, 1216, 1170, 1110, 994, 888, 830, 786, 696, 512
¹H-NMR (δ ppm in CD₃OD): 3.64 (2H, s), 3.65 (2H, s), 6.65-7.20 (8H, m)
MS m/z (%) [EI]: 229 (M⁺, 1), 122 (74), 106 (100), 78 (89)

| MS [HMS] (as C₁₄H₁₅NO₂): | |
|---|---|
| Calculated | 229.11024 |
| Found | 229.11024 |

### Synthetic Example 32

(1) 40.0 g (238 mmol) of 3-hydroxy-4-methoxybenzoic acid, 72 g (524 mmol) of potassium carbonate, and 240 ml of anhydrous dimethylformamide were charged into a 1 l eggplant-type flask. The internal atmosphere was replaced with argon and 62 ml (524 mmol) of benzyl bromide was added. After the addition of 10 g of potassium iodide, the mixture was stirred overnight at room temperature. Water (1 l) was added to the reaction mixture, followed by stirring for 30 minutes at room temperature. Deposited insoluble components were collected by filtration to obtain a white solid product. The solid was recrystallized from ethyl acetate/n-hexane to obtain 68.5 g (yield 83%) of benzyl 3-benzyloxy-4-methoxybenzoate as colorless fine needle-like crystals.
Benzyl 3-benzyloxy-4-methoxybenzoate
IR νmax (KBr) cm⁻¹ : 3036, 2948, 1712, 1596, 1586, 1514, 1426, 1268, 1208, 1182, 1134, 1102, 1010, 758, 748, 694
¹H-NMR (δ ppm in CDCl₃): 3.93 (3H, s), 5.17 (2H, s), 5.32 (2H, s), 6.90 (1H, d, J=8.5Hz), 7.30-7.50 (10H, m), 7.63 (1H, d, J=2.0Hz), 7.72 (1H, dd, J=8.5, 2.0Hz)
MS m/z (%) [EI]: 348 (M⁺, 12), 91 (100)
(2) 68.0 g (195 mmol) of benzyl 3-benzyloxy-4-methoxybenzoate, 500 ml of ethanol, and 500 ml of 10% sodium hydroxide were charged into a 2 l eggplant-type flask equipped with a reflux condenser, and heated for 30 minutes under refluxing. After removing ethanol by evaporation under reduced pressure, the reaction mixture was dissolved into water, acidified with 5% hydrochloric acid to deposit white precipitate. The precipitate was collected by filtration, thus obtaining 51.22 g (yield 100%) of colorless power of 3-benzyloxy-4-methoxybenzoic acid.
3-Benzyloxy-4-methoxybenzoic acid
IR νmax (KBr) cm⁻¹ : 3200-2800 (OH), 2968, 2836, 1668, 1596, 1584, 1512, 1442, 1386, 1296, 1272, 1230, 1182, 1140, 1112, 1028, 1000, 946, 774, 744, 700, 630
¹H-NMR (δ ppm in CDCl₃): 3.95 (3H, s), 5.19 (2H, s), 6.94 (1H, d, J=8.3Hz), 7.25-7.55 (5H, m), 7.66 (1H, d, J=2.0Hz), 7.77 (1H, dd, J=8.3, 2.0Hz)
MS m/z (%) [EI]: 258 (M⁺, 68), 91 (100)
(3) 45.0 g (302 mmol) of 4-hydroxy-3-methoxybenzonitrile, 50 g (362 mmol) of potassium carbonate, and 270 ml of anhydrous dimethylformamide were charged into a 1 l eggplant-type flask. The internal atmosphere was replaced with argon and 43 ml (362 mmol) of benzyl bromide was added. After stirring overnight at room temperature, water (1 l) was added to the reaction mixture, followed by stirring for 30 minutes at room temperature. Deposited insoluble components were collected by filtration to obtain a white solid product. This solid was recrystallized from ethyl acetate/n-hexane (about 2:1) to obtain 69.47 g (yield 96%) of 4-benzyloxy-3-methoxybenzonitrile as colorless fine needle-like crystals.
4-Benzyloxy-3-methoxybenzonitrile
¹H-NMR (δ ppm in CDCl₃): 3.90 (3H, s), 5.20 (2H, s), 6.90 (1H, d, J=8.3Hz), 7.10 (1H, d, J=2.0Hz), 7.21 (1H, dd, J=8.3, 2.0Hz), 7.30-7.50 (5H, m)
MS m/z (%) [EI]: 239 (M⁺, 4), 91 (100)
(4) 18.0 g (476 mmol) of lithium aluminum hydride was placed in a 2 l three-necked flask equipped with a reflux condenser and a dropping funnel. After replacing the internal atmosphere with argon, 400 ml of anhydrous tetrahydrofuran was added to suspend the lithium aluminum hydride. To the suspension was added dropwise to a solution of 76.0 (318 mmol) of 4-benzyloxy-3-methoxybenzonitrile in 400 ml of anhydrous tetrahydrofuran under cooling with ice over about 30 minutes. The mixture was stirred for 1 hour at room temperature. To the reaction mixture were successively added 18 ml of water (in portions), 18 ml of 15% sodium hydroxide, and 50 ml of water, thus producing a white cake. The cake was collected by filtration and washed well with ether. The organic layer of the filtrate was washed with water and saturated brine, one time each, and dried over K₂CO₃. The solvent was evaporated under reduced pressure to obtain 77.09 g (yield 99%) of 4-benzyloxy-3-methoxybenzylamine as an yellow oily product.
4-Benzyloxy-3-methoxybenzylamine
IR νmax (KBr) cm⁻¹ : 3400-2800 (NH), 1590, 1512, 1466, 1382, 1264, 1232, 1140, 1028, 1006, 746, 696
¹H-NMR (δ ppm in CDCl₃): 3.80 (2H, s), 3.90 (3H, s), 5.14 (2H, s), 6.76 (1H, dd, J=8.1, 2.0Hz), 6.84 (1H, d, J=8.1Hz), 6.89 (1H, d, J=2.0Hz), 7.25-7.50 (5H, m)
MS m/z (%) [EI]: 243 (M⁺, 17), 91 (100)
(5) 40.0 g (155 mmol) of 3-benzyloxy-4-methoxybenzoic acid was placed in a 500 ml eggplant-type flask equipped with a calcium chloride tube and a reflux condenser, and 160 ml of anhydrous benzene was added to it to suspend the 3-benzyloxy-4-methoxybenzoic acid. 34 ml (465 mmol) of thionyl chloride was added under cooling with ice and the mixture was heated (outside temperature: 60-70°C) for 2 hours while stirring. The reaction mixture was distilled under reduced pressure (distillation was repeated two or three times with the addition of anhydrous benzene) to obtain a white solid product. 240 ml of an anhydrous benzene solution of this acid chloride solid was added dropwise under cooling with ice while stirring into a mixed solution of 37.3 g (153 mmol) of 4-benzyloxy-3-methoxybenzylamine, 160 ml of anhydrous benzene, and 560 ml of 10% sodium hydroxide placed in another 2 l egg-type flask equipped with a dropping funnel. After stirring for one hour at room temperature, the reaction mixture was evaporated under reduced pressure to remove benzene. The insoluble components were collected by filtration (thoroughly washed with water) to obtain a white solid product. This solid was recrystallized from ethyl acetate to obtain 61.05 g (yield 83%) of N-(4-benzyloxy-3-methoxybenzyl)-3-benzyloxy-4-methoxybenzamide as colorless needle-like crystals.
N-(4-benzyloxy-3-methoxybenzyl)-3-benzyloxy-4-methoxybenzamide
m.p. (°C): 160-162
IR νmax (KBr) cm⁻¹ : 3290 (NH), 3064, 2908, 2864, 1628, 1580, 1512, 1462, 1420, 1382, 1330, 1304, 1270, 1224, 1136, 1010, 744, 696
¹H-NMR (δ ppm in DMSO-d₆): 3.87 (3H, s), 3.90 (3H, s), 4.52 (2H, d, J=5.6Hz), 5.15 (2H, s), 5.17 (2H, s), 6.20 (1H, t, J=5.6Hz, D₂O exchangeable), 6.80-6.90 (4H, m), 7.25-7.55 (12H, m)
MS m/z (%) [EI]: 483 (M⁺, 13), 392 (14), 241 (33), 91 (100)
(6) 7.0 g (14.48 mmol) of N-(4-benzyloxy-3-methoxybenzyl)-3-benzyloxy-4-methoxybenzamide and 700 mg of 5% palladium-carbon were placed in a 500 ml eggplant-type flask. After the addition of 200 ml of ethanol, the mixture was subjected to catalytic hydrogenation and stirred at room temperature overnight. The reaction mixture was filtered and the filtrate was evaporated under reduced pressure to obtain a residue. The residue was subjected to flash column chromatography (silica gel, 230-400 mesh; φ 4.5 cm x 5 cm, about 40 g; eluent: methanol:chloroform = 1:10; pressure: 0.2 kg/cm²; 1 fraction = 100 ml) to separate 4.29 g of colorless amorphous N-(4-hydroxy-3-methoxybenzyl)-3-hydroxy-4-methoxybenzamide. This amorphous product was further recrystallized from ethyl acetate to obtain 3.98 g (yield 91%) colorless needle-like crystals.
N-(4-hydroxy-3-methoxybenzyl)-3-hydroxy-4-methoxybenzamide
m.p. (°C): 149-151
IR νmax (KBr) cm⁻¹ : 3484, 3368, 3140 (OH, NH), 3012, 2844, 1634, 1614, 1582, 1548, 1518, 1454, 1430, 1314, 1262, 1202, 1124, 1026, 1020, 926, 886, 762, 622, 556
¹H-NMR (δ ppm in DMSO-d₆): 3.74 (3H, s), 3.81 (3H, s), 4.33 (2H, d, J=5.9Hz), 6.70 (2H, s), 6.91 (1H, d, J=8Hz), 6.96 (1H, d, J=8Hz), 7.35 (1H, d, J=8Hz), 7.36 (1H, d, J=8Hz), 8.67 (1H, t, J=5.9Hz, D₂O exchangeable), 8.80 (1H, br, D₂O exchangeable), 9.15 (1H, br, D₂O exchangeable)
MS m/z (%) [EI]: 303 (M⁺, 52), 151 (100)

| EA : | | | |
|---|---|---|---|
| Calculated | C: 63.35 | H: 5.65 | N: 4.62 |
| Found | C: 63.70 | H: 5.73 | N: 4.90 |

### Synthetic Example 33

(1) 20.0 g (41.36 mmol) of N-(4-benzyloxy-3-methoxybenzyl)-3-benzyloxy-4-methoxybenzamide obtained in Synthetic Example 32 and 100 ml of anhydrous dimethylsulfoxide were charged into a 500 ml eggplant-type flask. The internal atmosphere was replaced with argon and 4.6 g (82.72 mmol) of potassium hydroxide powder was added, followed by stirring for 30 minutes at room temperature. After the addition of 7.7 ml (124.08 mmol) of methyl iodide, the mixture was stirred overnight at room temperature. The reaction mixture was charged into ice water (containing a small amount of hexane) to collect insoluble components by filtration, thus obtaining a white solid product. This solid was recrystallized from ethyl acetate/n-hexane to obtain 19.59 g (yield 95%) of N-(4-benzyloxy-3-methoxybenzyl)-N-methyl-3-benzyloxy-4-methoxybenzamide as colorless fine needle-like crystals.
N-(4-benzyloxy-3-methoxybenzyl)-N-methyl-3-benzyloxy-4-methoxybenzamide
m.p. (°C): 105-106
IR νmax (KBr) cm⁻¹ : 3064, 3032, 2936, 2864, 1622, 1512, 1454, 1394, 1326, 1268, 1254, 1222, 1132, 1022, 818, 740, 694
¹H-NMR (δ ppm in DMSO-d₆, at 80°C): 2.82 (3H, s), 3.77 (3H, s), 3.81 (3H, s), 4.46 (2H, s), 5.04 (2H, s), 5.06 (2H, s), 6.70-7.10 (6H, m), 7.25-7.50 (10H, m)
MS m/z (%) [EI]: 497 (M⁺, 6), 406 (34), 241 (25), 151 (14), 91 (100)
(2) 6.0 g (12.06 mmol) of N-(4-benzyloxy-3-methoxybenzyl)-N-methyl-3-benzyloxy-4-methoxybenzamide and 600 mg of 5% palladium-carbon were charged into a 500 ml eggplant-type flask. After the addition of 180 ml of ethanol, the mixture was subjected to catalytic hydrogenation and stirred overnight at room temperature. The reaction mixture was filtered and the filtrate was evaporated under reduced pressure to obtain a residue. The residue was recrystallized from methanol to obtain 3.27 g (yield 86%) of colorless needle-like crystals of N-(4-hydroxy-3-methoxybenzyl)-N-methyl-3-hydroxy-4-methoxybenzamide.
N-(4-hydroxy-3-methoxybenzyl)-N-methyl-3-hydroxy-4-methoxybenzamide
m.p. (°C): 166-168
IR νmax (KBr) cm⁻¹ 3520 (OH), 3376 (OH), 2940, 2852, 1626, 1578, 1518, 1464, 1424, 1394, 1282, 1220, 1122, 1032, 1022, 992, 896, 832, 820, 758, 724
¹H-NMR (δ ppm in DMSO, at 80°C): 2.82 (3H, s), 3.75 (3H, s), 3.79 (3H, s), 4.45 (2H, s), 6.65 (1H, dd, J=8,2Hz), 6.75 (1H, d, J=8Hz), 6.77 (1H, dd, J=8,2Hz), 6.84 (1H, d, J=2Hz), 6.86 (1H, d, J=2Hz), 6.93 (1H, d, J=8Hz), 8.70 (2H, br, D₂O exchangeable)
MS m/z (%) [EI]: 317 (M⁺, 38), 166 (34), 151 (100)

| EA | | | |
|---|---|---|---|
| Calculated | C: 64.34 | H: 6.03 | N: 4.41 |
| Found | C: 63.98 | H: 6.27 | N: 4.30 |

### Synthetic Example 34

(1) 20.0 g (41.36 mmol) of N-(4-benzyloxy-3-methoxybenzyl)-3-benzyloxy-4-methoxybenzamide obtained in Synthetic Example 32 was placed in a 500 ml two-necked flask equipped with a reflux condenser. After replacing the internal atmosphere with argon, 100 ml of anhydrous tetrahydrofuran was added to dissolve the compound. To the solution was added 103 ml (103 mmol) of a 1 M boran/tetrahydrofuran solution under cooling with ice. The mixture was stirred for 1 hour at room temperature and heated under refluxing for one hour. 30 ml of 4 N hydrochloric acid was added under cooling with ice and the mixture was heated under refluxing for a further one hour. The reaction mixture was neutralized and alkalinized with the addition of 4 N aqueous solution of sodium hydroxide under cooling with ice. After having been made alkaline, the reaction mixture was extracted once with ether and once with ethyl acetate. The organic layer was washed with saturated brine once, dried over K₂CO₃, and the solvent was evaporated under reduced pressure, thus obtaining a residue. The residue was recrystallized from chloroform/n-hexane to obtain 17.28 g (yield 89%) of N-(4-benzyloxy-3-methoxybenzyl)-3-benzyloxy-4-methoxybenzylamine as colorless fine needle-like crystals.
N-(4-benzyloxy-3-methoxybenzyl)-3-benzyloxy-4-methoxybenzylamine
m.p. (°C): 85-88
IR νmax (KBr) cm⁻¹ : 3336, 3036, 2912, 2840, 1590, 1514, 1456, 1420, 1388, 1262, 1232, 1160, 1136, 1014, 848, 804, 738, 696
¹H-NMR (δ ppm in CDCl₃): 3.67 (2H, s), 3.69 (2H, s), 3.87 (3H, s), 3.88 (3H, s), 5.13 (2H, s), 5.15 (2H, s), 6.73 (1H, dd, J=8.3, 1.5Hz), 6.79 (1H, d, J=8.3Hz), 6.85 (1H, d, J=1.5Hz), 6.87 (1H, d, J=8.3Hz), 6.88 (1H, dd, J=8.3, 1.5Hz), 6.93 (1H, d, J=1.5Hz)
MS m/z (%) [EI]: 469 (M⁺, 2), 467 (6), 242 (27), 227 (21), 137 (20), 91 (100)
(2) 6.0 g (12.78 mmol) of N-(4-benzyloxy-3-methoxybenzyl)-3-benzyloxy-4-methoxybenzylamine and 300 mg of 5% palladium-carbon were charged into a 500 ml eggplant-type flask. After the addition of 180 ml of ethanol, the mixture was subjected to catalytic hydrogenation and stirred overnight at room temperature. The reaction mixture was filtered and the filtrate was evaporated under reduced pressure to obtain a residue. The residue was subjected to flash column chromatography (silica gel, 230-400 mesh; φ 4.5 cm x 5 cm, about 40 g; eluent: chloroform:methanol = 1:10; pressure: 0.2 kg/cm²; 1 fraction = 100 ml) to separate 2.71 mg (yield 73%) of colorless amorphous N-(4-hydroxy-3-methoxybenzyl)-3-hydroxy-4-methoxybenzylamine as the first elution fraction (15-27). Colorless needle-like crystals were obtained by recrystallization from methanol.
N-(4-hydroxy-3-methoxybenzyl)-3-hydroxy-4-methoxybenzylamine
m.p. (°C): 97-100
IR νmax (KBr) cm⁻¹ : 3552, 3000, 2832 (OH, NH), 1530, 1502, 1454, 1416, 1328, 1298, 1258, 1226, 1160, 1132, 1038, 1020, 866, 854, 804, 760, 568
¹H-NMR (δ ppm in DMSO-d₆): 3.51 (2H, s), 3.53 (2H, s), 3.73 (3H, s), 3.75 (3H, s), 6.65-6.95 (6H, m), 8.80 (2H, br, D₂O exchangeable)
MS m/z (%) [EI]: 289 (M⁺, 23), 138 (100)

| MS [HMS] (as C₁₆H₁₉NO₄): | |
|---|---|
| Calculated | 289.13139 |
| Found | 289.13154 |

### Synthetic Example 35

10.0 g (20.09 mmol) of N-(4-benzyloxy-3-methoxybenzyl)-N-methyl-3-benzyloxy-4-methoxybenzamide obtained in Synthetic Example 33 was placed in a 500 ml two-necked flask equipped with septum rubber and a reflux condenser. After replacing the internal atmosphere with argon, 50 ml of anhydrous tetrahydrofuran was added to dissolve the mixture.

50 ml (50 mmol) of a 1 M boran/tetrahydrofuran solution was added under cooling with ice, and the mixture was stirred for one hour at room temperature and heated for one hour under refluxing.

After the addition of 15 ml of 4 N hydrochloric acid under cooling with ice, the reaction mixture was heated under reflux for a further one hour. The reaction mixture was neutralized and alkalinized with 4 N sodium hydroxide, and extracted twice with ethyl acetate. The organic layer was washed once with saturated brine and dried over K₂CO₃. The solvent was evaporated under reduced pressure to obtain a residue. This residue was subjected to flash column chromatography (silica gel, 230-400 mesh; φ 6.5 cm x 5 cm, about 75 g; eluent: ethyl acetate:n-hexane = 1:1; pressure: 0.2 kg/cm²; 1 fraction = 100 ml) to separate 8.33 g (yield 86%) of colorless amorphous N-(4-benzyloxy-3-methoxybenzyl)-N-methyl-3-benzyloxy-4-methoxybenzylamine as the first elution fraction (5-50). 7.86 g (yield 81%) of colorless fine needle-like crystals were obtained by recrystallization from a mixed solvent of ethyl acetate and n-hexane.
N-(4-benzyloxy-3-methoxybenzyl)-N-methyl-3-benzyloxy-4-methoxybenzylamine
m.p. (°C): 94-95
IR νmax (KBr) cm⁻¹ : 3060, 3036, 2948, 2920, 2780, 2756, 1606, 1590, 1514, 1454, 1374, 1320, 1266, 1230, 1162, 1128, 1022, 1002, 848, 800, 746, 698
¹H-NMR (δ ppm in CDCl₃): 2.13 (3H, s), 3.42 (4H, s), 3.87 (3H, s), 3.89 (3H, s), 5.14 (2H, s), 5.15 (2H, s), 6.70-7.00 (6H, m), 7.20-7.50 (10H, m)
MS m/z (%) [EI]: 483 (M⁺, 9), 256 (41), 227 (29), 137 (27), 91 (100)
(2) 6.0 g (12.41 mmol) of N-(4-benzyloxy-3-methoxybenzyl)-N-methyl-3-benzyloxy-4-methoxybenzylamine and 300 mg of 5% palladium-carbon were placed in a 500 ml eggplant-type flask. After the addition of 180 ml of ethanol, the mixture was subjected to catalytic hydrogenation and stirred at room temperature overnight. The reaction mixture was filtered and the filtrate was evaporated under reduced pressure to obtain a residue. The residue was subjected to flash column chromatography (silica gel, 230-400 mesh; φ 4.5 cm x 5 cm, about 40 g; eluent: methanol:chloroform = 1:20; pressure: 0.2 kg/cm²; 1 fraction = 100 ml) to separate 2.58 g (yield 69%) of colorless amorphous N-(4-hydroxy-3-methoxybenzyl)-N-methyl-3-hydroxy-4-methoxybenzylamine as the first elution fraction (3-6).
N-(4-hydroxy-3-methoxybenzyl)-N-methyl-3-hydroxy-4-methoxybenzylamine
m.p. (°C): 37-40
IR νmax (KBr) cm⁻¹ : 3424 (OH), 2940 (OH), 2840, 2792, 1594, 1514, 1462, 1368, 1276, 1242, 1154, 1130, 1030, 866, 800, 760, 570
¹H-NMR (δ ppm in CDCl₃): 2.16 (3H, s), 3.40 (2H, s), 3.43 (2H, s), 3.87 (3H, s), 3.89 (3H, s), 5.60 (2H, br, D₂O exchangeable), 6.70-7.00 (6H, m)
MS m/z (%) [EI]: 303 (M⁺, 2), 166 (37), 137 (62), 107 (29), 83 (100)

### Synthetic Example 36

(1) 10.0 g (21.29 mmol) of N-(4-benzyloxy-3-methoxybenzyl)-3-benzyloxy-4-methoxybenzylamine obtained in Synthetic Example 34, 10 ml of anhydrous pyridine, and 10 ml of anhydrous acetic acid were charged into a 200 ml eggplant-type flask. The internal atmosphere was replaced with argon, followed by stirring for 5 hours at room temperature. After the addition of water, the reaction mixture was extracted with ether twice (200 ml x 2). The ether layer was washed with 5% hydrochloric acid, water, and saturated brine, once with each, and dried (MgSO₄). The solvent was evaporated under reduced pressure to obtain a residue. The residue was subjected to flash column chromatography (silica gel, 230-400 mesh; φ 6.5 cm x 20 cm, about 300 g; eluent: ethyl acetate:n-hexane = 1:1; pressure: 0.2 kg/cm²; 1 fraction = 100 ml) to separate 8.72 g (yield 80%) of colorless amorphous N-(4-benzyloxy-3-methoxybenzyl)-N-(3-benzyloxy-4-methoxybenzyl)acetamide as the first elution fraction (27-50).
N-(4-benzyloxy-3-methoxybenzyl)-N-(3-benzyloxy-4-methoxybenzyl)acetamide
IR νmax (KBr) cm⁻¹ : 2932, 1646, 1512, 1414, 1262, 1230, 1138, 1028, 740
¹H-NMR (δ ppm in DMSO d₆, at 80°C): 2.05 (3H, s), 3.72 (3H, s), 3.76 (3H, s), 4.37 (2H, s), 4.39 (2H, s), 5.00 (2H, s), 5.04 (2H, s), 6.60-7.05 (6H, m), 7.30-7.50 (10H, m)
MS m/z (%) [EI]: 511 (M⁺, 6), 284 (48), 242 (35), 227 (23), 91 (100)
(2) 6.0 g (11.73 mmol) of N-(4-benzyloxy-3-methoxybenzyl)-N-(3-benzyloxy-4-methoxybenzyl)acetamide and 600 mg of 5% palladium-carbon were placed in a 500 ml eggplant-type flask. After the addition of 180 ml of ethanol, the mixture was subjected to catalytic hydrogenation and stirred at room temperature overnight. The reaction mixture was filtered and the filtrate was evaporated under reduced pressure to obtain a residue. The residue was subjected to flash column chromatography (silica gel, 230-400 mesh; φ 4.5 cm x 5 cm, about 40 g; eluent: methanol:chloroform = 1:20; pressure: 0.2 kg/cm²; 1 fraction = 100 ml) to separate 3.52 g (yield 91%) of colorless amorphous N-(4-hydroxy-3-methoxybenzyl)-N-(3-hydroxy-4-methoxybenzyl)acetamide as the first elution fraction (2-3).
N-(4-hydroxy-3-methoxybenzyl)-N-(3-hydroxy-4-methoxybenzyl)acetamide
m.p. (°C): 35-40
IR νmax (KBr) cm⁻¹ : 3364 (OH), 2936, 2840, 2792, 1626, 1512, 1430, 1368, 1276, 1128, 1030, 988, 798, 748, 664, 590
¹H-NMR (δ ppm in DMSO-d₆ at 80°C): 2.06 (3H, s), 3.72 (3H, s), 3.75 (3H, s), 4.32 (2H, s), 4.34 (2H, s), 6.50-6.90 (6H, m), 8.50 (1H, br, D₂O exchangeable), 8.58 (1H, br, D₂O exchangeable)
MS m/z (%) [EI]: 331 (M⁺, 4), 194 (44), 152 (100), 137 (32)

| MS [HMS] (as C₁₈H₂₁NO₅): | |
|---|---|
| Calculated | 331.14195 |
| Found | 331.14265 |

### Synthetic Example 37

A solution of benzoyl chloride (53.0 ml) in ether (400 ml) was added dropwise to a solution of benzylamine (50.0 ml) and triethylamine (64.0 ml) in ether (500 ml) at room temperature, and the mixture was stirred for one hour. The reaction mixture was filtered, and the mother liquor was concentrated to dryness under reduced pressure. The concentrate was recrystallized from ethanol to obtain colorless needle-like crystals of N-benzylbenzamide (83.1g, 86%).
N-Benzylbenzamide
m.p. (°C): 105.0-105.9
IR νmax (KBr) cm⁻¹ : 3292, 3084, 3060, 3028, 2924, 1640, 1602, 1550, 1492, 1454, 1418, 1362, 1326, 1316, 1302, 1262, 986, 724, 694
¹H-NMR (δ ppm in CDCl₃): 4.62 (2H, d, J=5.9), 7.52-7.25 (8H, m), 7.81-7.76 (2H, m)
MS m/z (%) [EI-MS]: 211 (M⁺, 48), 210 (16), 106 (23) 105 (100), 103 (12), 77 (43), 51 (21)

| MS [HMS] (as C₁₄H₁₃NO): | |
|---|---|
| Calculated | 211.09967 |
| Found | 211.09927 |

| EA | | | |
|---|---|---|---|
| Calculated | C: 79.59 | H: 6.20 | N: 6.63 |
| Found | C: 79.70 | H: 6.28 | N: 6.54 |

### Synthetic Example 38

A solution of benzoyl chloride (45.2 ml) in ether (400 ml) was added dropwise to a solution of N-methylbenzylamine (50.0 ml) and triethylamine (54.0 ml) in ether (1000 ml), and the mixture was stirred for one hour at room temperature. The reaction mixture was filtered and concentrated to dryness under reduced pressure. The oily product thus obtained was distilled by a Kugel Roar under reduced pressure (150°C, 2.2 x 10-1 mmHg) to obtain N-benzyl-N-methylbenzamide (69.9 g, 80%).
N-Benzyl-N-methylbenzamide
IR νmax (NaCl) cm⁻¹ : 3060, 3028, 2920, 1632, 1604, 1580, 1498, 1480, 1450, 1402, 1360, 1308, 1264, 1222, 1178, 1158, 1068, 1026, 974, 930, 788, 714, 698, 664, 634
¹H-NMR (δ ppm in DMSO-d₆ at 80°C): 2.85 (3H, s), 4.58 (2H, s), 7.42-7.28 (10H, m)
MS m/z (%) [EI-MS]: 225 (M⁺, 38), 224 (39), 106 (12) 105 (100), 91 (16), 77 (55), 51 (16)

| MS [HMS] (as C₁₅H₁₅NO): | |
|---|---|
| Calculated | 225.11532 |
| Found | 25.11432 |

### Synthetic Example 39

A solution of acetyl chloride (1.8 ml) in ether (8.0 ml) was added dropwise to a solution of dibenzylamine (4.3 ml) and triethylamine (3.0 ml) in ether (45.0 ml) at 0°C. After stirring the mixture at 0°C for 30 minutes, deposited salt was filtered, and the filtrate was concentrated to dryness under reduced pressure to obtain crude N,N-dibenzylacetamide (6.08 g). The crude product was subjected to column chromatography (silica gel, 230-400 mesh; 300 g; φ 70 mm; eluent: hexane:ethyl acetate = 2:1; pressure: 0.5 kg/cm²; 1 fraction = 150 ml; fractions 10-17) to obtain colorless oil of N,N-dibenzylacetamide (5.2 g, 96%).
N,N-dibenzylacetamide
IR νmax (NaCl) cm⁻¹ : 3060, 3028, 2924, 1644, 1606, 1586, 1494, 1470, 1452, 1422, 1362, 1329, 1304, 1242, 1206, 1020, 984, 954, 730, 698, 664, 604
¹H-NMR (δ ppm in CDCl₃): 2.21 (3H, s), 4.44 (2H, s), 4.60 (2H, s), 7.38-7.18 (10H, m)
MS m/z (%) [EI-MS]: 239 (M⁺, 4), 148 (52), 106 (100), 91 (48), 79 (10), 77 (10), 65 (15), 43 (18)

| MS [HMS] (as C₁₆H₁₇NO): | |
|---|---|
| Calculated | 239.13105 |
| Found | 239.13175 |

### Synthetic Example 40

(1) 3-O-methyldopamine hydrochloride (106.9 mg) was suspended in ethanol (3.0 ml). An ethanol solution of sodium hydroxide (1.65 ml) was added dropwise to the suspension at 0°C. After the further addition of an ethanol solution of vanillin (78.7 mg/2.0 ml), the mixture was stirred for 15 hours at room temperature. The reaction mixture was concentrated to dryness under reduced pressure, and the concentrate was recrystallized from methanol to obtain colorless fine crystals of N-(4-hydroxy-3-methoxybenzylidene)-4-hydroxy-3-methoxy- phenetylamine (102.1 mg, 65.5%).
N-(4-hydroxy-3-methoxybenzylidene)-4-hydroxy-3-methoxyphenetylamine
IR νmax (KBr) cm⁻¹ : 2992, 2952, 2832, 1658, 1590, 1512, 1464, 1430, 1370, 1350, 1332, 1294, 1278, 1262, 1234, 1212, 1192, 1158, 1132, 1064, 1026, 854, 830, 818, 752
¹H-NMR (δ ppm in CD₃OD): 2.88 (2H, t, J=7.3), 3.73 (3H, s), 3.75 (2H, t, J=7.3), 3.86 (3H, s), 6.76-6.61 (4H, m), 7.08 (1H, dd, J=2.8, 8.3), 7.34 (1H, d, J=2.0), 7.87 (1H, s)
MS m/z (%) [EI-MS]: 301 (M⁺, 11), 165 (14), 164 (96), 138 (10), 137 (100), 122 (16), 109 (16), 104 (26), 94 (20), 77 (14), 65 (12)

| MS [HMS] (as C₁₇H₁₉NO₄): | |
|---|---|
| Calculated | 301.13134 |
| Found | 301.13124 |

(2) 5% Palladium-carbon catalyst (62.8 mg) was suspended into methanol (10.0 ml) and hydrogen was absorbed into the suspension. A solution of N-(4-hydroxy-3-methoxybenzylidene)-4-hydroxy-3-methoxyphenetylamine (503.8 mg) in methanol (15.0 ml) was added to the suspension, followed by stirring for 3 days under a hydrogen atmosphere. The reaction mixture was filtered, and the filtrate was concentrated to dryness under reduced pressure to obtain N-(4-hydroxy-3-methoxyphenetyl)-4-hydroxy-3-methoxybenzylamine (105.0 mg). The catalyst collected by filtration was washed with 5% hydrochloric acid, adjusted to pH 12 with an aqueous solution of 5% sodium hydroxide, and extracted with ethyl acetate. The organic layer was concentrated under reduced pressure to dryness, thus obtaining N-(4-hydroxy-3-methoxyphenetyl)-4-hydroxy-3-methoxybenzylamine (310.0 mg). The products were combined to obtain 415 mg (81%) of N-(4-hydroxy-3-methoxyphenetyl)-4-hydroxy-3-methoxybenzylamine in total.
N-(4-hydroxy-3-methoxyphenetyl)-4-hydroxy-3-methoxybenzylamine
IR νmax (KBr) cm⁻¹ : 3172, 2964, 2832, 2800, 2648, 1602, 1526, 1466, 1454, 1434, 1412, 1372, 1284, 1260, 1212, 1160, 1128, 1032, 862, 818, 806, 790
¹H-NMR (δ ppm in CD₃OD): 2.78-2.72 (4H, m), 3.66 (2H, s), 3.80 (3H, s), 3.81 (3H, s), 6.85-6.59 (6H, m)
MS m/z (%) [EI-MS]: 303 (M⁺, 2), 167 (6), 166 (18), 138 (43), 137 (100), 136 (8), 122 (10), 107 (9), 65 (5)

| MS [HMS] (as C₁₇H₂₁NO₄): | |
|---|---|
| Calculated | 303.14709 |
| Found | 303.14709 |

(3) N-(4-hydroxy-3-methoxyphenetyl)-4-hydroxy-3-methoxybenzylamine (6.09 g) was suspended into methanol (100.0 ml) and the suspension was added dropwise to 35% formalin (1.9 ml). After stirring for 2 hours to homogenize, the mixture was added to a suspension of 5% palladium-carbon catalyst (1.09 g) in methanol (50.0 ml) and stirred for 45 hours under a hydrogen atmosphere. The reaction mixture was concentrated to dryness under reduced pressure. The concentrate was dissolved into ethyl acetate and washed with water. The organic layer was dried over anhydrous sodium sulfate, concentrated to dryness under reduced pressure to obtain crude N-(4-hydroxy-3-methoxyphenetyl)-N-methyl-4-hydroxy-3-methoxybenzylamine (5.88 g). The crude product was subjected to column chromatography (silica gel, 230-400 mesh; 300 g; pressure: 0.4 kg/cm²; eluent: chloroform:methanol = 10::1; 1 fraction = 100 ml; fractions 9-17) to obtain a colorless amorphous powder of N-(4-hydroxy-3-methoxyphenetyl)-N-methyl-4-hydroxy-3-methoxybenzylamine (5.67 g, 89%).
N-(4-hydroxy-3-methoxyphenetyl)-N-methyl-4-hydroxy-3-methoxybenzylamine
IR νmax (KBr) cm⁻¹ : 3420, 3000, 2940, 2836, 2800, 1600, 1516, 1466, 1432, 1368, 1276, 1236, 1154, 1122, 1032, 928, 856, 814
¹H-NMR (δ ppm in CD₃OD): 2.26 (3H, s), 2.72-2.55 (4H, m), 3.46 (2H, s), 3.78 (3H, s), 3.79 (3H, s), 6.81-6.51 (6H, m)
MS m/z (%) [FD-MS]: 317 (M⁺), 180

### Synthetic Example 41

N,N-dibenzylamine (4.0 ml) was dissolved into methanol (30.0 ml) and 35% formalin (2.1 ml) was added dropwise to the solution. This solution was added to a suspension of 5% palladium-carbon catalyst (502.9 mg) in methanol (10.0 ml) and stirred for 16 hours under a hydrogen atmosphere. The reaction mixture was filtered and concentrated to dryness under reduced pressure, thus obtaining crude N,N-dibenzylmethylamine (4.3 g). The crude product was distilled by a Kugel Roar (2 x 10-1 mmHg, 170°C) to obtain colorless oil of N,N-dibenzyl-N-methylamine (3.34 g, 76%).
N,N-dibenzyl-N-methylamine
IR νmax (NaCl) cm⁻¹ : 3084, 3060, 3028, 2980, 2944, 2876, 2836, 2784, 2712, 1602, 1494, 1454, 1418, 1336, 1346, 1314, 1292, 1262, 1242, 1210, 1192, 1132, 1074, 1024, 976, 910, 864
¹H-NMR (δ ppm in D₂O): (N,N-dibenzylmethylamine maleate) 2.77 (3H, s), 4.36 (4H, s), 6.30 (2H, s), 7.57-7.46 (10H, m)
MS m/z (%) [EI-MS]: 211 (M⁺, 30), 210 (11), 134 (40), 120 (22), 92 (14), 91 (100), 65 (13)

| MS [HMS] (as C₁₅H₁₇N): | |
|---|---|
| Calculated | 211.13616 |
| Found | 211.13796 |

### Synthetic Example 42

A solution of p-methoxybenzoyl chloride (50.0 ml) in ether (200 ml) was added dropwise to a solution of p-methoxybenzylamine (47.0 ml) and triethylamine (60.0 ml) in ether (450 ml) taking a care for the reaction heat. After the addition of ether (800 ml), the reaction mixture was stirred for 16 hours at room temperature. The reaction mixture was filtered, water (1,500 ml) was added to the filtrate, and the mixture was stirred for 2 hours at room temperature. The solution was filtered and the residue was dried under reduced pressure to obtain N-(4-methoxybenzyl)-4-methoxybenzamide (77.68 g, 79%).
N-(4-methoxybenzyl)-4-methoxybenzamide
IR νmax (KBr) cm⁻¹ : 3328, 3096, 3056, 3004, 2956, 2932, 2908, 2836, 1632, 1608, 1574, 1552, 1504, 1462, 1442, 1422, 1322, 1306, 1284, 1256, 1190, 1176, 1110, 1026, 810, 770, 668
¹H-NMR (δ ppm in CDCl₃): 3.79 (3H, s), 3.83 (3H, s), 4.55 (2H, d, J=5.6), 6.87 (2H, d, J=8.8), 6.90 (2H, d, J=8.8), 7.27 (2H, d, J=8.8), 7.74 (2H, d, J=8.8)
MS m/z (%) [EI-MS]: 271 (M⁺, 42), 136 (26), 135 (100), 121 (13), 92 (10), 77 (18)

| MS [HMS] (as C₁₆H₁₇NO₃ (M⁺): | |
|---|---|
| Calculated | 271.12072 |
| Found | 271.12012 |

| EA | | | |
|---|---|---|---|
| Calculated | C: 70.83 | H: 6.32 | N: 5.16 |
| Found | C: 70.80 | H: 6.37 | N: 5.29 |

### Synthetic Example 43

N-(4-methoxybenzyl)-4-methoxybenzamide (4.57 g) obtained in Synthetic Example 42 was dissolved into dichloromethane (80.0 ml) and cooled at 0°C. A dichloromethane solution of boron tribromide (85.0 ml) was added dropwise to the solution, followed by stirring for one hour at 0°C to room temperature. The reaction mixture was poured into ice water (1000 ml) and stirred for 2 hours. Deposited precipitate was filtered and dried at room temperature under reduced pressure to obtain crude N-(4-hydroxybenzyl)-4-hydroxybenzamide (3.79 g). The crude product was recrystallized from methanol/chloroform (12 ml/15 ml) to obtain colorless prismatic crystals of N-(4-hydroxybenzyl)-4-hydroxybenzamide (3.49 g, 70%).
N-(4-hydroxybenzyl)-4-hydroxybenzamide
m.p. (°C): 216.9-217.7
IR νmax (KBr) cm⁻¹ : 3444, 3400, 3020, 2932, 1634, 1594, 1542, 1516, 1502, 1444, 1430, 1368, 1312, 1274, 1246, 1216, 1170, 1118, 1104, 1012, 990, 854, 830, 754
¹H-NMR (δ ppm in DMSO-d₆): 4.32 (2H, d, J=5.9), 6.70 (2H, d, J=8.3), 6.79 (2H, d, J=8.5), 7.10 (2H, d, J=8.3), 7.75 (2H, d, J=8.5), 8.66 (1H, t, J=5.9)
MS m/z (%) [EI-MS]: 243 (M⁺, 51), 123 (13), 122 (45), 121 (100), 107 (13), 93 (20), 77 (12), 65 (26)

| MS [HMS] (as C₁₉H₂₃NO₄): | |
|---|---|
| Calculated | 329.16259 |
| Found | 329.16119 |

### Synthetic Example 44

N-(4-methoxybenzyl)-4-methoxybenzamide (8.07 g) obtained in Synthetic Example 42 was dissolved into dimethylsulfoxide (30.0 ml) and powder of potassium hydroxide (8.34 g) was added to the solution, followed by stirring for 30 minutes at room temperature. After adding methyl iodide (6.0 ml) dropwise, the mixture was stirred for 30 minutes at room temperature. The reaction mixture was poured into ice water and extracted twice with ether. The organic layer was washed with water, dried over anhydrous sodium sulfate, and concentrated to dryness under reduced pressure. The concentrate was purified by column chromatography (silica gel, 230-400 mesh; 400 g; φ 60 mm, length 200 mm; pressure: 0.5 kg/cm²; eluent: chloroform:methanol = 50:1; 1 fraction = 150 ml, fractions 5-13) to obtain colorless oil of N-(4-methoxybenzyl)-N-methyl-4-methoxybenzamide (8.47 g, 99%).
N-(4-methoxybenzyl)-N-methyl-4-methoxybenzamide
IR νmax (NaCl) cm⁻¹ : 2956, 2932, 2836, 1630, 1612, 1586, 1512, 1484, 1462, 1444, 1396, 1302, 1252, 1174, 1068, 1030, 840
¹H-NMR (δ ppm in DMSO-d₆): 2.84 (3H, s), 3.75 (3H, s), 3.79 (3H, s), 4.52 (2H, s), 6.91 (2H, d, J=9.0), 6.96 (2H, d, J=9.0), 7.18 (2H, d, J=9.0), 7.38 (2H, s, J=9.0) MS m/z (%) [EI-MS]: 285 (M⁺, 33), 284 (11), 150 (171), 136 (10), 135 (100), 121 (25), 107 (10), 77 (19)

| MS [HMS] (as C₁₇H₁₉NO₃ (M⁺)): | |
|---|---|
| Calculated | 285.13654 |
| Found | 285.13704 |

### Synthetic Example 45

N-(4-methoxybenzyl)-N-methyl-4-methoxybenzamide (5.97 g) obtained in Synthetic Example 44 was dissolved into dichloromethane (100.0 ml) and cooled to 0°C, followed by dropwise addition of a dichloromethane solution of boron tribromide (63 ml). The reaction mixture was charged into ice water (1000 ml) and extracted with ethyl acetate. The organic layer was washed with water, dried over anhydrous sodium sulfate, and concentrated under reduced pressure to obtain a colorless amorphous powder of N-(4-hydroxybenzyl)-N-methyl-4-hydroxybenzamide (4.62 g, 86%).
N-(4-hydroxybenzyl)-N-methyl-4-hydroxybenzamide
IR νmax (KBr) cm⁻¹ : 3396, 3020, 2964, 2804, 2964, 2804, 2704, 2644, 1616, 1574, 1518, 1484, 1452, 1432, 1402, 1364, 1272, 1214, 1186, 1178, 1168, 1102, 844, 828, 814
¹H-NMR (δ ppm in DMSO-d₆): 2.81 (3H, s), 4.45 (2H, s), 6.74 (2H, D, J=8.3), 6.78 (2H, d, J=8.3), 7.04 (2H, d, J=8.1), 7.26 (2H, d, J=8.1)
MS m/z (%): 257 (M⁺, 40), 256 (13), 136 (25), 122 (28), 121 (100), 107 (100), 93 (26), 65 (19)

| MS [HMS] (as C₁₅H₁₅NO₃): | |
|---|---|
| Calculated | 257.10527 |
| Found | 257.10557 |

### Synthetic Example 46

N-(4-methoxybenzyl)-4-methoxybenzamide (30.51 g) obtained in Synthetic Example 42 was suspended in tetrahydrofuran (200 ml) and cooled to 0°C. To the suspension was added a boran/tetrahydrofuran solution (330 ml). The mixture was stirred for 1 hour at room temperature and refluxed for 2 hours, then allowed to cool to room temperature, whereupon 6 N hydrochloric acid (50.0 ml) was added and the mixture was refluxed for 1 hour. After cooling to the room temperature, the reaction mixture adjusted to about pH 10 with the addition of 6 N aqueous solution of sodium hydroxide. After concentrating under reduced pressure, the concentrate was extracted with ether. The organic layer was washed with water, dried over anhydrous K₂CO₃, and concentrated to dryness under reduced pressure. The concentrate was purified by passing twice through a chromatographic column (silica gel, 230-400 mesh; 800 g; φ 100 mm, length 170 mm; pressure: 0.5 kg/cm²; eluent: chloroform:methanol = 20:1) to obtain a colorless oil of N,N-bis(4-methoxybenzyl)amine (25.36 g, 97%) from an eluted fraction of 1,000-2,300 ml.

2.07 g of N,N-bis(4-methoxybenzyl)amine thus obtained was dissolved in ether (50 ml) and the solution was added dropwise to an ether solution of maleic acid (1.15 g/70 ml). After stirring for 4 hours at room temperature, precipitate produced was collected by filtration and dried under reduced pressure to obtain N,N-bis(4-methoxybenzyl)amine maleate (2.85 g, 95%).
N,N-bis(4-methoxybenzyl)amine maleate
IR νmax (KBr) cm⁻¹ : 3004, 2960, 2836, 1616, 1584, 1518, 1480, 1468, 1412, 1386, 1368, 1306, 1254, 1216, 1186, 1032, 988, 876, 820, 714
¹H-NMR (δ ppm in D₂O): 3.86 (6H, s), 4.20 (4H, s), 6.26 (2H, s), 7.06 (4H, d, J=8.8), 7.41 (4H, d, J=8.8)
MS m/z (%) [EI-MS]:
[N,N-bis(4-methoxybenzyl)amine]
257 (M⁺, 11), 149 (10), 136 (32), 121 (100)

| MS [HMS] (as C₁₆H₁₉NO₂(M⁺)): | |
|---|---|
| Calculated | 257.14157 |
| Found | 257.14177 |

| EA | | | |
|---|---|---|---|
| Calculated | C: 64.33 | H: 6.21 | N: 3.75 |
| Found | C: 64.39 | H: 6.11 | N: 3.92 |

### Synthetic Example 47

A dichlorometane solution of boron tribromide (87 ml) was added dropwise under cooling with ice to a dichlorometane (130.0 ml) solution of N,N-bis(4-methoxybenzyl)amine (7.46 g) obtained in Synthetic Example 46. The reaction mixture was stirred for 3 hours at temperatures between 0°C and room temperature, poured into ice water, and extracted with ethyl acetate. The water layer was neutralized with sodium bicarbonate and extracted with ethyl acetate. The ethyl acetate layer was combined with the ethyl acetate layer of the first extraction, washed with water, dried over anhydrous sodium sulfate, and concentrated under reduced pressure. The concentrate was treated with activated carbon to obtain a colorless oil of N,N-bis(4-hydroxybenzyl)amine (2.61 g, 39%).
N,N-bis(4-hydroxybenzyl)amine
IR νmax (KBr) cm⁻¹ : 3024, 2808, 2596, 1612, 1512, 1452, 1368, 1244, 1170, 1110, 1066, 927, 830, 758, 624, 548, 510
¹H-NMR (δ ppm in DMSO-d₆): 3.52 (4H, s), 6.69 (4H, d, J=8.5), 7.10 (4H, d, J=8.5)
MS m/z (%) [EI-MS]: 229 (M⁺, 1), 122 (15), 107 (20), 78 (53), 52 (32), 51 (24), 50 (16)

| MS [HMS] (as C₁₄H₁₅NO₂ ): | |
|---|---|
| Calculated | 229.11028 |
| Found | 229.11058 |

### Synthetic Example 48

N,N-bis(4-methoxybenzyl)amine (7.59 g) obtained in Synthetic Example 46 was dissolved in methanol (30.0 ml), and 35% formalin (3.0 ml ) was added to the solution, followed by stirring at room temperature. The reaction mixture was added to a suspension of 5% palladium-carbon catalyst (1.0 g) in methanol (20.0 ml) and stirred for 6 hours in a hydrogen stream. The reaction mixture was filtered, concentrated under reduced pressure, and purified by column chromatography (silica gel, 230-400 mesh; 400 g; φ=60 mm, length=200 mm; pressure: 0.4 kg/cm²; eluent: chloroform:methanol = 20::1; 1 fraction = 150 ml; fractions 5-15) to obtain a colorless oil of N,N-bis(4-methoxybenzyl)-N-methylamine (7.25 g, 90%).

An ether (50 ml) solution of N,N-bis(4-methoxybenzyl)-N-methylamine (2.02 g) thus obtained was added dropwise to an ether solution of maleic acid (1.15 g/70 ml). After stirring for 3 hours at 0°C, precipitate produced was collected by filtration and dried under reduced pressure to obtain the maleic acid salt (2.51 g, 83%) as a colorless amorphous powder.
N,N-bis(4-methoxybenzyl)-N-methylamine
IR νmax (NaCl) cm⁻¹ : 2996, 2984, 2908, 2836, 2784, 1614, 1586, 1512, 1462, 1442, 1336, 1300, 1246, 1172, 1104, 1034, 1010, 818
¹H-NMR (δ ppm in DMSO-d₆): 2.42 (3H, s), 3.79 (6H, s), 4.18 (4H, br), 6.06 (2H, s), 7.02 (4H, d, J=8.6), 7.43 (4H, d, J=8.6)
MS m/z (%) [EI-MS]: 271 (M⁺, 22), 162 (5), 150 (17), 148 (16), 135 (12), 122 (17), 121 (100), 91 (8), 77 (9), 54 (12), 45 (23)

| MS [HMS] (as C₁₇H₂₁NO₂ (M⁺)): | |
|---|---|
| Calculated | 271.15711 |
| Found | 271.15681 |

### Synthetic Example 49

To N-(4-hydroxybenzyl)-N-methyl-4-hydroxybenzamide (2.00 g) obtained in Synthetic Example 45 suspended in tetrahydrofuran (10.0 ml) was added dropwise a 1 M tetrahydrofuran solution of boran/tetrahydrofuran complex. The mixture was refluxed for 2.5 hours. After cooling with ice water, 6 N hydrochloric acid (8.0 ml) was added and the mixture was again refluxed for 1 hour. The reaction mixture was cooled with ice and adjusted to about pH 8 with the addition of a saturated sodium bicarbonate solution. After concentrating under reduced pressure and evaporating tetrahydrofuran, the concentrate was extracted with ethyl acetate. The organic layer was washed with water, dried over anhydrous sodium sulfate, and concentrated under reduced pressure. The concentrate was purified by column chromatography (silica gel, 230-400 mesh; 250 g; φ 60 mm, length 120 mm; eluent: chloroform:methanol = 10:1; pressure: 0.4 kg/cm²; 1 fraction = 50 ml; fractions 19-35) to obtain a colorless amorphous powder of N,N-bis(4-hydroxybenzyl)-N-methylamine (1.31 g, 69%).
N,N-bis(4-hydroxybenzyl)-N-methylamine
IR νmax (KBr) cm⁻¹ : 3016, 2948, 2796, 2592, 1614, 1596, 1512, 1462, 1366, 1252, 1172, 1120, 1096, 1002, 992, 906, 830, 752, 512
¹H-NMR (δ ppm in DMSO-d₆): 2.05 (3H, s), 3.38 (4H, s), 6.70 (4H, d, J=8.5), 7.08 (4H, d, J=8.5), 8.83 (2H, s) MS m/z (%) [EI-MS]: 243 (M⁺, 1), 137 (9), 136 (12), 134 (11), 107 (29), 106 (100), 78 (56), 77 (16), 52 (32), 51 (25), 50 (17)

| MS [HMS] (as C₁₅H₁₇NO₂ (M⁺)): | |
|---|---|
| Calculated | 243.12593 |
| Found | 243.12633 |

### Synthetic Example 50

To a solution of N,N-bis(4-methoxybenzyl)amine (7.0 g) obtained in Synthetic Example 46 and triethylamine (4.5 ml) in ether (100.0 ml) was added dropwise an ether (20.0 ml) solution of acetyl chloride (2.1 ml). After stirring at room temperature, the reaction mixture was filtered and concentrated under reduced pressure. The concentrated residue was dissolved into ether, washed with water, dried over anhydrous sodium sulfate, and concentrated to dryness under reduced pressure. The residue was purified by column chromatography (silica gel, 230-400 mesh; φ 60 mm, length 200 mm; eluent: chloroform:methanol = 50:1; 1 fraction = 150 ml; fractions 10-17) to obtain a colorless oil of N,N-bis(4-methoxybenzyl)acetamide (6.99 g, 86%).
N,N-bis(4-methoxybenzyl)acetamide
IR νmax (NaCl) cm⁻¹ : 3000, 2936, 2836, 1644, 1614, 1586, 1512, 1464, 1442, 1416, 1360, 1302, 1246, 1176, 1110, 1032, 984, 920, 818, 758
¹H-NMR (δ ppm in DMSO-d₆): 2.08 (3H, s), 3.74 (6H, s), 4.39 (4H, s), 6.88 (4H, d, J=8.8), 7.11 (4H, d, J=8.8)
MS m/z (%) [EI-MS]: 299 (M⁺, 4), 178 (47), 137 (12), 136 (100), 122 (13), 121 (34), 77 (10)

| MS [HMS] (as C₁₈H₂₁NO₃): | |
|---|---|
| Calculated | 229.15205 |
| Found | 229.15125 |

### Synthetic Example 51

A 1 M dichlorometane solution of boron tribromide (48 ml) was added dropwise at 0°C to a solution of N,N-bis(4-methoxybenzyl)acetamide (4.0 g) obtained in Synthetic Example 50 in dichlorometane (70.0 ml). The reaction mixture was stirred for 18 hours at temperatures between 0°C and room temperature, poured into ice water, and extracted with ethyl acetate. The organic layer was washed with water, dried over anhydrous sodium sulfate, and concentrated under reduced pressure to obtain a colorless amorphous powder of N,N-bis(4-hydroxybenzyl)acetamide (2.03 g, 56%).
N,N-bis(4-hydroxybenzyl)acetamide
IR νmax (KBr) cm⁻¹ : 3288, 3204, 2944, 2884, 2792, 2676, 2592, 2496, 1598, 1584, 1512, 1498, 1452, 1380, 1350, 1326, 1294, 1242, 1206, 1172, 1152, 1102, 988, 828, 814, 562
¹H-NMR (δ ppm in DMSO-d₆): 2.06 (3H, s), 4.32 (4H, s), 6.71 (4H, d, J=8.6), 6.98 (4H, d, J=8.6), 9.04 (2H, br)
MS m/z (%) [EI-MS]: 271 (M⁺, 5), 165 (10), 164 (58), 123 (10), 122 (100), 107 (24), 106 (6), 77 (10)

| MS [HMS] (as C₁₆H₁₇NO₃): | |
|---|---|
| Calculated | 271.12079 |
| Found | 271.12019 |

### Synthetic Example 52

(1) N-(4-benzyloxy-3-methoxyphenetyl)-3-benzyloxy-4-methoxyphenylacetamide (17.08 g) obtained in Synthetic Example 18 was dissolved into tetrahydrofuran (85.0 ml) and cooled at 0°C. To the solution was added dropwise a boran/tetrahydrofuran solution slowly taking care not to form bubbles. The reaction mixture was refluxed for 1 hour and cooled to the room temperature. After the addition of 6 N hydrochloric acid (17.0 ml), the mixture was again refluxed for 50 minutes, adjusted to about pH 12 with the addition of 6 N sodium hydroxide aqueous solution. After evaporation of tetrahydrofuran under reduced pressure, the residue was extracted with about 1,000 ml of ether. The organic layer was washed with water, dried over anhydrous sodium sulfate, and concentrated under reduced pressure to dryness. The concentrate was recrystallized from ether to obtain colorless needle-like crystals of N-(4-benzyloxy-3-methoxyphenetyl)-3-benzyloxy-4-methoxyphenylamine (13.32 g, 80%).
N-(4-benzyloxy-3-methoxyphenetyl)-3-benzyloxy-4-methoxyphenetylamine
IR νmax (KBr) cm⁻¹ : 3416, 3260, 3032, 2936, 2836, 1606, 1590, 1516, 1466, 1454, 1420, 1386, 1336, 1260, 1232, 1158, 1138, 1018, 848, 802
¹H-NMR (δ ppm in CDCl₃): 2.84-2.71 (8H, m), 3.85 (6H, s), 5.11 (4H, s), 6.81-6.59 (6H, m), 7.45-7.25 (10H, m)
MS m/z (%) [EI-MS]: 497 (M⁺, 0.5), 402 (2), 360 (1), 284 (2), 270 (71), 137 (12), 91 (100), 42 (12)

| MS [HMS] (as C₃₂H₃₅NO₄): | |
|---|---|
| Calculated | 497.25655 |
| Found | 497.25565 |

(2) N-(4-benzyloxy-3-methoxyphenetyl)-3-benzyloxy-4-methoxyphenetylamine (4.59 g) was dissolved in methanol (30.0 ml), and 35% formalin (1.0 ml) was added to the solution, followed by stirring at room temperature. This solution was then added to a suspension of 5% palladium-carbon catalyst (1.03 g) in methanol (20.0 ml) and stirred overnight in a hydrogen atmosphere. The reaction mixture was filtered, concentrated to dryness under reduced pressure, and purified by column chromatography (silica gel, 230-400 mesh; 250 g; φ=60 mm, length=130 mm; eluent: chloroform:methanol = 10:1; pressure: 0.5 kg/cm²; 1 fraction = 900-1,000 ml) to obtain a colorless amorphous powder of N-(4-hydroxy-3-methoxyphenetyl)-N-methyl-3-hydroxy-4-methoxyphenetylamine (1.03 g, 35%).
N-(4-hydroxy-3-methoxyphenetyl)-N-methyl-3-hydroxy-4-methoxyphenetylamine
IR νmax (KBr) cm⁻¹ : 3328, 2972, 2932, 2824, 2548, 1590, 1512, 1470, 1368, 1272, 1228, 1158, 1122, 1034, 992, 956, 934, 870
¹H-NMR (δ ppm in CDCl₃): 2.40 (3H, s), 2.80-2.65 (8H. br), 3.87 (3H, s), 3.86 (3H, s), 6.85-6.64 (6H, m)
MS m/z (%) [FD-MS]: 332 (M⁺, 100)

| MS [HMS] (C₁₉H₂₅NO₄): | |
|---|---|
| Calculated | 331.17826 |
| Found | 331.17796 |

### Synthetic Example 53

(1) Vanillic acid (23.69 g), potassium carbonate (45.37 g), and potassium iodide (0.55 g) were dissolved or suspended in dimethylformamide (150 ml). After cooling to 0°C, 40.0 mg of benzyl bromide was added dropwise. The reaction mixture was stirred for 3 days at 0°C and poured into ice water, followed by stirring for 1 hour. Deposited crude crystals were collected by filtration, dried under reduced pressure, dissolved into ethyl acetate, and filtered while the mixture was hot. Ethyl acetate was evaporated under reduced pressure and the residue was crystallized from ethyl acetate to obtain colorless needle-like crystals of benzyl 4-benzyloxy-3-methoxybenzoate (43.97 g, 88%).
Benzyl 4-benzyloxy-3-methoxybenzoate
m.p. (°C): 88.2-89.0
IR νmax (KBr) cm⁻¹ : 1706, 1598, 1514, 1462, 1414, 1382, 1346, 1292, 1274, 1212, 1182, 1130, 1102, 1022, 982, 922, 872, 760, 746, 698
¹H-NMR (δ ppm in CDCl₃): 3.92 (3H, s), 5.21 (2H, s), 5.34 (2H, s), 6.88 (1H, d, J=8.3), 7.41-7.31 (10H, m), 7.59 (1H, d, J=2.0), 7.64 (1H, dd, J=8.3, 2.0)
MS m/z (%) [EI-MS]: 348 (M⁺, 14), 92 (9), 91 (100), 65 (6), 51 (1)

| MS [HMS] (as C₂₂H₂₀O₄): | |
|---|---|
| Calculated | 348.13619 |
| Found | 348.13619 |

(2) Benzyl 4-benzyloxy-3-methoxybenzoate (112.36 g) was dissolved into ethanol (350 ml). After the addition of a 5% sodium hydroxide aqueous solution (400 ml), the mixture was refluxed for 2 hours. The reaction mixture was concentrated under reduced pressure and ethanol was removed. After adjusting the mixture to about pH 1 with 3 N hydrochloric acid, deposited crude crystals were collected by filtration, dried under reduced pressure, and recrystallized from a mixed solvent of ethyl acetate an hexane to obtain colorless needle-like crystals of 4-benzyloxy-3-methoxybenzoic acid (73.67 g, 88%).
4-Benzyloxy-3-methoxybenzoic acid
IR νmax (KBr) cm⁻¹ : 1678, 1600, 1588, 1518, 1470, 1454, 1428, 1350, 1308, 1276, 1232, 1186, 1136, 1026
¹H-NMR (δ ppm in CDCl₃): 3.95 (3H, s), 5.23 (2H, s), 6.93 (1H, d, J=8.3), 7.42-7.33 (5H, m), 7.62 (1H, d, J=2.0), 7.70 (1H, dd, J=2.0, 8.3)
MS m/z (%) [EI-MS]: 258 (M⁺, 9), 92 (8), 91 (100), 89 (1), 79 (2), 77 (2), 65 (9), 51 (3)

| MS [HMS] (as C₁₅H₁₄O₄): | |
|---|---|
| Calculated | 258.08928 |
| Found | 258.08938 |

(3) 4-Benzyloxy-3-methoxybenzoic acid (51.59 g), thionyl chloride (62.0 ml), and benzene (200 ml) were refluxed for about 2.5 hours and concentrated under reduced pressure to dryness. The residue was dissolved into benzene (300 ml) to produce a benzene solution of 4-benzyloxy-3-methoxybenzoyl chloride. This benzene solution of 4-benzyloxy-3-methoxybenzoyl chloride was added dropwise to a mixture of a solution of 4-benzyloxy-3-methoxyphenetylamine (62.36 g) in benzene (200 ml) and a 10% sodium hydroxide solution (700 ml), followed by stirring at room temperature for 18 hours. The reaction mixture was concentrated under reduced pressure, filtered under reduced pressure, washed with water, dried, and recrystallized from ethyl acetate to obtain colorless needle-like crystals of N-(4-benzyloxy-3-methoxyphenetyl) -4-benzyloxy-3-methoxybenzamide (74.89 g, 58%).
N-(4-benzyloxy-3-methoxyphenetyl)-4-benzyloxy-3-methoxybenzamide
IR νmax (KBr) cm⁻¹ : 1632, 1600, 1584, 1544, 1512, 1470, 1454, 1418, 1386, 1320, 1272, 1260, 1230, 1200, 1186, 1160, 1138, 1024
¹H-NMR (δ ppm in CDCl₃): 2.84 (2H, t, J=6.8), 3.65 (2H, q, J=6.3), 3.83 (3H, s), 3.91 (3H, s), 5.13 (2H, s), 5.18 (2H, s), 7.07-6.66 (6H, m), 7.45-7.32 (10H, m), 253 (103)
MS m/z (%) [EI-MS]: 498 (M⁺, 2), 241 (13), 240 (24), 151 (10), 150 (11), 149 (11), 91 (100)

| MS [HMS] (as C₃₁H₃₁NO₅): | |
|---|---|
| Calculated | 497.22016 |
| Found | 497.21986 |

| EA: | | | |
|---|---|---|---|
| Calculated | C: 74.83 | H: 6.28 | N: 2.81 |
| Found | C: 74.83 | H: 6.27 | N: 3.10 |

(4) N-(4-benzyloxy-3-methoxyphenetyl)-4-benzyloxy-3-methoxybenzamide (4.24 g) was stirred together with 5% palladium-carbon catalyst (508.8 mg) in ethanol (80.0 ml) for 6 hours in a hydrogen atmosphere. The reaction mixture was filtered and concentrated to dryness under reduced pressure to obtain crude N-(4-hydroxy-3-methoxyphenetyl)-4-hydroxy-3-methoxybenzamide (2.81 g). This crude product was purified by column chromatography (silica gel, 230-400 mesh; 200 g; φ=60 mm; pressure: 0.4 kg/cm²; eluent: chloroform:methanol = 20:1; 1 fraction = 60 ml, fractions 8-13) to obtain a colorless amorphous powder of N-(4-hydroxy-3-methoxyphenetyl)-4-hydroxy-3-methoxybenzamide (2.61 g, 97%).
N-(4-hydroxy-3-methoxyphenetyl)-4-hydroxy-3-methoxybenzamide
IR νmax (KBr) cm⁻¹ : 3380, 1632, 1602, 1552, 1512, 1462, 1454, 1430, 1370, 1282, 1232, 1154, 1124, 1032, 866, 820, 756
¹H-NMR (δ ppm in CDCl₃): 2.85 (2H, t, J=6.8), 3.66 (2H, q, J=6.8), 3.92 (3H, s), 3.85 (3H, s), 5.57 (1H, s), 5.96 (1H, s), 5.16-5.04 (1H, m), 6.73-6.70 (2H, m), 6.90-6.85 (2H, m), 7.08 (1H, dd, J=2.0, 8.3), 7.41 (1H, d, J=2.0)
MS m/z (%) [EI-MS]: 317 (M⁺, 4), 151 (61), 150 (100), 137 (11), 123 (11)

| MS [HMS] (as C₁₇H₁₉NO₅): | |
|---|---|
| Calculated | 317.12621 |
| Found | 317.12431 |

### Synthetic Example 54

(1) Dimethylsulfoxide (65.0 ml) and potassium hydroxide (3.02 g) were added to N-(4-benzyloxy-3-methoxyphenetyl)-4-benzyloxy-3-methoxybenzamide (10.05 g) obtained in Synthetic Example 53, and the mixture was stirred for 1 hour at room temperature. After adding methyl iodide (2.5 ml) dropwise, the mixture was stirred for 13 hours at room temperature. The reaction mixture was poured into ice water, and deposited crystals were separated by decantation and dissolved into ethyl acetate. The organic layer was washed with water, dried over anhydrous sodium sulfate, and concentrated to dryness under reduced pressure. The concentrate was purified by column chromatography (silica gel, 230-400 mesh; 800 g; φ 10 cm; eluent: chloroform:methanol = 50:1; pressure: 0.4 kg/cm²; 1 fraction = 150 ml, fractions 16-30) to obtain a colorless crystalline powder of N-(4-benzyloxy-3-methoxyphenetyl)-N-methyl-4-benzyloxy-3-methoxybenzamide (10.16 g, 97%).
N-(4-benzyloxy-3-methoxyphenetyl)-N-methyl-4-benzyloxy-3-methoxybenzamide
IR νmax (KBr) cm⁻¹ : 2932, 2864, 1632, 1604, 1586, 1512, 1488, 1466, 1454, 1412, 1380, 1328, 1262, 1226, 1202, 1176, 1156, 1140, 1022, 1002, 854, 742
¹H-NMR (δ ppm in DMSO-d₆): 2.90 (3H, s), 3.73 (3H, s), 3.76 (3H, s), 5.03 (2H, s), 5.10 (2H, s), 2.77 (2H, d, J=6.8), 3.55 (2H, t, J=6.8), 7.01-6.59 (6H, m), 7.45-7.27 (10H, m)
MS m/z (%) [EI-MS]: 511 (M⁺, 2), 241 (31), 241 (38), 151 (8), 149 (8), 92 (9), 91 (100)

| MS [HMS] (as C₃₂H₃₃NO₅): | |
|---|---|
| Calculated | 511.23593 |
| Found | 511.23603 |

| EA: | | | |
|---|---|---|---|
| Calculated | C: 75.12 | H: 6.50 | N: 2.71 |
| Found | C: 74.76 | H: 6.48 | N: 2.98 |

(2) N-(4-benzyloxy-3-methoxyphenetyl)-N-methyl-4-benzyloxy-3-methoxybenzamide (6.98 g) and 5% palladium-carbon catalyst (586.3 mg) were stirred in ethanol (130.0 ml) for 18 hours in a hydrogen atmosphere. The reaction mixture was filtered, concentrated to dryness under reduced pressure, and purified by column chromatography (silica gel, 230-400 mesh; 300 g; eluent: chloroform:methanol = 20:1; pressure: 0.4 kg/cm²; 1 fraction = 100 ml, fractions 5-13) to obtain a colorless crystalline powder of N-(4-hydroxy-3-methoxyphenetyl)-N-methyl-4-hydroxy-3-methoxybenzamide (4.19 g, 92%).
N-(4-hydroxy-3-methoxyphenetyl)-N-methyl-4-hydroxy-3-methoxybenzamide
IR νmax (KBr) cm⁻¹ : 3356, 3096, 3012, 2992, 2948, 2864, 2836, 2784, 1594, 1524, 1482, 1452, 1402, 1302, 1276, 1260, 1228, 1206, 1196, 1158, 1126, 1022, 818
¹H-NMR (δ ppm in DMSO-d₆): 2.73 (2H, t, J=7.0), 2.90 (3H, s), 3.52 (2H, t, J=7.0), 3.75 (3H, s), 3.72 (3H, s), 6.78-6.52 (6H, m)
MS m/z (%) [EI-MS]: 331 (M⁺, 2), 182 (18), 151 (100), 150 (41), 123 (12)

| MS [HMS] (as C₁₈H₂₁NO₅): | |
|---|---|
| Calculated | 331.14193 |
| Found | 331.14073 |

### Synthetic Example 55

A 1 M tetrahydrofuran solution of boran/tetrahydrofuran (100 ml) was added dropwise to a tetrahydrofuran (50 ml) solution of N-(4-hydroxy-3-methoxyphenetyl)-4-hydroxy-3-methoxybenzamide (5.25 g) obtained in Synthetic Example 53 at 0°C. The reaction mixture was refluxed for 2 hours and then allowed to cool. After adding 6 N hydrochloric acid (20.0 ml), the reaction mixture was refluxed for a further 1 hour, then adjusted to about pH 8 with the addition of a 6 N sodium hydroxide aqueous solution. The organic layer was washed with water, dried over anhydrous sodium sulfate, concentrated under reduced pressure to dryness, and recrystallized from tetrahydrofuran to obtain N-(4-hydroxy-3-methoxyphenetyl)-4-hydroxy-3-methoxybenzylamine (2.76 g, 55%) as a light yellow crystalline powder.
N-(4-hydroxy-3-methoxyphenetyl)-4-hydroxy-3-methoxybenzylamine
IR νmax (KBr) cm⁻¹ : 3416, 3148, 2988, 2956, 2832, 2608, 2432, 1600, 1516, 1462, 1422, 1264, 1194, 1152, 1126, 1032, 938, 898, 820
¹H-NMR (δ ppm in DMSO-d₆): 2.69-2.62 (4H, m), 3. 62 (2H, s), 3.73 (6H, s), 6.88-6.55 (6H, m)
MS m/z (%) [EI-MS]: 303 (M⁺, 7), 220 (16), 206 (10), 205 (61), 138 (9), 137 (8), 106 (100), 55 (7), 42 (7)

| MS [HMS] (as C₁₇H₂₁NO₄): | |
|---|---|
| Calculated | 303.14711 |
| Found | 303.14761 |

### Synthetic Example 56

(1) N-(4-benzyloxy-3-methoxyphenetyl)-4-benzyloxy-3-methoxybenzamide (25.99 g) obtained in Synthetic Example 53 was dissolved into tetrahydrofuran (250 ml) and cooled at 0°C. To the solution was added dropwise a 1 M tetrahydrofuran solution of boran/tetrahydrofuran complex (180 ml) while taking care not to form bubbles. The mixture was refluxed for one hour and the resulting reaction mixture was allowed to cool. After adding 6 N hydrochloric acid, the reaction mixture was refluxed for a further 1 hour. This mixture was then allowed to cool and adjusted to about pH 11 with the addition of a 6 N sodium hydroxide aqueous solution. After evaporation of tetrahydrofuran under reduced pressure, the deposited solid was separated by decantation, dissolved into ethyl acetate, washed with water, and dried over anhydrous potassium carbonate. The organic layer was concentrated under reduced pressure to dryness to obtain crude N-(4-benzyloxy-3-methoxyphenetyl) -4-benzyloxy-3-methoxybenzylamine (32.24 g). This crude product was purified by column chromatography (silica gel, 230-400 mesh; 1,000 g; φ = 10cm; eluent: chloroform:methanol = 20:1; pressure: 0.4 kg/cm²; 1 fraction = 150 ml, fractions 13-25) to obtain a colorless crystalline powder of N-(4-benzyloxy-3-methoxyphenetyl)-4-benzyloxy-3-methoxybenzylamine (14.09 g, 56%).
N-(4-benzyloxy-3-methoxyphenetyl)-4-benzyloxy-3-methoxybenzylamine
IR νmax (KBr) cm⁻¹ : 2912, 2864, 1606, 1594, 1514, 1466, 1454, 1420, 1382, 1322, 1252, 1226, 1158, 1138, 1116, 1028, 1008, 1000, 856, 744
¹H-NMR (δ ppm in CDCl₃): 2.86-2.75 (4H, m), 3.72 (2H, s), 3.86 (3H, s), 3.85 (3H, s), 5.13 (2H, s), 5.12 (2H, s), 6.85-6.64 (6H, m), 7.45-7.25 (10H, m)
MS m/z (%) [EI-MS]: 482 [(M-1)⁺, 3), 255 (13), 227 (21), 137 (8), 92 (9), 91 (100), 65 (12)

| MS [HMS] (as C₃₁H₃₂NO₄ (M-1)⁺): | |
|---|---|
| Calculated | 482.23304 |
| Found | 482.23284 |

(2) N-(4-benzyloxy-3-methoxyphenetyl)-4-benzyloxy-3-methoxybenzylamine (8.63 g) was dissolved into pyridine (30.0 ml). To the solution was added dropwise acetic acid anhydride (5.5 ml), and the mixture was stirred for 18 hours at room temperature. The reaction mixture was poured into ice water, extracted with ethyl acetate. The organic layer was dried over anhydrous sodium sulfate and concentrated under reduced pressure to dryness to obtain crude N-(4-benzyloxy-3-methoxyphenetyl)-N-(4-benzyloxy-3-methoxybenzyl)acetamide (9.42 g). This crude product was purified by column chromatography (silica gel, 230-400 mesh; 400 g; eluent: chloroform; pressure: 0.4 kg/cm²; 1 fraction = 100 ml, fractions 40-55) to obtain N-(4-benzyloxy-3-methoxyphenetyl)-N-(4-benzyloxy-3-methoxybenzyl)acetamide (6.68 g, 71%) as a colorless viscous product.
N-(4-benzyloxy-3-methoxyphenetyl)-N-(4-benzyloxy-3-methoxybenzyl)acetamide
IR νmax (NaCl) cm⁻¹ : 3584, 3032, 3000, 2936, 1642, 1592, 1512, 1454, 1416, 1384, 1322, 1264, 1228, 1158, 1140, 1032, 740, 696
¹H-NMR (δ ppm in DMSO-d₆): 1.96 (3H, s), 2.69 (2H, t, J=7.6), 3.44 (2H, t, J=7.6), 3.76 (3H, s), 3.76 (3H, s), 4.41 (2H, s), 5.03 (2H, s), 5.05 (2H, s), 6.64 (1H, dd, J=1.7, 8.1), 6.73 (1H, dd, J=1.7, 8.1), 6.77 (1H, d, J=1.7), 6.85 (1H, d, J=1.7), 6.90 (1H, d, J=8.1), 6.96 (1H, d, J=8.1), 7.38-7.26 (10H, m)
MS m/z (%) [EI-MS]: 525 (M⁺, 2), 285 (18), 240 (19), 91 (100)

| MS [HMS] (as C₃₃H₃₅NO₅): | |
|---|---|
| Calculated | 525.25155 |
| Found | 525.25185 |

(3) N-(4-benzyloxy-3-methoxyphenetyl)-N-(4-benzyloxy-3-methoxybenzyl)acetamide (6.35 g) was stirred in 5% palladium-carbon catalyst (512.4 mg) and ethanol (80.0 ml) for 18 hours in a hydrogen atmosphere. The reaction mixture was filtered and concentrated to dryness under reduced pressure, to obtain a light yellow amorphous powder of N-(4-hydroxy-3-methoxyphenetyl)-N-(4-hydroxy-3-methoxybenzyl)acetamide (4.05 g, 97%). N-(4-hydroxy-3-methoxyphenetyl)-N-(4-hydroxy-3-methoxybenzyl)acetamide
IR νmax (KBr) cm⁻¹ : 3380, 3012, 2936, 1620, 1598, 1514, 1452, 1428, 1368, 1274, 1238, 1212, 1154, 1122, 1032, 852, 818
¹H-NMR (δ ppm in DMSO-d₆): 1.96 (3H, s), 2.64 (2H, t, J=7.1), 3.40 (2H, t, J=7.1), 3.75 (3H, s), 3.74 (3H, s), 4.32 (2H, s), 6.78-6.52 (6H, m)
MS m/z (%) (EI-MS]: 345 (M⁺, 1), 195 (11), 150 (63), 137 (100), 122 (7), 107 (5)

| MS [HMS] (as C₁₉H₂₃NO₅): | |
|---|---|
| Calculated | 345.15768 |
| Found | 345.15888 |

### Synthetic Example 57

(1) 4-Benzyloxy-3-methoxyphenetylamine acetate (13.06 g) and paraformaldehyde (1.32 g) were dissolved in formic acid (51.0 ml) and stirred at 50°C for 15 hours. The reaction mixture was concentrated under reduced pressure and dissolved in ethanol (128 ml). An ethanol solution of oxalic acid (8.78 g/128 ml) was added to the solution. Deposited precipitate was filtered under reduced pressure and dried at 50°C under reduced pressure to obtain a light yellow crystalline powder of 7-benzyloxy-6-methoxy-1,2,3,4-tetrahydroisoquinoline oxalate (12.95 g, 88%).
7-Benzyloxy-6-methoxy-1,2,3,4-tetrahydroisoquinoline oxalate
IR νmax (KBr) cm⁻¹ : 3448, 3069, 2952, 1614, 1520, 1454, 1424, 1402, 1384, 1340, 1290, 1260, 1228, 1206, 1122, 1078, 1022, 476
¹H-NMR (δ ppm in DMSO-d₆, at 40°C): 2.89 (2H, t, J=6.4), 3.31 (2H, t, J=6.4), 3.76 (3H, s), 4.12 (2H, s), 5.04 (2H, s), 6.82 (1H, s), 6.90 (1H, s), 7.45-7.29 (5H, m)
MS m/z (%) [EI-MS]: 269 (M⁺, 43), 178 (67), 149 (20), 91 (100), 44 (98), 43 (20)

| MS [HMS] (C₁₇H₁₉NO₂ (M⁺)): | |
|---|---|
| Calculated | 269.14150 |
| Found | 269.14071 |

(2) 4-Benzyloxy-3-methoxybenzoic acid (0.5 g) was dissolved into benzene (2.0 ml), and thionyl chloride (1.0 ml) was added to the solution. After refluxing for 2 hours, the reaction mixture was concentrated under reduced pressure to dryness. Benzene (2.0 ml) was added to the residue produce a benzene solution of 4-benzyloxy-3-methoxybenzoyl chloride.
6-methoxy-7-benzyloxy-1,2,3,4-tetrahydroisoquinoline oxalate (0.7278 g) was suspended into benzene (4.0 ml), and a 5% sodium hydroxide solution (7.0 ml) was added to it. Said benzene solution of 4-benzyloxy-3-methoxybenzoyl chloride (2.0 ml) was added dropwise to the reaction mixture, followed by stirring at room temperature for 2 hours. The reaction mixture was extracted with benzene. The organic layer was washed with 3 N sodium hydroxide solution, water, 3 N hydrochloric acid, water, saturated aqueous solution of sodium bicarbonate, and saturated brine, in sequence, dried over anhydrous sodium sulfate, and concentrated under reduced pressure to dryness, to obtain a colorless crystalline powder of N-(4-benzyloxy-3-methoxybenzoyl)-7-benzyloxy-6-methoxy-1,2,3,4-tetrahydroisoquinoline (0.79 g, 77%).
N-(4-benzyloxy-3-methoxybenzoyl)-7-benzyloxy-6-methoxy-1,2,3,4-tetrahydroisoquinoline
IR νmax (KBr) cm⁻¹ : 3448, 3064, 3032, 2932, 2844, 1634, 1606, 1588, 1516, 1454, 1432, 1372, 1332, 1316, 1266, 1316, 1266, 1216, 1184, 1134, 1106, 1050, 1030, 1002
¹H-NMR (δ ppm in DMSO-d₆): 2.77 (2H, t, J=6.1), 3.67 (2H, t, J=6.1), 3.76 (3H, s), 3.80 (3H, s), 4.55 (2H, s), 5.02 (2H, s), 5.13 (2H, s), 6.77 (1H, s), 6.81 (1H, s), 6.95 (1H, dd, J=2.0, 8.3), 7.03 (1H, d, J=2.0), 7.07 (1H, d, J=8.3), 7.27-7.46 (10H, m)
MS m/z (%) [EI-MS]: 509 (M⁺, 24), 418 (25), 241 (12), 91 (100), 65 (10)

| MS [HMS] (as C₃₂H₃₁NO₅ (M⁺)): | |
|---|---|
| Calculated | 509.22013 |
| Found | 509.21903 |

(3) N-(4-benzyloxy-3-methoxybenzoyl)-7-benzyloxy-6-methoxy-1,2,3,4-tetrahydroisoquinoline (9.02 g) and 5% palladium-carbon catalyst (0.9234 g) were dissolved and suspended in a mixed solution of ethanol (170.0 ml) and ethyl acetate (170.0 ml), and stirred overnight at room temperature in a hydrogen atmosphere. The reaction mixture was filtered and concentrated to dryness under reduced pressure, to obtain a colorless crystalline powder of N-(4-hydroxy-3-methoxybenzoyl)-7-hydroxy-6-methoxy-1,2,3,4-tetrahydroisoquinoline (5.70 g, 98%). N-(4-hydroxy-3-methoxybenzoyl)-7-hydroxy-6-methoxy-1,2,3,4-tetrahydroisoquinoline
IR νmax (KBr) cm⁻¹ : 3412, 3008, 1614, 1598, 1514, 1468, 1444, 1434, 1382, 1366, 1274, 1254, 1240, 1222, 1212, 1190, 1162, 1130, 1104, 1028, 1018, 870, 840
¹H-NMR (δ ppm in DMSO-d₆): 2.72 (2H, t, J=6.1), 3.65 (2H, t, J=6.1), 3.74 (3H, s), 3.79 (3H, s), 4.49 (2H, s), 6.52 (1H, s), 6.69 (1H, s), 6.97-6.79 (3H, m)
MS m/z (%) [EI-MS]: 329 (M⁺, 68), 179 (10), 178 (44), 163 (26), 162 (21), 151 (100), 137 (19)

| MS [HMS] (as C₁₈H₁₉NO₅ (M⁺)): | |
|---|---|
| Calculated | 329.12635 |
| Found | 329.12765 |

### Synthetic Example 58

N-(4-hydroxy-3-methoxybenzoyl)-7-hydroxy-6-methoxy-1,2,3,4-tetrahydroisoquinoline (5.15 g) obtained in Synthetic Example 57 was dissolved into tetrahydrofuran (50 ml). To the solution was added dropwise a 1 M solution of boran/tetrahydrofuran (95.0 ml) at 0°C. The reaction mixture was refluxed for 2 hours and, after adding 6 N hydrochloric acid (20.0 ml), was refluxed for a further 2 hours. The reaction mixture was adjusted to about pH 12 with 6 N sodium hydroxide aqueous solution, and, after removing water layer by decantation, adjusted to about pH 8 with the addition of 6 N hydrochloric acid, and extracted with chloroform. The organic layer was concentrated under reduced pressure to dryness and recrystallized from methanol to obtain a colorless crystalline powder of N-(4-hydroxy-3-methoxybenzyl)-7-hydroxy-6-methoxy-1,2,3,4-tetrahydroisoquinoline (2.82 g, 57%).
N-(4-hydroxy-3-methoxybenzyl)-7-hydroxy-6-methoxy-1,2,3,4-tetrahydroisoquinoline
IR νmax (KBr) cm⁻¹ : 3390, 1614, 1600, 1514, 1468, 1444, 1434, 1382, 1366, 1274, 1254, 1240, 1222, 1212, 1190, 1130, 1104, 1030, 1018, 870, 840
¹H-NMR (δ ppm in CDCl₃): 2.79-2.65 (4H, m), 3.52 (2H, s), 3.58 (2H, s), 3.84 (3H, s), 3.86 (3H, s), 6.54 (1H, s), 6.57 (1H, s), 6.91-6.82 (3H, m)
MS m/z (%) [EI-MS]: 315 (M⁺, 17), 179 (32), 178 (100), 150 (54), 137 (25), 136 (41), 107 (36), 106 (20), 65 (12)

| MS [HMS] (as C₁₈H₂₁NO₄ (M⁺)): | |
|---|---|
| Calculated | 315.14704 |
| Found | 315.14784 |

### Synthetic Example 59

N-(4-methoxybenzyl)-3-methoxybenzamide (3.45 g) obtained in Synthetic Example 22 was dissolved in dimethylsulfoxide (20.0 ml) and potassium hydroxide (2.20 g) was added to the solution. Ethyl iodide (2.5 ml) was added dropwise to the solution, followed by stirring for 17 hours at 60°C. The reaction mixture was poured into ice water and the deposited oily material was extracted with ethyl acetate. The organic layer was washed with water, dried over anhydrous sodium sulfate, and concentrated under reduced pressure. The concentrate was purified by a short column of silica gel to obtain a colorless oil of N-ethyl-N-(4-methoxybenzyl)-3-methoxybenzamide (3.77 g, 99%).
N-ethyl-N-(4-methoxybenzyl)-3-methoxybenzamide
IR νmax (NaCl) cm⁻¹ : 2968, 2936, 1632, 1582, 1512, 1488, 1468, 1434, 1418, 1364, 1314, 1290, 1248, 1176, 1036, 792
¹H-NMR (δ ppm in DMSO-d₆): 1.04 (3H, t, J=7.1), 3.26 (2H, q, J=7.1), 3.75 (3H, s), 3.76 (3H, s), 4.50 (2H, s), 7.35-6.87 (8H, m)
MS m/z (%) [EI-MS]: 299 (M⁺, 42), 298 (16), 270 (17), 136 (19), 135 (100), 121 (29), 107 (17), 77 (13)

| MS [HMS] (as C₁₈H₂₁NO₃ (M⁺)): | |
|---|---|
| Calculated | 299.13203 |
| Found | 299.15173 |

### Synthetic Example 60

N-ethyl-N-(4-methoxybenzyl)-3-methoxybenzamide (2.02 g) obtained in Synthetic Example 59 was dissolved into dichloromethane (40.0 ml). A 0.8 M dichlorometane solution of boron tribromide (20.0 ml) was added dropwise at 0°C to the solution, followed by stirring for 3 hours. The reaction mixture was poured into ice water and extracted with ethyl acetate. The organic layer was washed with water, dried over anhydrous sodium sulfate, and concentrated to dryness under reduced pressure to obtain crude N-ethyl-N-(4-hydroxybenzyl)-3-hydroxybenzamide (1.79 g). This crude product was purified by column chromatography (silica gel, 230-400 mesh; 200 g; φ=60 mm, length=100 mm; pressure: 0.4 kg/cm²; eluent: hexane:ethyl acetate = 1:1; 1 fraction = 80 ml, fractions 27-45) to obtain a colorless amorphous powder of N-ethyl-N-(4-hydroxybenzyl)-3-hydroxybenzamide (1.47 g, 80%).
N-ethyl-N-(4-hydroxybenzyl)-3-hydroxybenzamide
IR νmax (KBr) cm⁻¹ : 3356, 2980, 2940, 2880, 2804, 2684, 2600, 1584, 1574, 1514, 1470, 1456, 1382, 1362, 1308, 1264, 1240, 1174, 1100, 1076, 998, 878
¹H-NMR (δ ppm in DMSO-d₆): 1.01 (3H, t, J=7.1), 3.23 (2H, q, J=7.1), 4.44 (2H, s), 7.22-6.71 (8H, m)
MS m/z (%) [EI-MS]: 271 (M⁺, 36), 270 (16), 242 (13), 135 (19), 122 (11), 121 (100), 107 (27), 106 (28), 93 (15), 78 (11), 44 (24)

| MS [HMS] (as C₁₆H₁₇NO₃ (M⁺)): | |
|---|---|
| Calculated | 271.12080 |
| Found | 271.12070 |

### Synthetic Example 61

N-ethyl-N-(4-hydroxybenzyl)-3-hydroxybenzamide (1.07 g) obtained in Synthetic Example 60 was dissolved into tetrahydrofuran (10.0 ml). To the solution was added dropwise a 1 M solution of boran/tetrahydrofuran (30.0 ml) at 0°C. The reaction mixture was refluxed for 1.5 hours and, after the addition of 6 N hydrochloric acid (2.0 ml), was refluxed for another 1.5 hours. The reaction mixture was adjusted to about pH 8 with 6 N sodium hydroxide aqueous solution and extracted with ether. The organic layer was concentrated under reduced pressure to dryness and purified by column chromatography (silica gel, 230-400 mesh; 100 g; eluent: chloroform:methanol = 20:1; pressure: 0.4 kg/cm²; 1 fraction = 80 ml, fractions 21-33) to obtain a colorless amorphous powder of N-ethyl-N-(4-hydroxybenzyl)-3-hydroxybenzylamine (1.01 g, 99%).
N-ethyl-N-(4-hydroxybenzyl)-3-hydroxybenzylamine
IR νmax (KBr) cm⁻¹ : 3020, 2968, 2804, 2600, 1594, 1514, 1480, 1462, 1368, 1254, 1168, 1096, 1048, 998 838, 784
¹H-NMR (δ ppm in DMSO-d₆): 0.97 (3H, t, J=7.1), 2.45 (2H, q, J=7.1), 3.45 (4H. s), 8.15-6.67 (8H, m)
MS m/z (%) [EI-MS]: 257 (M⁺, 1), 150 (13), 136 (14), 108 (27), 107 (100), 106 (33), 78 (40), 77 (22), 58 (15)

| MS [HMS] (as C₁₆H₁₉NO₂ (M⁺)): | |
|---|---|
| Calculated | 257.14128 |
| Found | 257.14108 |

### Synthetic Example 62

N-(4-methoxybenzyl)-3-methoxybenzamide (3.26 g) obtained in Synthetic Example 22 was dissolved into dimethylsulfoxide (20.0 ml). Sodium hydroxide (2.35 g) was added to the solution, followed by dropwise addition of n-propyl iodide (3.5 ml) while stirring the mixture. After stirring at 60°C for 1 hour, the reaction mixture was poured into ice water and extracted with ethyl acetate. The organic layer was washed with water, dried over anhydrous sodium sulfate, and concentrated to dryness under reduced pressure. The concentrate was purified by column chromatography (silica gel, 230-400 mesh; 280 mg; φ 80 mm, length 150 mm; pressure: 0.4 kg/cm²; eluent: hexane:ethyl acetate = 2:1; 1 fraction = 80 ml, fractions 14-25) to obtain N-(4-methoxybenzyl)-N-n-propyl-3-methoxybenzamide (3.61 g, 95%) as a colorless viscous product.
N-(4-methoxybenzyl)-N-n-propyl-3-methoxybenzamide
IR νmax (NaCl) cm⁻¹ : 2960, 2936, 1632, 1584, 1512, 1488, 1464, 1432, 1416, 1302, 1288, 1248, 1176, 1100, 1036
¹H-NMR (δ ppm in DMSO-d₆): 0.75 (3H, t, J=7.3), 1.51 (2H, sept, J=7.3), 3.19 (2H, t, J=7.3), 3.74 (3H, s), 3.76 (3H, s), 4.49 (2H, s), 7.35-6.87 (8H, m)
MS m/z (%) [EI-MS]: 313 (M⁺, 47), 312 (13), 270 (19), 136 (19), 135 (100), 121 (59), 107 (22), 92 (11), 77 (22)

| MS [HMS] (as C₁₉H₂₃NO₃ (M⁺)): | |
|---|---|
| Calculated | 313.16779 |
| Found | 313.16779 |

### Synthetic Example 63

N-(4-methoxybenzyl)-N-n-propyl-3-methoxybenzamide (2.49 g) was dissolved into dichloromethane (40.0 ml). A 0.8 M dichlorometane solution of boron tribromide (40.0 ml) was added dropwise at 0°C to the solution, followed by stirring for 3.5 hours. The reaction mixture was poured into ice water and extracted with ethyl acetate. The organic layer was washed with water, dried over anhydrous sodium sulfate, and concentrated to dryness under reduced pressure to obtain crude N-(4-hydroxybenzyl)-N-n-propyl-3-hydroxybenzamide (2.35 g). This crude product was purified by column chromatography (silica gel 60, 230-400 mesh; 200 g; φ=75 mm, length=100 mm; pressure: 0.4 kg/cm²; eluent: hexane:ethyl acetate = 1:1; 1 fraction = 100 ml, fractions 18-28) to obtain N-(4-hydroxybenzyl)-N-n-propyl-3-hydroxybenzamide (2.49 g, 87%) as a colorless viscous product.
N-(4-hydroxybenzyl)-N-n-propyl-3-hydroxybenzamide
IR νmax (KBr) cm⁻¹ : 3316, 2964, 2936, 2872, 1586, 1514, 1462, 1446, 1354, 1310, 1228, 1170, 1100, 824, 792, 748, 696
¹H-NMR (δ ppm in DMSO-d₆): 0.74 (3H, t, J=7.3), 1.48 (2H, sext., J=7.3), 3.16 (2H, t, J=7.3), 4.44 (2H, s), 7.23-6.71 (8H, m)
MS m/z (%) [EI-MS]: 285 (M⁺, 15), 122 (12), 121 (100), 107 (50), 106 (10), 93 (28), 78 (33), 65 (32)

| MS [HMS] (as C₁₇H₁₉NO₃ (M⁺)): | |
|---|---|
| Calculated | 285.13645 |
| Found | 285.13555 |

### Synthetic Example 64

N-(4-hydroxybenzyl)-N-n-propyl-3-hydroxybenzamide (1.19 g) obtained in Synthetic Example 63 was dissolved into tetrahydrofuran (12.0 ml). To the solution was added dropwise a 1 M solution of boran/tetrahydrofuran (36.0 ml) at 0°C. The reaction mixture was refluxed for 2.5 hours and, after addition of 6 N hydrochloric acid (2.0 ml), was further refluxed for 2 hours. The reaction mixture was then adjusted to about pH 8 with 6 N sodium hydroxide aqueous solution and extracted with ether. The organic layer was concentrated under reduced pressure to dryness and purified by column chromatography (silica gel, 230-400 mesh; 120 g; eluent: chloroform:methanol = 20:1; pressure: 0.4 kg/cm²; 1 fraction = 40 ml, fractions 10-15) to obtain N-(4-hydroxybenzyl)-N-n-propyl-3-hydroxybenzylamine (1.02 g, 90%) as a colorless viscous product.
N-(4-hydroxybenzyl)-N-n-propyl-3-hydroxybenzylamine
IR νmax (KBr) cm⁻¹ : 3264, 3024, 2960, 2932, 2872, 2800, 2588, 1884, 1590, 1512, 1480, 1462, 1452, 1364, 1252, 1166, 1098, 1066, 1014, 996, 966, 844, 822, 784, 750, 694, 642, 584, 558, 514
¹H-NMR (δ ppm in DMSO-d₆): 0.78 (3H. t. J=7.3), 1.43 (2H, sept., J=7.3), 2.35 (2H, t, J=7.3), 3.44 (4H, s), 7.11-6.59 (8H, m)
MS m/z (%) [EI-MS]: 271 (M⁺, 2), 136 (44), 108 (15), 107 (100), 106 (26), 78 (27), 77 (19), 58 (12), 52 (11), 51 (12)

| MS [HMS] (as C₁₇H₂₁NO₂ (M⁺)): | |
|---|---|
| Calculated | 271.15758 |
| Found | 271.15788 |

### Synthetic Example 65

(1) N-(4-benzyloxy-3-methoxyphenetyl)-3-benzyloxy-4-methoxyphenetylamine (4.09 g) obtained in Synthetic Example 52 was suspended into triethylamine (1.30 ml) and ether (70.0 ml), and cooled to 0°C. A solution of acetyl chloride in ether (0.64 ml/10.0 ml) was added dropwise to it while taking care to the exothermic heat. After stirring for 16 hours, white precipitate produced was collected by filtration. The precipitate was washed with water and purified by column chromatography (silica gel, 230-400 mesh; 350 g; φ 60 mm, length 160 mm; pressure 0.5 kg/cm²; eluent: chloroform:methanol = 30:1; fraction 200-500 ml) to obtain N-(4-benzyloxy-3-methoxyphenetyl)-N-(3-benzyloxy- 4-methoxyphenetyl)acetamide (4.44 g, 99%) as a colorless powder.
N-(4-benzyloxy-3-methoxyphenetyl)-N-(3-benzyloxy-4-methoxyphenetyl)acetamide
IR νmax (NaCl) cm⁻¹ : 3060, 3004, 2936, 2868, 2836, 1642, 1590, 1512, 1454, 1442, 1372, 1330, 1262, 1232, 1156, 1140, 1024, 850, 806, 748, 696, 664
¹H-NMR (δ ppm in DMSO-d₆): 1.82 (3H, s), 2.68 (4H, t, J=7.6), 3.51-3.38 (4H, br), 3.74 (3H, s), 3.76 (3H, s), 5.03 (2H, s), 5.05 (2H, s), 6.93-6.66 (6H, m), 7.40-7.29 (10H, m)
MS m/z (%) [EI-MS]: 539 (M⁺, 5), 312 (6), 299 (28), 241 (10), 240 (42), 208 (31), 137 (15), 91 (100), 65 (6)

| MS [HMS] (as C₃₄H₃₇NO₅): | |
|---|---|
| Calculated | 539.26722 |
| Found | 539.26792 |

(2) 5% palladium-carbon catalyst (0.91 g) was suspended in methanol (10.0 ml) and hydrogen was absorbed in the suspension. To this was added a solution of N-(4-benzyloxy-3-methoxyphenetyl)-N-(3-benzyloxy-4-methoxyphenetyl)acetamide (2.39 g) in methanol (20.0 ml), and the mixture was stirred for 3 hours. The reaction mixture was filtered through cerite and concentrated under reduced pressure to dryness, to obtain a colorless amorphous powder of N-(4-hydroxy-3-methoxyphenetyl)-N-(3-hydroxy-4-methoxyphenetyl)acetamide (0.82 g, 51%).
N-(4-hydroxy-3-methoxyphenetyl)-N-(3-hydroxy-4-methoxyphenetyl)acetamide
IR νmax (KBr) cm⁻¹ : 3244, 2936, 2840, 1622, 1594, 1514, 1432, 1368, 1272, 1242, 1154, 1130, 1030, 806, 758, 640, 614, 594
¹H-NMR (δ ppm in DMSO-d₆): 1.85 (3H, s), 2.75-2.60 (4H, m), 3.50-3.40 (4H, br), 3.73 (3H, s), 3.75 (3H, s), 6.83-6.54 (6H, m)
MS m/z (%) [EI-MS]: 359 (M⁺, 7), 222 (30), 210 (14), 209 (28), 180 (48), 151 (64), 150 (100), 138 (28), 137 (31), 135 (9), 119 (9), 91 (19)

| MS [HMS] (as C₂₀H₂₅NO₅): | |
|---|---|
| Calculated | 359.17329 |
| Found | 359.17549 |

### Synthetic Example 66

1.00 g of 5% palladium-carbon catalyst was suspended in 10.0 ml of ethanol and hydrogen was absorbed in the suspension. To this was added a solution of N-(4-benzyloxy-3-methoxyphenetyl)-3-benzyloxy-4-methoxyphenetylamine (3.50 g) in ethanol (30.0 ml), and the mixture was stirred for 3 hours. The reaction mixture was filtered through cerite and treated with discoloring carbon to obtain a colorless amorphous powder of N-(4-hydroxy-3-methoxyphenetyl)-3-hydroxy-4-methoxyphenetylamine (1.34 g, 60%).
N-(4-hydroxy-3-methoxyphenetyl)-3-hydroxy-4-methoxyphenetylamine
IR νmax (KBr) cm⁻¹ : 2956, 2836, 1594, 1512, 1462, 1442, 1326, 1280, 1256, 1226, 1152, 1130, 1032, 952, 906, 858, 814, 758, 632, 608
¹H-NMR (δ ppm in CD₃OD): 2.79-2.65 (8H, m), 3.79 (3H, s), 3.81 (3H, s), 6.77-6.53 (6H, m)
MS m/z (%): 317 (M⁺, 4), 206 (11), 181 (12), 180 (100), 151 (67), 137 (16), 119 (11), 91 (14), 43 (4)

### Preparation Example 1

| | | |
|---|---|---|
| (1) | Corn starch | 44 g |
| (2) | Crystalline cellulose | 40 g |
| (3) | Carboxymethyl cellulose calcium | 5 g |
| (4) | Light silicic acid anhydride | 0.5 g |
| (5) | Magnesium stearate | 0.5 g |
| (6) | Compound of Synthetic Example 1 | 10 g |
| | Total | 100 g |

Components (1)-(6) of the above formulation were mixed homogeneously and press-molded in a tablet machine to prepare tablets, each weighing 200 mg.

Each tablet contained 20 mg of the compound prepared in Synthetic Example 1. An adult dose is 3 to 10 tablets per day, administered at several times a day.

### Preparation Example 2

| | | |
|---|---|---|
| (1) | Crystalline cellulose | 84.5 g |
| (2) | Magnesium stearate | 0.5 g |
| (3) | Carboxymethyl cellulose calcium | 5 g |
| (6) | Compound of Synthetic Example 2 | 10 g |
| | Total | 100 g |

Components (1), (4), and a portion of component (2) were mixed homogeneously and press-molded. The molded product was pulverized. Component (3) and the remaining portion of component (2) were added to the pulverized material, and the mixture was press-molded in a tablet machine to prepare tablets, each weighing 200 mg.

Each tablet contained 20 mg of the compound prepared in Synthetic Example 2. An adult dose is 3 to 10 tablets per day, administered at several times a day.

### Preparation Example 3

| | | |
|---|---|---|
| (1) | Crystalline cellulose | 34.5 g |
| (2) | Ethanol solution of 10% hydroxypropyl cellulose | 50 g |
| (3) | Carboxymethyl cellulose calcium | 5 g |
| (4) | Magnesium stearate | 0.5 g |
| (5) | Compound of Synthetic Example 3 | 10 g |
| | Total | 100 g |

Components (1), (2), and (5) were homogeneously mixed, kneaded by a conventional method, granulated by an extrusion granulator, dried, and pulverized. Components (3) and (4) were added to the pulverized material, and the mixture was press-molded in a tablet machine to prepare tablets, each weighing 200 mg.

Each tablet contained 20 mg of the compound prepared in Synthetic Example 3. An adult dose is 3 to 10 tablets per day, administered at several times a day.

### Preparation Example 4

| | | |
|---|---|---|
| (1) | Corn starch | 84 g |
| (2) | Magnesium stearate | 0.5 g |
| (3) | Carboxymethyl cellulose calcium | 5 g |
| (4) | Light silicic acid anhydride | 0.5 g |
| (5) | Compound of Synthetic Example 4 | 10 g |
| | Total | 100 g |

Components (1)-(5) of the above formulation were mixed homogeneously and press-molded by a press-molding machine. The molded product was pulverized and sieved to obtain granules.

1 g of the granules contained 100 mg of the compound prepared in Synthetic Example 4. An adult dose of the granules is 0.6-2 g per day, administered at several times a day.

### Preparation Example 5

| | | |
|---|---|---|
| (1) | Crystalline cellulose | 55 g |
| (2) | Ethanol solution of 10% hydroxypropyl cellulose | 35 g |
| (3) | Compound of Synthetic Example 5 | 10 g |
| | Total | 100 g |

Components (1), (2), and (3) were homogeneously mixed, kneaded, granulated by an extrusion granulator, dried, and sieved to obtain granules.

1 g of the granules contained 100 mg of the compound prepared in Synthetic Example 5. An adult dose of the granules is 0.6-2 g per day, administered at several times a day.

### Preparation Example 6

| | | |
|---|---|---|
| (1) | Corn starch | 89.5 g |
| (2) | Light silicic acid anhydride | 0.5 g |
| (3) | Compound of Synthetic Example 6 | 10 g |
| | Total | 100 g |

Components (1)-(3) of the above formulation were mixed homogeneously and filled in No. 2 capsules, in an amount of 200 mg per each capsule.

Each capsule contained 20 mg of the compound prepared in Synthetic Example 6. An adult dose of is 3-10 capsules per day, administered at several times a day.

### Preparation Example 7

| | | |
|---|---|---|
| (1) | Distilled water for injection | 89.5 g |
| (2) | Soybean oil | 5 g |
| (3) | Soybean phospholipid | 2.5 g |
| (4) | Glycerine | 2 g |
| (5) | Compound of Synthetic Example 7 | 1 g |
| | Total | 100 g |

Components (5) and (2) were dissolved into component (3). To this solution a solution of components (1) and (4) was added and emulsified to obtain an injection preparation.

### Industrial Applicability

As illustrated above, because the phellodendrine analogs (I) and related compounds thereof exhibit an excellent allergy type IV suppressing action, they can be used as a drug effective for curing diseases involving allergy type IV, such as chronic hepatitis, intractable asthma, nephrotic syndrome, rheumatism, and the like.

## Claims

1. A phellodendrine analog represented by the following formula (I), 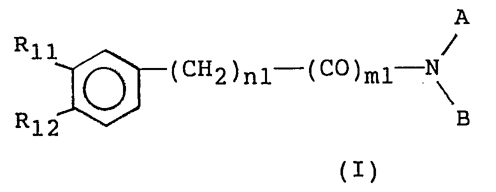 wherein A represents a group 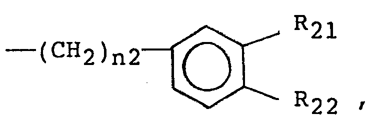 wherein R₂₁ and R₂₂ individually represent a hydrogen atom, a hydroxy group, or a lower alkoxy group; and n2 is 1 or 2; B represents a hydrogen atom, a lower alkyl group, or a lower acyl group, or A and B may form, together with the adjacent nitrogen atom, a substituted 1,2,3,4-tetrahydroisoquinolyl ring represented by the following formula, 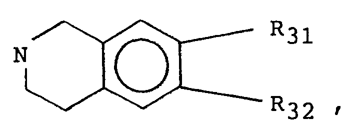 wherein R₃₁ and R₃₂ individually represent a hydrogen atom, a hydroxy group, or a lower alkoxy group; R₁₁ and R₁₂ individually represent a hydrogen atom, a hydroxy group, or a lower alkoxy group; n1 denotes a number 0-2; and m1 denotes a number 0-1; provided that when A is a group 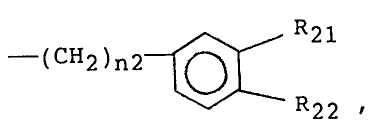 if n2 is 2, B is a lower acyl group, and further that when A and B together form a substituted 1,2,3,4-tetrahydroisoquinolyl ring, n1 is 1 and m1 is not 0.

2. A phellodendrine analog according to Claim 1, represented by the following formula (Ia), 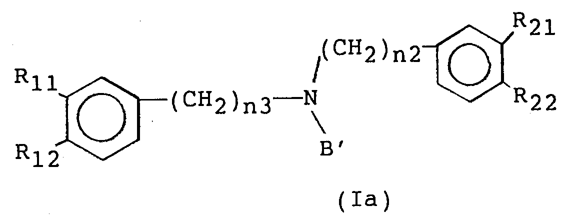 wherein R₁₁, R₁₂, R₂₁, R₂₂, and n2 have the same meanings as defined above, n3 is 1 or 2, and B' represents a hydrogen atom, a lower alkyl group, or a lower acyl group, provided that when either one of R₁₁ or R₁₂ is a hydrogen atom and either one of R₂₁ or R₂₂ is a hydrogen atom, B' is a lower acyl group.

3. A phellodendrine analog according to Claim 1, represented by the following formula (Ib), 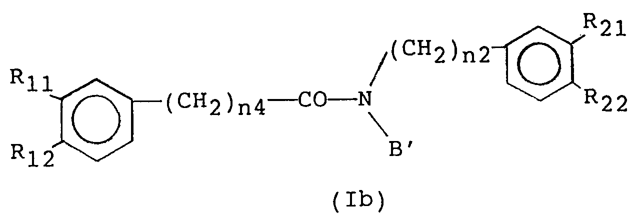 wherein R₁₁, R₁₂, R₂₁, R₂₂, and n2 have the same meanings as defined above, n4 is 0 or 1, and B' represents a hydrogen atom, a lower alkyl group, or a lower acyl group, provided that when either one of R₁₁ or R₁₂ is a hydrogen atom and either one of R₂₁ or R₂₂ is a hydrogen atom, B' is a lower acyl group.

4. A phellodendrine analog according to Claim 1, represented by the following formula (Ic), 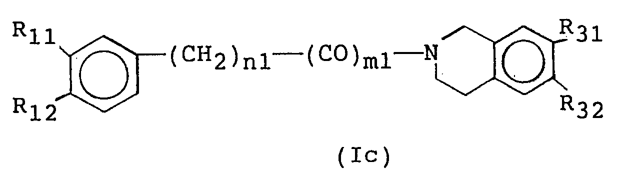 wherein R₁₁, R₁₂, R₃₁, R₃₂, n1, and m1 have the same meanings as defined above.

5. An allergy type IV suppressor comprising as an active ingredient a phellodendrine analog represented by the following formula (I'), 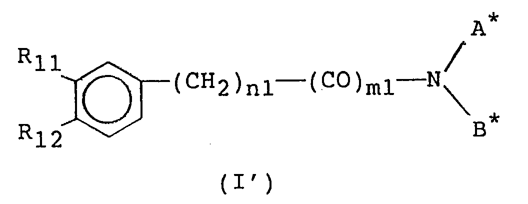 wherein A* represents a group, 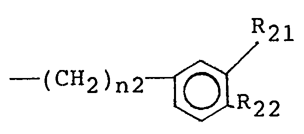 wherein R₂₁ and R₂₂ individually represent a hydrogen atom, a hydroxy group, or a lower alkoxy group, and n2 is 1 or 2; B* represents a hydrogen atom, a lower alkyl group, or a lower acyl group, or A* and B* may form, together with the adjacent nitrogen atom, a substituted 1,2,3,4-tetrahydroisoquinolyl ring represented by the following formula, 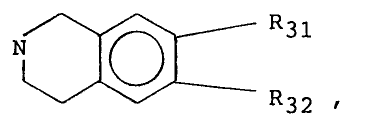 wherein R₃₁ and R₃₂ individually represent a hydrogen atom, a hydroxy group, or a lower alkoxy group; R₁₁ and R₁₂ individually represent a hydrogen atom, a hydroxy group, or a lower alkoxy group; n1 denotes a number 0-2; and m1 denotes a number 0-1.

6. An allergy type IV suppressor comprising as an active ingredient a phellodendrine analog represented by the following formula (X), 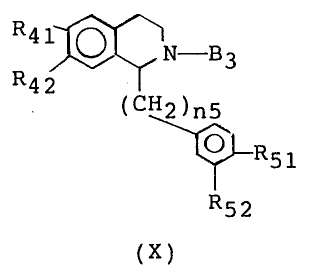 wherein R₄₁, R₄₂, R₅₁, and R₅₂ individually represent a hydrogen atom, a hydroxy group, or a lower alkoxy group; B₃ represents a hydrogen atom, a lower alkyl group, or a lower acyl group; and n5 denotes 0 or 1.
